(19) Europäisches Patentamt
European Patent Office
Office européen des brevets

(11) EP 0 528 369 B1

(12) EUROPÄISCHE PATENTSCHRIFT

(45) Veröffentlichungstag und Bekanntmachung des Hinweises auf die Patenterteilung: **24.11.1999 Patentblatt 1999/47**

(21) Anmeldenummer: **92113877.2**

(22) Anmeldetag: **14.08.1992**

(51) Int. Cl.⁶: **C07D 207/08**, C07D 207/26, C07D 401/04, C07D 401/06, C07D 401/12, C07D 401/14, C07D 403/04, C07D 403/06, C07D 403/12, C07D 405/06, C07D 405/12, C07D 409/12, C07D 417/04, C07D 417/06, C07D 417/12, A61K 31/395, A61K 31/435, A61K 31/495, A61K 31/425

(54) **Cyclische Iminoderivate, diese Verbindungen enthaltende Arzneimittel und Verfahren zu ihrer Herstellung**

Cyclic imino derivatives, pharmaceutical compositions containing them and process for their preparation

Dérivés à fonction imine cyclique, compositions pharmaceutiques les contenant et procédé pour leur préparation

(84) Benannte Vertragsstaaten:
**AT BE CH DE DK ES FR GB GR IE IT LI LU NL PT SE**

(30) Priorität: **19.08.1991 DE 4127404**

(43) Veröffentlichungstag der Anmeldung:
**24.02.1993 Patentblatt 1993/08**

(73) Patentinhaber:
**Boehringer Ingelheim Pharma KG**
**55216 Ingelheim am Rhein (DE)**

(72) Erfinder:
- **Austel, Volkhard, Dr. Dipl.-Chem.**
  **W-7950 Biberach 1 (DE)**
- **Eisert, Wolfgang, Prof. Dr. Dr. Dr.**
  **W-7950 Biberach 1 (DE)**
- **Himmelsbach, Frank, Dr. Dipl.-Chem.**
  **W-7951 Mittelbiberach (DE)**
- **Linz, Günter, Dr. Dipl.-Chem.**
  **W-7951 Mittelbiberach (DE)**
- **Müller, Thomas, Dr. Dipl.-Chem.**
  **W-7950 Biberach 1 (DE)**
- **Pieper, Helmut, Dr. Dipl.-Chem.**
  **W-7951 Mittelbiberach (DE)**
- **Weisenberger, Johannes, Dr. Dipl.-Chem.**
  **W-7950 Biberach 1 (DE)**

(74) Vertreter: **Laudien, Dieter, Dr.**
**Boehringer Ingelheim GmbH,**
**Binger Strasse 173**
**55216 Ingelheim (DE)**

(56) Entgegenhaltungen:
**EP-A- 0 381 033**
**EP-A- 0 483 667**
**EP-A- 0 505 868**
**EP-A- 0 542 363**
**US-A- 3 364 221**

Bemerkungen:
Die Akte enthält technische Angaben, die nach dem Eingang der Anmeldung eingereicht wurden und die nicht in dieser Patentschrift enthalten sind.

EP 0 528 369 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

In der US-A-3,364,221 werden bereits N-Carboxyalkylpiperidinyl-alkan-piperidinyle, welche Zwischenprodukte zur Herstellung von Polyamid-Kunststoffen und Korrosions-Inhibitoren darstellen,

in der EP-A-0,381,033 und in der EP-A-0,542,363 Verbindungen, die keine Pyrrolidin- oder Pyrrolidinongruppe enthalten können, und

in der EP-A-0,505,868 Verbindungen beschrieben, die einen heteroaromatischen Rest und einen Pyrrolidinylen- oder Phenylenrest enthalten können, wobei die beiden letzteren Ringe durch eine TOOC-$CH_2$-O- oder $TOOCCH_2OCH_2$-Gruppe substituiert sind.

Es wurde nun gefunden, daß die neuen cyclischen Iminoderivate der allgemeinen Formel

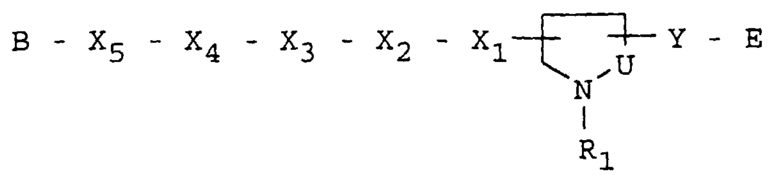

, (I)

deren Stereoisomere, deren Tautomere, deren Gemische und deren Additionssalze, insbesondere deren physiologisch verträglichen Additionssalze mit anorganischen oder organischen Säuren oder Basen, wertvolle Eigenschaften aufweisen. So stellen die Verbindungen der allgemeinen Formel I, in der B eine Cyanogruppe darstellt, wertvolle Zwischenprodukte zur Herstellung der übrigen Verbindungen der allgemeinen Formel I dar, welche wertvolle pharmakologische Eigenschaften aufweisen, vorzugsweise aggregationshemmende Wirkungen.

Gegenstand der vorliegenden Erfindung sind somit die cyclischen Iminoderivate der obigen allgemeinen Formel I, deren Stereoisomere, deren Tautomere, deren Gemische und deren Additionssalze, insbesondere deren physiologisch verträglichen Additionssalze mit anorganischen oder organischen Säuren oder Basen, Verfahren zu ihrer Herstellung, die die pharmazeutich wirksamen Verbindungen der allgemeinen Formel I enthaltende Arzneimittel und deren Verwendung.

In der obigen allgemeinen Formel bedeutet mit der Maßgabe, daß mindestens einer der Reste $R_1$, $X_3$, $X_5$, $X_5$-$X_4$-$X_3$ zusammen oder $X_5$-$X_4$ zusammen einen heterocyclischen Rest enthält, und der kürzeste Abstand zwischen den Gruppen B und E mindestens 10 Bindungen beträgt,

U eine Methylengruppe und $R_1$ ein Wasserstoffatom, eine Pyridincarbonyl- oder Pyrimidinylgruppe oder

U eine Carbonylgruppe und $R_1$ ein Wasserstoffatom, eine 3-Phenylpropyl- oder Pyridinylgruppe,

$X_1$ eine Methylen- oder Ethylengruppe,

$X_2$ eine Bindung, ein Sauerstoffatom, eine -CONH- oder Iminogruppe,

$X_3$ eine gegebenenfalls durch eine Methylgruppe substituierte 1,4-Phenylen-, Pyridazin-3,6-ylen-, 1H-Pyridin-2-on-1,3-ylen-, 1H-Pyridin-2-on-1,5-ylen-, 2H-Pyridazin-3-on-4,6-ylen- oder 1H,3H-Pyrimidin-2,4-dion-3,5-ylen-gruppe mit der Maßgabe, daß ein Stickstoffatom der Gruppe $X_3$ nicht mit einem Heteroatom oder der -CONH-Gruppe der Gruppe $X_2$ verknüpft ist,

$X_4$ eine Bindung oder eine -NHCO-Gruppe mit der Maßgabe, daß die -NHCO-Gruppe nicht mit einem Stickstoffatom der Gruppe $X_3$ verknüpft ist, und

$X_5$ eine Piperidin-1,4-ylen-gruppe, eine gegebenenfalls durch ein Chloratom substituierte 1,4-Phenylen-, Pyridin-2,4-ylen-, Pyridin-2,5-ylen-, Pyrimidin-2,5-ylen-, Pyrazin-2,5-ylen- oder Thiazol-2,5-ylen-gruppe mit der Maßgabe, daß das Ringstickstoffatom der Piperidin-1,4-ylengruppe nicht mit einem Stickstoffatom der Gruppe $X_3$ oder $X_4$ verknüpft ist, oder

$X_5$-$X_4$-$X_3$ zusammen auch eine Isoindolin-2,5-ylen- oder 1,2,3,4-Tetrahydro-isochinolin-2,7-ylen-gruppe mit der

Maßgabe, daß das Ringstickstoffatom dieser Gruppen nicht mit einem Heteroatom der Gruppe $X_2$ verknüpft ist, oder

$X_5$-$X_4$ zusammen auch eine Isochinolin-1,6-ylen-gruppe, wobei der Rest B in Stellung 1 und der Rest $X_3$ in Stellung 6 steht, oder eine 1,2,3,4-Tetrahydroisochinolin-2,6-ylen-gruppe, wobei der Rest B in Stellung 2 und der Rest $X_3$ in Stellung 6 steht,

B eine Cyanogruppe, eine Amidinogruppe, in der an einem der Stickstoffatome ein Wasserstoffatom durch eine Alkoxycarbonylgruppe mit insgesamt 2 bis 5 Kohlenstoffatomen ersetzt sein kann, eine gegebenenfalls durch eine Benzylgruppe substituierte Aminogruppe mit der Maßgabe, daß die Aminogruppe nicht mit einem Ringstickstoffatom der Gruppe $X_5$-$X_4$-$X_3$ oder $X_5$ verknüpft ist, oder, falls B mit einem Ringstickstoffatom der Gruppe $X_5$-$X_4$-$X_3$ oder $X_5$ verknüpft ist oder, falls B mit der Gruppe $X_5$-$X_4$ verknüpft ist, zusätzlich auch ein Wasserstoffatom,

Y eine Methylengruppe und

E eine Carboxygruppe oder eine Alkoxycarbonylgruppe mit insgesamt 2 bis 5 Kohlenstoffatomen.

Bevorzugte Verbindungen der obigen allgemeinen Formel I sind diejenigen, in denen mit der Maßgabe, daß mindestens einer der Reste $R_1$, $X_3$, $X_5$, $X_5$-$X_4$-$X_3$ zusammen oder $X_5$-$X_4$ zusammen einen heterocyclischen Rest enthält, und der kürzeste Abstand zwischen den Gruppen B und E mindestens 10 Bindungen beträgt,

U eine Methylengruppe und $R_1$ ein Wasserstoffatom, eine Pyridin-3-carbonyl- oder Pyrimidin-2-ylgruppe oder

U eine Carbonylgruppe und $R_1$ ein Wasserstoffatom, eine 3-Phenylpropyl-, Pyridin-2-yl- oder Pyridin-3-ylgruppe,

$X_2$-$X_1$ eine $-O-CH_2-$, $-NH-CH_2-$, $-CONH-CH_2-$, $-CONH-CH_2CH_2-$ oder, falls $X_3$ eine 1H-Pyridin-2-on-1,3-ylen-, 1H-Pyridin-2-on-1,5-ylen- oder 1H,3H-Pyrimidin-2,4-dion-3,5-ylen-gruppe darstellt, auch eine Methylengruppe,

$X_3$ eine 1,4-Phenylen-, Pyridazin-3,6-ylen-, 1H-Pyridin-2-on-1,3-ylen-, 1H-Pyridin-2-on-1,5-ylen-, 2H-Pyridazin-3-on-4,6-ylen-, 2-Methyl-2H-pyridazin-3-on-4,6-ylen- oder 1H,3H-Pyrimidin-2,4-dion-3,5-ylen-Gruppe, wobei die 1H-Pyridin-2-on-1,3-ylen-, 1H-Pyridin-2-on-1,5-ylen- und die 1H,3H-Pyrimidin-2,4-dion-3,5-ylen-gruppe über ein Ringstickstoffatom an die Gruppe $X_2$-$X_1$ gebunden ist,

$X_4$ eine Bindung oder eine -NHCO-Gruppe und

$X_5$ eine 1,4-Phenylen-, Pyridin-2,4-ylen-, Pyridin-2,5-ylen-, Pyrimidin-2,5-ylen-, Pyrazin-2,5-ylen-, Piperidin-1,4-ylen- oder Thiazol-2,5-ylen-Gruppe, wobei die Piperidin-1,4-ylen- und die Pyridin-2,4-ylengruppe jeweils über die Position 4 an $X_4$ gebunden ist, oder

$X_5$-$X_4$-$X_3$ zusammen auch eine Isoindolin-2,5-ylen-gruppe, wobei diese über den Benzoteil an die Gruppe $X_2$-$X_1$ gebunden ist, oder

$X_5$-$X_4$ zusammen auch einen Isochinolin-1,6-ylen- oder 1,2,3,4-Tetrahydroisochinolin-2,6-ylen-ring, falls $X_3$ eine 1,4-Phenylengruppe darstellt, wobei der Isochinolin-1,6-ylen- und 1,2,3,4-Tetrahydroisochinolin-2,6-ylen-ring jeweils über die Position 6 an die 1,4-Phenylengruppe gebunden ist,

B eine Amidinogruppe, in der an einem der Stickstoffatome ein Wasserstoffatom durch eine Alkoxycarbonylgruppe mit insgesamt 2 bis 5 Kohlenstoffatomen ersetzt sein kann, oder, falls $X_5$ eine 1,4-Piperidinylengruppe darstellt, zusätzlich auch ein Wasserstoffatom, oder, falls $X_5$-$X_4$ zusammen einen Isochinolin-1,6-ylen-ring darstellt, eine Aminogruppe oder ein Wasserstoffatom oder, falls $X_5$-$X_4$ einen 1,2,3,4-Tetrahydroisochinolin-2,6-ylen-ring darstellt, ein Wasserstoffatom oder, falls $X_5$ einen Pyridin-2,4-ylenring darstellt, eine Benzylaminogruppe,

Y eine Methylengruppe und

E eine Carboxygruppe oder eine Alkoxycarbonylgruppe mit insgesamt 2 bis 5 Kohlenstoffatomen bedeuten,

deren Stereoisomere, deren Tautomere, deren Gemische und deren Salze.

Besonders bevorzugte Verbindungen der obigen allgemeinen Formel I sind diejenigen, in denen $R_1$, U, B, E, $X_1$ bis $X_5$ und Y wie vorstehend erwähnt definiert sind und der Rest -Y-E in Position 3 und der Rest B-$X_5$-$X_4$-$X_3$-$X_2$-$X_1$- in Position 5 des 2-Pyrrolidinon- oder Pyrrolidinringes steht, und einer der Reste $X_5$ oder $X_3$ eine 1,4-Phenylengruppe darstellt,
deren Stereoisomere, deren Tautomere, deren Gemische und deren Salze.

Als besonders bevorzugte Verbindungen seien beispielsweise folgende erwähnt:

(1) (3S,5S)-5-[[4-(5-Amidino-2-pyridyl)phenyl]oxymethyl]-3-carboxymethyl-2-pyrrolidinon,

(2) (3S,5S)-5-[[4-(2-Amidino-5-pyrimidyl)phenyl]oxymethyl]-3-carboxymethyl-2-pyrrolidinon,

(3) (3S,5S)-5-[[4-(5-Amidino-2-pyrimidyl)phenyl]oxymethyl]-3-carboxymethyl-2-pyrrolidinon,

(4) (3S,5S)-5-[[4-(5-Amidino-2-thiazolyl)phenyl]oxymethyl]-3-carboxymethyl-2-pyrrolidinon,

(5) (3S,5S)-5-[[4-(2-Amidino-5-pyridyl)phenyl]oxymethyl]-3-carboxymethyl-2-pyrrolidinon,

(6) (3S,5S)-5-[[4-(5-Amidino-2-pyrazinyl)phenyl]oxymethyl]-3-carboxymethyl-2-pyrrolidinon,

(7) (3S,5S)-5-[(4'-Amidino-4-biphenylyl)oxymethyl]-3-carboxymethyl-1-(2-pyrimidyl)-pyrrolidin,

(8) (3S,5S)-5-[(4'-Amidino-4-biphenylyl)oxymethyl]-3-carboxymethyl-1-nicotinoyl-pyrrolidin,

(9) (3S,5S)-5-[(4'-Amidino-4-biphenylyl)oxymethyl]-3-carboxymethyl-1-(2-pyridyl)-2-pyrrolidinon,

(10) (3S,5S)-5-[(4'-Amidino-4-biphenylyl)oxymethyl]-3-carboxymethyl-1-(3-pyridyl)-2-pyrrolidinon,

(11) (3S,5S)-5-[[6-(4-Amidinophenyl)-3-pyridazinyl]oxymethyl-3-carboxymethyl-1-(3-phenylpropyl)-2-pyrrolidinon,

(13) (3S,5S)-5-[[6-(4-Amidinophenyl)-3-pyridazinyl]aminomethyl]-3-carboxymethyl-2-pyrrolidinon,

(14) (3S,5S)-5-[[4-[5-(N-Methoxycarbonylamidino)-2-pyridyl]-phenyl]oxymethyl]-3-[methoxycarbonyl)methyl]-2-pyrrolidinon,

(15) (3S,5S)-5-[[4-[2-(N-Methoxycarbonylamidino)-5-pyridyl]-phenyl]oxymethyl]-3-[(methoxycarbonyl)methyl]-2-pyrrolidinon,

(16) (3S,5S)-5-[[4-[5-(N-Methoxycarbonylamidino)-2-pyrazinyl]-phenyl]oxymethyl]-3-[(methoxycarbonyl)methyl]-2-pyrrolidinon,

deren Tautomere und deren Salze.

Erfindungsgemäß erhält man die neuen Verbindungen der allgemeinen Formel I beispielsweise nach folgenden an und für sich bekannten Verfahren:

a) Zur Herstellung von Verbindungen der allgemeinen Formel I, in der E eine Carboxygruppe darstellt:
Überführung einer Verbindung der allgemeinen Formel

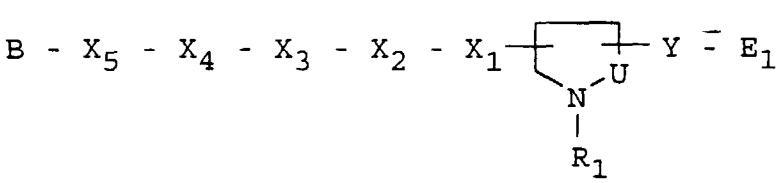

, (II)

in der

$R_1$, B, U, $X_1$ bis $X_5$ und Y wie eingangs definiert sind und

$E_1$, das an ein Kohlenstoffatom gebunden ist, eine mittels Hydrolyse, Behandlung mit Säuren, Thermolyse oder Hydrogenolyse in eine Carboxygruppe überführbare Gruppe darstellt.

Beispielsweise können funktionelle Derivate der Carboxylgruppe wie deren unsubstituierte oder substituierte Amide, Ester, Thioester, Trimethylsilylester, Orthoester, Iminoester, Amidine oder Anhydride, oder die Nitrilgruppe mittels Hydrolyse in eine Carboxylgruppe, Ester mit tertiären Alkoholen, z.B. der tert. Butylester, mittels Behandlung mit einer Säure oder Thermolyse in eine Carboxylgruppe und Ester mit Aralkanolen, z.B. der Benzylester, mittels Hydrogenolyse in eine Carboxylgruppe übergeführt werden.

Die Hydrolyse wird zweckmäßigerweise entweder in Gegenwart einer Säure wie Salzsäure, Schwefelsäure, Phosphorsäure, Trichloressigsäure oder Trifluoressigsäure, in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10°C und 120°C, z.B. bei Temperaturen zwischen Raumtemperatur und der Siedetemperatur des Reaktionsgemisches, durchgeführt. Bei der Behandlung mit einer organischen Säure wie Trichloressigsäure oder Trifluoressigsäure können gegebenenfalls vorhandene alkoholische Hydroxygruppen gleichzeitig in eine entsprechende Acyloxygruppe wie die Trifluoracetoxygruppe übergeführt werden.

Bedeutet $E_1$ in einer Verbindung der Formel II eine Cyano- oder Aminocarbonylgruppe, so können diese Gruppen auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen O und 50°C in die Carboxylgruppe übergeführt werden.

Bedeutet $E_1$ in einer Verbindung der Formel II beispielsweise die tert.-Butyloxycarbonylgruppe, so kann die tert.-Butylgruppe auch durch Behandlung mit einer Säure wie Trifluoressigsäure, Ameisensäure, p-Toluolsulfonsäure, Schwefelsäure, Phosphorsäure oder Polyphosphorsäure gegebenenfalls in einem inerten Lösungsmittel wie Methylenchlorid, Chloroform, Benzol, Toluol, Tetrahydrofuran oder Dioxan vorzugsweise bei Temperaturen zwischen -10°C und 120°C, z.B. bei Temperaturen zwischen 0 und 60°C, oder auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40°C und 100°C, abgespalten werden.

Bedeutet $E_1$ in einer Verbindung der Formel II beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Ethanol, Ethanol/Wasser, Eisessig, Essigsäureäthylester, Dioxan oder Dimethylformamid vorzugsweise bei Temperaturen zwischen 0 und 50°C, z.B. bei Raumtemperatur, und einem Wasserstoffdruck von 1 bis 10 bar abgespalten werden. Bei der Hydrogenolyse können gleichzeitig andere Reste, z.B. eine Nitrogruppe in eine Aminogruppe, oder eine Benzyloxygruppe in eine Hydroxygruppe übergeführt werden.

b) Zur Herstellung von Verbindungen der allgemeinen Formel I, in der B eine Amidinogruppe darstellt:
Umsetzung einer gegebenenfalls im Reaktionsgemisch gebildeten Verbindung der allgemeinen Formel

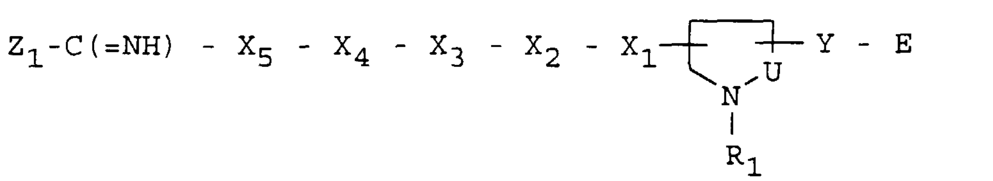

, (III)

in der

$R_1$, E, U, $X_1$ bis $X_5$ und Y wie wie eingangs erwähnt definiert sind und

$Z_1$ eine Alkoxy- oder Aralkoxygruppe wie die Methoxy-, Ethoxy-, n-Propoxy-, Isopropoxy- oder Benzyloxygruppe oder eine Alkylthio- oder Aralkylthiogruppe wie die Methylthio-, Ethylthio-, n-Propylthio- oder Benzylthiogruppe darstellt, mit Ammoniak oder mit dessen Säureadditionssalzen.

Die Umsetzung wird zweckmäßigerweise in einem Lösungsmittel wie Methanol, Ethanol, n-Propanol, Wasser, Methanol/Wasser, Tetrahydrofuran oder Dioxan bei Temperaturen zwischen 0 und 150°C, vorzugsweise bei Temperaturen zwischen 20 und 120°C, mit einem entsprechenden freien Amin oder mit einem entsprechenden Säureadditionssalz wie beispielsweise Ammoniumcarbonat durchgeführt.

Eine Verbindung der allgemeinen Formel III erhält man beispielsweise durch Umsetzung eines entsprechenden Nitrils mit einem entsprechenden Alkohol wie Methanol, Ethanol, n-Propanol, Isopropanol oder Benzylalkohol in Gegenwart einer Säure wie Salzsäure oder in Gegenwart eines entsprechenden Alkoholats wie Natriummethylat oder Natriumethylat oder durch Umsetzung eines entsprechenden Amids mit einem Trialkyloxoniumsalz wie Triethyloxonium-tetrafluorborat in einem Lösungsmittel wie Methylenchlorid, Tetrahydrofuran oder Dioxan bei Temperaturen zwischen -10 und 50°C, vorzugsweise jedoch bei Temperaturen zwischen 0 und 20°C, oder eines entsprechenden Nitrils mit Schwefelwasserstoff zweckmäßigerweise in einem Lösungsmittel wie Pyridin oder Dimethylformamid und in Gegenwart einer Base wie Triethylamin und anschließender Alkylierung des gebildeten Thioamids mit einem entsprechenden Alkyl- oder Aralkylhalogenid.

c) Zur Herstellung von Verbindungen der allgemeinen Formel I, in der B eine durch eine Benzylgruppe substituierte Aminogruppe darstellt:
Umsetzung einer Verbindung der allgemeinen Formel

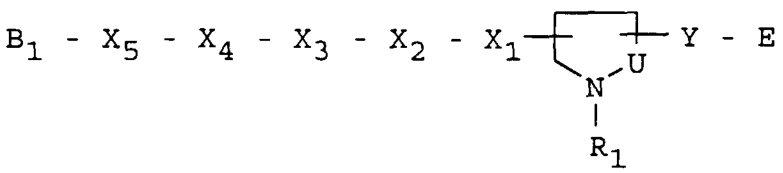

, (IV)

in der

$R_1$, $X_1$ bis $X_5$, E, U und Y wie eingangs erwähnt definiert sind und
$B_1$ eine Aminogruppe darstellt, mit einer Verbindung der allgemeinen Formel

$$Z_2 - R_b \qquad (V)$$

in der
$R_b$ eine Benzylgruppe und
$Z_2$ eine nukleophile Austrittsgruppe wie ein Halogenatom oder eine Sulfonyloxygruppe, z.B. ein Chlor-, Brom- oder Jodatom, eine Methansulfonyloxy- oder p-Toluolsulfonyloxygruppe, bedeuten.

Die Umsetzung wird zweckmäßigerweise in einem Lösungsmittel oder Lösungsmittelgemisch wie Methylenchlorid, Dimethylformamid, Dimethylsulfoxid, Toluol, Chlorbenzol, Tetrahydrofuran, Toluol/Tetrahydrofuran oder Dioxan in Gegenwart eines Alkylierungsmittels wie Methyljodid, Ethylbromid, Butylbromid, Dimethylsulfat oder Benzylchlorid vorzugsweise in Gegenwart eines säurebindenden Mittels, z.B. eines Alkoholats wie Kalium-tert.butylat, eines Alkalihydroxids wie Natrium- oder Kaliumhydroxid, eines Alkalicarbonats wie Kaliumcarbonat, eines Alkaliamids wie Natriumamid eines Alkalihydrids wie Natriumhydrid oder einer tertiären organischen Base wie N-Ethyl-diisopropylamin zweckmäßigerweise bei Temperaturen zwischen 0 und 150°C, vorzugsweise bei Temperaturen zwischen 0 und 50°C, durchgeführt.

d) Zur Herstellung von Verbindungen der allgemeinen Formel I, in der $X_2$ ein Sauerstoffatom oder eine Iminogruppe darstellt:
Umsetzung einer Verbindung der allgemeinen Formel

$$B - X_5 - X_4 - X_3 - X_2' - H , \qquad (VI)$$

in der

$X_3$ bis $X_5$ und B wie eingangs erwähnt definiert sind und
$X_2$' ein Sauerstoffatom oder eine Iminogruppe darstellt, oder deren Alkali- oder Erdalkalisalze mit einer Verbindung der allgemeinen Formel

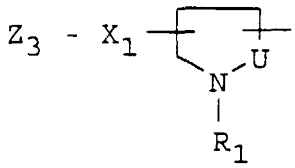 , (VII)

in der

$R_1$, $X_1$, E, U und Y wie eingangs erwähnt definiert sind und

$Z_3$ eine nukleophile Austrittsgruppe wie ein Halogenatom oder eine Sulfonyloxygruppe, z.B. ein Chlor-, Brom- oder Jodatom, eine Methansulfonyloxy- oder p-Toluolsulfonyloxygruppe bedeutet.

Die Umsetzung wird vorzugsweise in einem Lösungsmittel wie Tetrahydrofuran, Acetonitril, Dioxan, Dimethylsulfoxyd, Sulfolan, Dimethylformamid oder Dimethylacetamid gegebenenfalls in Gegenwart einer anorganischen Base wie Kaliumkarbonat, Cäsiumkarbonat, Natriumhydroxid, Kaliumhydroxid, Natriumhydrid oder Kalium-tert.butylat oder in Gegenwart einer tertiären organischen Base wie N-Ethyl-diisopropylamin, welche gegebenenfalls auch als Lösungsmittel dienen kann, und gegebenenfalls in Gegenwart eines Phasentransfer-Katalysators wie Polyethylenglykol-750-monomethylether an Polystyrol oder Hexadecyl-trimethylammoniumchlorid bei Temperaturen zwischen 0 und 180°C, vorzugsweise jedoch bei Temperaturen zwischen 10 und 160°C, durchgeführt.

e) Zur Herstellung von Verbindungen der allgemeinen Formel I, in der $R_1$ eine Pyridincarbonylgruppe darstellt: Acylierung einer Verbindung der allgemeinen Formel

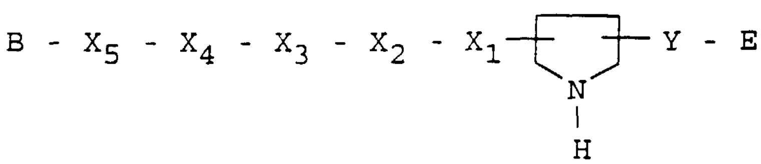 , (VIII)

in der

$X_1$ bis $X_5$, B, E und Y wie eingangs erwähnt definiert sind, mit einer Verbindung der allgemeinen Formel

$$Z_4 - R_1' \qquad (IX)$$

in der

$R_1$' eine Pyridincarbonylgruppe und

$Z_4$ eine Hydroxygruppe oder eine Austrittsgruppe wie ein Halogenatom, z.B. ein Chlor- oder Bromatom, eine Azidogruppe oder eine Acyloxygruppe, z.B. die Acetoxy-, Methoxycarbonyloxy-, Ethoxycarbonyloxy- oder Isobutoxycarbonyloxygruppe bedeuten.

Die Umsetzung wird zweckmäßigerweise in einem Lösungsmittel wie Methanol, Methylenchlorid, Chloroform, Tetrachlorkohlenstoff, Ether, Tetrahydrofuran, Dioxan, Benzol, Toluol, Acetonitril, Sulfolan oder Dimethylformamid gegebenenfalls in Gegenwart einer anorganischen oder organischen Base, gegebenenfalls in Gegenwart eines die Säure aktivierenden Mittels, gegebenenfalls in Gegenwart eines wasserentziehenden Mittels oder gegebenenfalls eines die Iminogruppe aktivierenden Mittels bei Temperaturen zwischen -20 und 200°C, vorzugsweise jedoch bei Temperaturen zwischen -10 und 160°C, durchgeführt.

Bedeutet $Z_4$ eine Hydroxygruppe, so wird die Acylierung zweckmäßigerweise in einem Lösungsmittel wie Tetrahydrofuran, Methylenchlorid, Chloroform, Sulfolan oder Dimethylformamid in Gegenwart eines die Säure aktivierenden Mittels oder eines wasserentziehenden Mittels, z.B. in Gegenwart von Chlorameisensäureäthylester, Thionylchlorid, Phosphortrichlorid, Phosphorpentoxid, N,N'-Dicyclohexylcarbodiimid, N,N'-Dicyclohexylcarbodiimid/N-Hydroxysuccinimid oder 1-Hydroxy-benztriazol, N,N'-Carbonyldiimidazol oder N,N'-Thionyldiimidazol oder Triphenylphosphin/Tetrachlorkohlenstoff, oder eines die Iminogruppe aktivierenden Mittels, z.B. Phosphortrichlorid, und gegebenenfalls in Gegenwart einer Base wie Natriumcarbonat, Kaliumcarbonat, Kalium-tert.butylat oder 1-Hydroxy-benztriazol/Triethylamin oder in Gegenwart einer tertiären organischen Base wie 4-Dimethylamino-pyridin, Triethylamin, N-Ethyl-diisopropylamin, N-Methyl-morpholin oder Pyridin, welche gleichzeitig auch als Lösungsmittel dienen können, bei Temperaturen zwischen -10 und 100°C, vorzugsweise jedoch bei Temperaturen zwischen 0 und 50°C, durchgeführt.

Die Acylierung wird jedoch vorzugsweise mit einem entsprechenden Säurehalogenid oder Säureanhydrid gegebenenfalls in Gegenwart eines säurebindenden Mittels wie vorstehend beschrieben durchgeführt.

f) Zur Herstellung von Verbindungen der allgemeinen Formel I, in der $X_2$ eine -CONH- Gruppe darstellt: Umsetzung einer Verbindung der allgemeinen Formel

$$B - X_5 - X_4 - X_3 - E_2 \qquad (X)$$

in der

$X_3$ bis $X_5$ und B wie eingangs erwähnt definiert sind und

$E_2$ eine Carboxygruppe darstellt, mit einer Verbindung der allgemeinen Formel

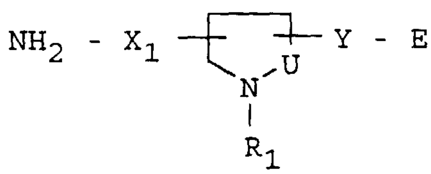
, (XI)

in der

$R_1$, $X_1$, Y, E und U wie eingangs erwähnt definiert sind,

oder mit deren reaktionsfähigen Derivaten.

Die Umsetzung wird zweckmäßigerweise in einem Lösungsmittel wie Tetrahydrofuran, Methylenchlorid, Chloroform, Acetonitril, Sulfolan oder Dimethylformamid oder mit deren Gemischen vorzugsweise in Gegenwart eines die Säure aktivierenden Mittels oder eines wasserentziehenden Mittels, z.B. in Gegenwart von Chlorameisensäureisobutylester, Thionylchlorid, Phosphortrichlorid, Phosphorpentoxid, N,N'-Dicyclohexyl-carbodiimid, N,N'-Dicyclohexylcarbodiimid/N-Hydroxysuccinimid oder 1-Hydroxy-benztriazol, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer Base wie Natriumcarbonat, Kaliumcarbonat oder Kalium-tert.butylat oder in Gegenwart einer tertiären organischen Base wie 4-Dimethylamino-pyridin, Triethylamin, N-Ethyl-diisopropylamin, N-Methyl-morpholin oder Pyridin, welche gleichzeitig als Lösungsmittel dienen können, bei Temperaturen zwischen -50 und 100°C, vorzugsweise jedoch bei Temperaturen zwischen -30 und 50°C, durchgeführt.

Die Acylierung kann jedoch auch mit einem entsprechenden Säurehalogenid oder Säureanhydrid gegebenenfalls in Gegenwart eines säurebindenden Mittels wie in Gegenwart einer anorganischen oder organischen Base durchgeführt werden.

g) Zur Herstellung von Verbindungen der allgemeinen Formel I, in der E eine Carboxygruppe oder eine Alkoxycarbonylgruppe mit insgesamt 2 bis 5 Kohlenstoffatomen darstellt: Oxidation einer Verbindung der allgemeinen Formel

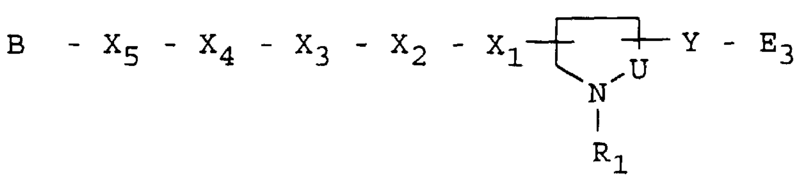
, (XII)

in der

$R_1$, B, U, $X_1$ bis $X_5$ und Y wie eingangs erwähnt definiert sind und
$E_3$ eine Vinyl- oder 1,2-Dihydroxyalkylgruppe darstellt, und erforderlichenfalls anschließende Versesterung einer so erhaltenen Verbindung mit einem entsprechenden Alkohol.

Die Oxidation wird in einem Lösungsmittel wie Methylenchlorid, Acetonitril, Acetonitril/Wasser, Methylenchlorid/Acetonitril/Wasser oder Tetrachlorkohlenstoff/Acetonitril/Wasser in Gegenwart eines Oxidationsmittels wie Kaliumpermanganat oder Rutheniumtetroxid, wobei das Rutheniumtetroxid vorzugsweise im Reaktionsgemisch durch Umsetzung eines Rutheniumsalzes wie Rutheniumtrichlorid mit einem Oxidationsmittel wie Natriumperjodat gebildet wird, bei Temperaturen zwischen -10 und 60°C, vorzugsweise bei Temperaturen zwischen 0 und 50°C, durchgeführt.

Die gegebenenfalls anschließende Veresterung wird zweckmäßigerweise in einem geeigneten Lösungsmittel, z.B. in einem entsprechenden Alkohol, Pyridin, Toluol, Methylenchlorid, Tetrahydrofuran oder Dioxan, in Gegenwart eines säureaktivierenden und/oder wasserentziehenden Mittels wie Chlorwasserstoff, konzentrierte Schwefelsäure, Thionylchlorid, Chlorameisensäureäthylester, Carbonyldiimidazol oder N,N'-Dicyclohexyl-carbodiimid oder dessen Isoharnstoffestern, gegebenenfalls in Gegenwart eines Reaktionsbeschleunigers wie Kupferchlorid, oder durch Umesterung, z.B. mit einem entsprechenden Kohlensäurediester, bei Temperaturen zwischen 0 und 100°C, vorzugsweise jedoch bei Temperaturen zwischen 20°C und der Siedetemperatur des betreffenden Lösungsmittels, durchgeführt.

h) Zur Herstellung von Verbindungen der allgemeinen Formel I, in der $R_1$ eine Pyrimidinylgruppe darstellt:
Umsetzung einer Verbindung der allgemeinen Formel

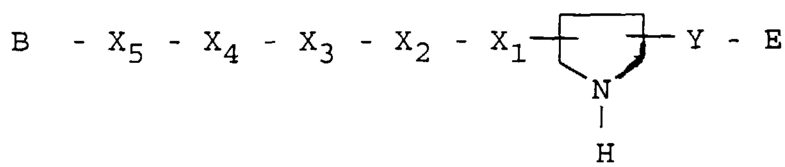 , (XIII)

in der

$X_1$ bis $X_5$, B, E und Y wie eingangs erwähntdefiniert sind, mit einer Verbindung der allgemeinen Formel

$$Z_6 - R_1'' , \qquad (XIV)$$

in der
$R_1''$ eine Pyrimidinylgruppe und
$Z_6$ eine nukleophile Austrittsgruppe wie ein Halogenatom, eine Alkylsulfenyl-, Alkylsulfinyl- oder Alkylsulfonylgruppe, z.B. ein Chlor- oder Bromatom, eine Methylsulfenyl-, Methylsulfinyl- oder Methylsulfonylgruppe, darstellt.

Die Umsetzung wird gegebenenfalls in einem Lösungsmittel wie Dioxan, Tetrahydrofuran, Acetonitril, Dimethylformamid, Dimethylacetamid oder Dimethylsulfoxid gegebenenfalls in Gegenwart einer anorganischen Base wie Natriumkarbonat oder einer organischen Base wie Pyridin, Triethylamin oder N-Ethyldiisopropylamin, welche gleichzeitig auch als Lösungsmittel dienen können, bei Temperaturen zwischen 20 und 200°C, vorzugsweise jedoch bei Temperaturen zwischen 60 und 160°C, durchgeführt.

i) Zur Herstellung von Verbindungen der allgemeinen Formel I, in der E eine Alkoxycarbonylgruppe mit insgesamt 2 bis 5 Kohlenstoffatomen darstellt:
Umsetzung einer Verbindung der allgemeinen Formel

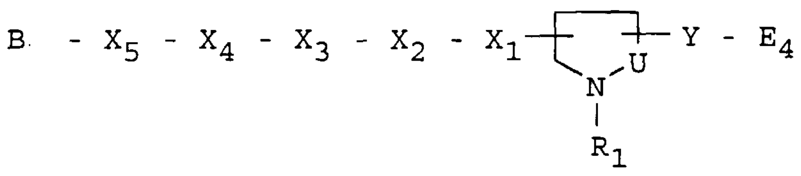 , (XV)

in der

$R_1$, B, U, $X_1$ bis $X_5$ und Y wie eingangs erwähnt definiert sind und
$E_4$ eine Carboxygruppe oder deren reaktionsfähigen Derivate darstellt, mit einer Verbindung der allgemeinen Formel

$$H - R_e ,\qquad (XVI)$$

in der
$R_e$ eine Alkoxygruppe mit 1 bis 4 Kohlenstoffatomen darstellt.

Die Umsetzung wird zweckmäßigerweise in einem Lösungsmittel wie Tetrahydrofuran, Chloroform, Methylenchlorid, Dimethylformamid oder in einem entsprechenden Alkohol gegebenenfalls in Gegenwart eines die Säure aktivierenden Mittels oder eines wasserentziehenden Mittels, z.B. in Gegenwart von Chlorameisensäureisobutylester, Thionylchlorid, Trimethylchlorsilan, Chlorwasserstoff, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid oder 1-Hydroxy-benztriazol, N,N'-Carbonyldiimidazol oder N,N'-Thionyldiimidazol oder Triphenylphosphin/Tetrachlorkohlenstoff, und gegebenenfalls in Gegenwart einer Base wie Natriumcarbonat, Kaliumcarbonat, Kalium-tert.butylat oder 1-Hydroxy-benztriazol/Triethylamin oder in Gegenwart einer tertiären organischen Base wie Triethylamin, N-Ethyl-diisopropylamin, N-Methyl-morpholin oder Pyridin, welche gleichzeitig als Lösungsmittel dienen können, bei Temperaturen zwischen -30 und 100°C, vorzugsweise jedoch bei Temperaturen zwischen -10 und 80°C, durchgeführt. Die Umsetzung kann jedoch auch mit einem entsprechenden Säurehalogenid oder Säureanhydrid gegebenenfalls in Gegenwart eines säurebindenden Mittels wie vorstehend beschrieben durchgeführt werden.

j) Zur Herstellung von Verbindungen der allgemeinen Formel I in der $X_2$ eine -CONH-Gruppe darstellt und $X_5$-$X_4$-$X_3$ eine cyclische Iminogruppe enthält, deren Ringstickstoffatom mit der Carbonylgruppe von $X_2$ verknüpft ist:
Umsetzung einer Verbindung der allgemeinen Formel

$$B - X_5 - X_4 - X_3 - H ,\qquad (XVII)$$

in der

B und $X_5$-$X_4$-$X_3$ wie eingangs erwähnt definiert sind, mit einer Verbindung der allgemeinen Formel

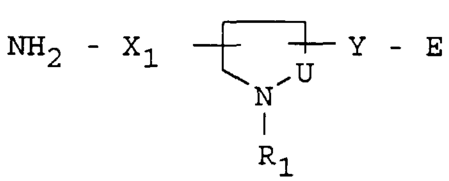 , (XVIII)

in der
$R_1$, E, U, $X_1$ und Y wie eingangs erwähnt definiert sind, in Gegenwart einer Verbindung der allgemeinen Formel

$$Z_7 - CO - Z_8 ,\qquad (XIX)$$

in der

$Z_7$ und $Z_8$, die gleich oder verschieden sein können, nukleophile Austrittsgruppen wie Halogenatome oder eine N-Azolylgruppe, z.B. ein Chloratom, eine N-Imidazolyl- oder N-(1,2,4-Triazolyl)-Gruppe, bedeuten.

Die Umsetzung wird vorzugsweise in Gegenwart von Phosgen oder N,N'-Carbonyldiimidazol zweckmäßigerweise in einem Lösungsmittel wie Tetrahydrofuran, Dioxan, Acetonitril, Methylenchlorid oder Dimethylformamid in Gegenwart einer organischen Base wie Imidazol, Triethylamin oder Pyridin, welche auch als Lösungsmittel dienen können, bei Temperaturen zwischen -20 und 60°C, vorzugsweise bei Temperaturen zwischen -10 und 30°C, durchgeführt.

Die Umsetzung wird jedoch vorzugsweise so durchgeführt, daß eine der Verbindungen der allgemeinen Formel XXI oder XXII mit einer der oben erwähnten Verbindungen der allgemeinen Formel XXIII umgesetzt wird und dann im Reaktionsgemisch mit der anderen Verbindung der Formel XXI oder XXII umgesetzt wird.

k) Zur Herstellung von Verbindungen der allgemeinen Formel I, in der $X_2$ eine Iminogruppe und $X_3$ eine der eingangs erwähnten Heteroarylengruppen darstellt:

Umsetzung einer Verbindung der allgemeinen Formel

$$B - X_5 - X_4 - X_3' - Z_9 , \qquad (XX)$$

in der

B, $X_4$ und $X_5$ wie eingangs erwähnt definiert sind,

$X_3'$ eine der für $X_3$ eingangs erwähnten Heteroarylengruppen und

$Z_9$ eine nukleophile Austrittsgruppe wie ein Halogenatom, eine Alkylsulfenyl-, Alkylsulfinyl- oder Alkylsulfonylgruppe, z.B. ein Chlor- oder Bromatom, eine Methylsulfenyl-, Methylsulfinyl- oder Methylsulfonylgruppe, bedeuten, mit einer Verbindung der allgemeinen Formel

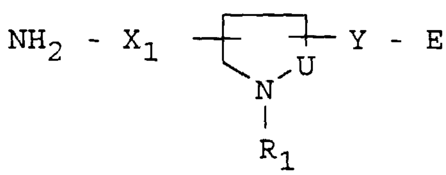

, (XVIII)

in der

$R_1$, E, U, Y und $X_1$ wie eingangs erwähnt definiert sind.

Die Umsetzung wird zweckmäßigerweise in einem geeigneten Lösungsmittel wie Dimethylsulfoxid, Dimethylformamid, Dimethylacetamid, Dioxan oder Toluol gegebenenfalls in Gegenwart einer anorganischen Base wie Natriumcarbonat oder Natriumhydrogencarbonat oder einer tertiären organischen Base wie Pyridin, Triethylamin oder N-Ethyl-diisopropylamin, welche gleichzeitig auch als Lösungsmittel dienen können, bei Temperaturen zwischen 20 und 180°C, vorzugsweise jedoch bei Temperaturen zwischen 60 und 140°C, durchgeführt.

l) zur Herstellung von Verbindungen der allgemeinen Formel I, in der $X_2$ eine Bindung, $X_3$ eine der eingangs erwähnten Heteroarylengruppen darstellt und $X_3$ mit dem Ringstickstoffatom einer -CO-N-Gruppe an $X_1$ gebunden ist:

Umsetzung einer Verbindung der allgemeinen Formel

$$B - X_5 - X_4 - X_3'' - H , \qquad (XXI)$$

in der

B, $X_4$ und $X_5$ wie eingangs erwähnt definiert sind und

$X_3''$ eine der für $X_3$ eingangs erwähnten Heteroarylengruppen, in der eine oder zwei zu einem Stickstoffatom benachbarte Methingruppen jeweils durch eine Hydroxymethingruppe ersetzt sind, darstellt, oder deren Tautomere mit einer Verbindung der allgemeinen Formel

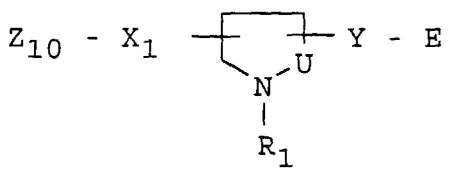

, (XXII)

in der

$R_1$, E, U, $X_1$ und Y wie eingangs erwähnt definiert sind und

$Z_{10}$ eine nukleophile Austrittsgruppe wie ein Halogenatom oder eine Sulfonyloxygruppe, z.B. ein Chlor- oder Bromatom, eine Methansulfonyloxy- oder p-Toluolsulfonyloxygruppe, bedeutet.

Die Umsetzung wird zweckmäßigerweise in einem geeigneten Lösungsmittel wie Dimethylformamid, Dimethylacetamid, Dimethylsulfoxid, N-Methyl-pyrrolidon, Tetrahydrofuran oder Dioxan vorzugsweise in Gegenwart einer Base wie Natriumhydrid, Kalium-tert.butylat, Kaliumcarbonat oder Natriumhydrogencarbonat bei Temperaturen zwischen 0 und 160°C, vorzugsweise bei Temperaturen zwischen Raumtemperatur und 120°C, durchgeführt.

m) Zur Herstellung von Verbindungen der allgemeinen Formel I, in der B, $B-X_5-X_4-X_3$, $B-X_5-X_4$ oder $B-X_5$ eine durch eine Alkoxycarbonylgruppe mit insgesamt 2 bis 5 Kohlenstoffatomen substituierte Amidinogruppe enthält:

Umsetzung einer Verbindung der allgemeinen Formel

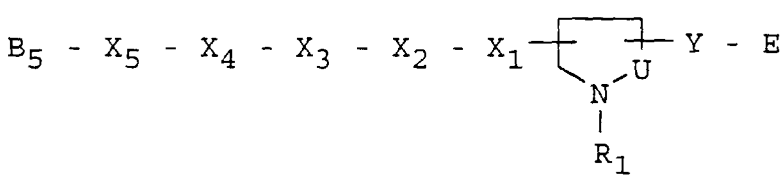

, (XXIII)

in der

$R_1$, E, U, $X_1$ bis $X_5$ und Y wie eingangs erwähnt definiert sind und

$B_5$, $B_5-X_5-X_4-X_3$, $B_5-X_5-X_4$ oder $B_5-X_5$ eine Amidinogruppe enthält, mit einer Verbindung der allgemeinen Formel

$$Z_{11} - R_f , \qquad \text{(XXIV)}$$

in der

$R_f$ eine Alkoxycarbonylgruppe mit insgesamt 2 bis 5 Kohlenstoffatomen und

$Z_{11}$ eine nukleophile Austrittsgruppe wie ein Halogenatom oder eine Aryloxygruppe, z. B. ein Chlor- oder Bromatom oder eine p-Nitrophenoxygruppe, bedeuten.

Die Umsetzung wird zweckmäßigerweise in einem Lösungsmittel wie Tetrahydrofuran, Methylenchlorid, Chloroform, Essigester oder Dimethylformamid zweckmäßigerweise in Gegenwart einer Base wie Natriumcarbonat, Kaliumcarbonat oder Natronlauge oder in Gegenwart einer tertiären organischen Base wie Triethylamin, N-Ethyl-diisopropylamin, N-Methyl-morpholin oder Pyridin, welche gleichzeitig als Lösungsmittel dienen können, bei Temperaturen zwischen -30 und 100°C, vorzugsweise jedoch bei Temperaturen zwischen -10 und 60°C, durchgeführt.

n) Zur Herstellung von Verbindungen der allgemeinen Formel I, in der E eine Alkoxycarbonylgruppe mit insgesamt 2 bis 5 Kohlenstoffatomen darstellt:

Umsetzung einer Verbindung der allgemeinen Formel

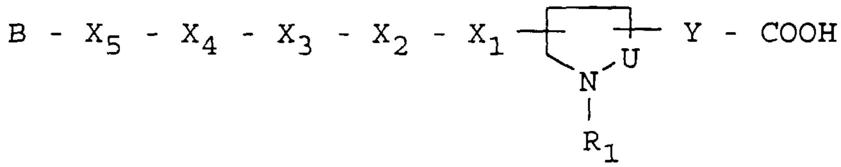 , (XXV)

in der

$R_1$, B, U, $X_1$ bis $X_5$ und Y wie eingangs erwähnt definiert sind, mit einer Verbindung der allgemeinen Formel

$$Z_{12} - E_5 , \quad (XXVI)$$

in der
$E_5$ eine Alkylgruppe mit 1 bis 4 Kohlenstoffatomen und
$Z_{12}$ eine nukleophile Austrittsgruppe wie ein Halogenatom, z. B. ein Chlor-, Brom- oder Jodatom, eine Methansulfonyloxy- oder p-Toluolsulfonyloxygruppe, darstellen.

Die Umsetzung wird zweckmäßigerweise in einem Lösungsmittel wie Methylenchlorid, Tetrahydrofuran, Dioxan, Dimethylsulfoxid oder Dimethylformamid gegebenenfalls in Gegenwart eines Reaktionsbeschleunigers wie Natrium- oder Kaliumjodid und vorzugsweise in Gegenwart einer Base wie Natriumcarbonat, Kaliumcarbonat oder Natronlauge oder in Gegenwart einer tertiären organischen Base wie N-Ethyl-diisopropylamin oder N-Methylmorpholin, welche gleichzeitig auch als Lösungsmittel dienen können, oder gegebenenfalls in Gegenwart von Silberkarbonat oder Silberoxid bei Temperaturen zwischen -30 und 100°C, vorzugsweise jedoch bei Temperaturen zwischen -10 und 80°C, durchgeführt.

Erhält man erfindungsgemäß eine Verbindung der allgemeinen Formel I, die eine Carbonylbrücke enthält, so kann diese mittels Reduktion in eine entsprechende Verbindung der allgemeinen Formel I, die eine Methylenbrücke enthält, übergeführt werden und/oder
eine Verbindung der allgemeinen Formel I, die eine Estergruppe enthält, so kann diese mittels Umesterung in einen entsprechenden Ester übergeführt werden und/oder

Die nachträgliche Reduktion einer Carbonylbrücke wird vorzugsweise in einem Lösungsmittel wie Methanol, Ethanol oder Isopropanol gegebenenfalls in Gegenwart einer Säure wie Salzsäure oder Essigsäure mit Wasserstoff in Gegenwart eines Katalysators wie Platin oder Palladium/Kohle bei Temperaturen zwischen 0 und 100°C, vorzugsweise jedoch bei Temperaturen zwischen 20°C und 80°C, durchgeführt.

Die nachträgliche Umsetzung einer Estergruppe mit einem Alkohol wird vorzugsweise in einem entsprechenden Alkohol als Lösungsmittel gegebenenfalls in Gegenwart eines weiteren Lösungsmittel wie Methylenchlorid oder Ether vorzugsweise in Gegenwart einer Säure wie Salzsäure bei Temperaturen zwischen 0 und 100°C, vorzugsweise bei Temperaturen zwischen 20 und 80°C, durchgeführt.

Bei den vorstehend beschriebenen Umsetzungen können gegebenenfalls vorhandene reaktive Gruppen wie Hydroxy-, Carboxy-, Amidino-, Amino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, tert.Butyl-, Trityl-, Benzyl- oder Tetrahydropyranylgruppe,

als Schutzreste für eine Carboxylgruppe die Trimethylsilyl-, Methyl-, Ethyl-, tert.Butyl-, Benzyl- oder Tetrahydropyranylgruppe,

als Schutzreste für eine Amidinogruppe die Benzyloxycarbonylgruppe,

als Schutzrest für eine Amino- oder Iminogruppe die Acetyl-, Benzoyl-, Ethoxycarbonyl-, tert.Butoxycarbonyl-, Benzyloxycarbonyl-, Benzyl- oder Methoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Methanol/Wasser, Isopropanol/Wasser, Tetrahydrofuran/Wasser oder Dioxan/Wasser, in Gegenwart einer Säure wie Trifluoressigsäure, Salzsäure oder Schwefelsäure oder in

Gegenwart einer Alkalibase wie Natriumhydroxid oder Kaliumhydroxid oder mittels Etherspaltung, z.B. in Gegenwart von Jodtrimethylsilan, bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Dioxan, Essigsäureethylester oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50°C, vorzugsweise jedoch bei Raumtemperatur, erfolgen.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan, Essigsäureethylester oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971)) in ihre optischen Antipoden und Verbindungen der allgemeinen Formel I mit mindestens 2 asymmetrischen Kohlenstoffatomen auf Grund ihrer physikalischen-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze bildenden optisch aktiven Substanz, insbesondere Säuren, und Trennen des auf diese Weise erhaltenen diastereomeren Salzgemisches, z.B. auf Grund von verschiedenen Löslichkeiten, wobei aus den reinen diastereomeren Salzen die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure.

Desweiteren können die erhaltenen Verbindungen der Formel I in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der allgemeinen Formel I, falls diese eine Carboxylgruppe enthalten, gewünschtenfalls anschließend in ihre Additionssalze mit anorganischen oder organischen Basen, insbesondere für die pharmazeutische Anwendung in ihre physiologisch verträglichen Additionssalze, überführen. Als Basen kommen hierbei beispielsweise Natriumhydroxid, Kaliumhydroxid, Cyclohexylamin, Äthanolamin, Diäthanolamin und Triäthanolamin in Betracht.

Die als Ausgangsstoffe verwendeten Verbindungen der Formeln II bis XXVI sind teilweise literaturbekannt oder man erhält diese nach literaturbekannten Verfahren (siehe Beispiele).

Wie bereits eingangs erwähnt, weisen die neuen cyclischen Iminoderivate der allgemeinen Formel I und deren Additionssalze, insbesondere deren physiologisch verträgliche Additionssalze mit anorganischen oder organischen Säuren oder Basen, wertvolle Eigenschaften auf. So weisen die Verbindungen der allgemeinen Formel I, in denen B-$X_5$-$X_4$ eine Isochinolinylgruppe darstellt oder B, B-$X_5$, B-$X_5$-$X_4$- oder B-$X_5$-$X_4$-$X_3$ eine eine gegebenenfalls substituierte Amino- oder Iminogruppe oder eine gegebenenfalls in vivo in eine gegebenenfalls substituierte Amino- oder Iminogruppe überführbare Gruppe, z.B. eine durch eine Alkoxycarbonylgruppe substituierte Amino- oder Iminogruppe, enthält und Y-E eine Carboxylgruppe oder eine in vivo in eine Carboxylgruppe überführbare Gruppe, z.B. eine durch eine Alkoxygruppe substituierte Carbonylgruppe, enthält, wertvolle pharmakologische Eigenschaften auf, neben einer entzündungshemmenden und den Knochenabbau hemmenden Wirkung insbesondere antithrombotische, antiaggregatorische und tumor- bzw. metastasenhemmende Wirkungen. Die übrigen Verbindungen der allgemeinen Formel I stellen insbesondere wertvolle Zwischenprodukte zur Herstellung der vorstehend erwähnten pharmakologisch wirksamen Verbindungen dar.

Beispielsweise wurden die Verbindungen der allgemeinen Formel I auf ihre biologischen Wirkungen wie folgt untersucht:

1. Fibrinogen-Bindung an Humanthrombozyten

Das durch Punktion einer Antekubitalvene gewonnene Blut wird mit Trinatriumcitrat (Endkonzentration: 13 mM) antikoaguliert und 10 Minuten bei 170 *g zentrifugiert. Das überstehende plättchenreiche Plasma wird auf eine Sepharose 2B-Säule (Pharmacia) gegeben und mit einer Lösung aus 90 mM Kochsalz, 14 mM Trinatriumcitrat, 5 mM Glucose und 50 mM Tris(hydroxymethyl)aminomethan, eingestellt auf pH 7,4, eluiert. Die vor den Plasmaproteinen erscheinenden gelfiltrierten Plättchen (GFP) werden für die Bindungsversuche verwendet.

50 µl einer 60 mM Calziumchlorid-Lösung, 50 µl einer 0,6 mM Adenosindiphosphat-Lösung, 100 µl Substanzlösung bzw. Lösungsmittel und 50 µl Fibrinogenlösung (enthaltend 3 µg $^{125}$J-Fibrinogen) werden zu 750 µl GFP gegeben und bei Raumtemperatur 20 Minuten inkubiert. Die unspezifische Bindung wird in Gegenwart von 3 mg/ml kaltem Fibrinogen bestimmt.

900 µl des Inkubates werden vorsichtig auf 250 µl Silikonöl (AP 38: AR 20, 1:2 v/v, Wacker Chemie) in Eppendorf-Gefäße pipettiert und 2 Minuten bei 10 000 *g zentrifugiert. Der wäßrige Überstand und ein Teil des Öls werden abgezogen, die Gefäßspitze mit dem Plättchenpellet abgeschnitten und im Gamma-Zähler die Menge des gebundenen Fibrinogens bestimmt. Aus einer Konzentrationsreihe wird die Substanzkonzentration ermittelt, welche die Fibrinogenbindung zu 50 % hemmt und als $IC_{50}$ angegeben.

2. Antithrombotische Wirkung

Methodik

Die Thrombozytenaggregation wird nach der Methode von Born und Cross (J. Physiol 170, 397 (1964)) in plättchenreichem Plasma gesunder Versuchspersonen gemessen. Zur Gerinnungshemmung wird das Blut mit Natriumcitrat 3,14 % im Volumenverhältnis 1:10 versetzt.

Collagen-induzierte Aggregation

Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density".

Die Collagen-Menge wird möglichst gering gewählt, aber doch so, daß sich eine irreversibel verlaufende Reaktionskurve ergibt. Verwendet wird das handelsübliche Collagen der Firma Hormonchemie, München.

Vor der Collagen-Zugabe wird das Plasma jeweils 10 Minuten mit der Substanz bei 37°C inkubiert.

Aus den erhaltenen Meßzahlen wird graphisch eine $EC_{50}$ bestimmt, die sich auf eine 50%ige Änderung der "optical density" im Sinne einer Aggregationshemmung bezieht.

Die nachfolgende Tabelle enthält die gefundenen Ergebnisse:

| Substanz (Beispiel Nr.) | Fibrinogen-Bindungstest $IC_{50}$[µM] | Hemmung der Plättchenaggregation $EC_{50}$[µM] |
|---|---|---|
| 1 | 0,010 | 0,06 |
| 1(3) | 0,042 | 0,10 |
| 4(2) | 0,200 | 0,54 |
| 4(3) | 0,160 | 38,00 |
| 4(4) | 0,140 | 0,21 |
| 4(6) | 0,031 | 0,16 |
| 4(9) | 0,032 | 0,10 |
| 4(11) | 0,059 | 0,59 |

(fortgesetzt)

| Substanz (Beispiel Nr.) | Fibrinogen-Bindungstest $IC_{50}$[μM] | Hemmung der Plättchenaggregation $EC_{50}$[μM] |
|---|---|---|
| 4(13) | 0,590 | 3,00 |
| 4(16) | 0,055 | 0,43 |
| 11 | 0,270 | 0,89 |
| 12(4) | 0,570 | 3,80 |

Außerdem hemmt beispielsweise die Verbindung des Beispiels 5 die Collagen-induzierte Thrombozytenaggregation ex vivo am Rhesusaffen nach oraler Gabe von 1 mg/kg länger als 8 Stunden.

Die neuen Verbindungen sind gut verträglich, da beispielsweise nach intravenöser Gabe von 30 mg/kg der Verbindungen der Beispiele 1 und 4(16) an jeweils 3 Mäusen kein Tier verstarb.

Auf Grund ihrer Hemmwirkung auf Zell-Zell- bzw. Zell-Matrix-Wechselwirkungen eignen sich die neuen cyclischen Iminoderivate der allgemeinen Formel I und ihre physiologisch verträglichen Additionssalze zur Bekämpfung bzw. Verhütung von Krankheiten, bei denen kleinere oder größere Zell-Aggregate auftreten oder Zell-Matrixinteraktionen eine Rolle spielen, z.B. bei der Bekämpfung bzw. Verhütung von venösen und arteriellen Thrombosen, von zerebrovasculären Erkrankungen, von Lungenembolien, des Herzinfarkts, der Arteriosklerose, der Osteoporose und der Metastasierung von Tumoren und der Therapie genetisch bedingter oder auch erworbener Störungen der Interaktion von Zellen untereinander oder mit soliden Strukturen. Weiterhin eignen sich diese bei der Begleittherapie bei der Thrombolyse mit Fibrinolytica oder Gefäßinterventionen wie transluminaler Angioplastie oder auch bei der Therapie von Schockzuständen, der Psoriasis, des Diabetes und von Entzündungen.

Für die Bekämpfung bzw. Verhütung der vorstehend erwähnten Krankheiten liegt die Dosis zwischen 0,1 μg und 30 mg/kg Körpergewicht, vorzugsweise bei 1 μg bis 15 mg/kg Körpergewicht, bei bis zu 4 Gaben pro Tag. Hierzu lassen sich die erfindungsgemäß hergestellten Verbindungen der Formel I, gegebenenfalls in Kombination mit anderen Wirksubstanzen wie Thromboxan-Rezeptor-Antagonisten und Thromboxansynthesehemmer oder deren Kombinationen, Serotonin-Antagonisten, α-Rezeptorantagonisten, Alkylnitrate wie Glycerintrinitrat, Phosphodiesterasehemmer, Prostacyclin und deren Analoga, Fibrinolytica wie tPA, Prourokinase, Urokinase, Streptokinase, oder Antikoagulantien wie Heparin, Dermatansulfat, aktiviertes Protein C, Vitamin K-Antagonisten, Hirudin, Inhibitoren des Thrombins oder anderer aktivierter Gerinnungsfaktoren, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Stearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen, Lösungen, Sprays oder Zäpfchen einarbeiten.

Die nachfolgenden Beispiele sollen die Erfindung näher erläutern:

Beispiel I

2-tert.Butyloxycarbonyl-5-isoindolincarbonsäure

6,64 g 5-Isoindolincarbonsäure-hydrochlorid in 80 ml Dioxan/Wasser (2:1) werden mit 70 ml 1N Natronlauge und dann mit 8,72 g Pyrokohlensäure-di-tert.butylester versetzt und 3 1/2 Stunden bei Raumtemperatur gerührt. Das Reaktionsgemisch wird etwas eingeengt, mit gesättigter wäßriger Kaliumhydrogensulfat-Lösung auf pH 2 eingestellt und mit Essigester mehrmals extrahiert. Die vereinigten organischen Phasen werden mit gesättigter Kochsalzlösung gewaschen, getrocknet und einrotiert. Das Rohprodukt wird in 400 ml Essigester gelöst, und dreimal mit je 20 ml Wasser gewaschen. Die organische Phase wird mit Magnesiumsulfat und Aktivkohle gerührt, dann filtriert und eingeengt.

Ausbeute: 6,0 g (71 % der Theorie),
Schmelzpunkt: 175-177°C (Zers.)
$R_f$-Wert: 0,48 (Kieselgel; Essigester)

Analog werden folgende Verbindungen erhalten:

(1) 7-Acetyl-2-tert.butyloxycarbonyl-1,2,4,5-tetrahydroisochinolin

$R_f$-Wert: 0,68 (Kieselgel; Cyclohexan/Essigester = 1:1)

(2) 4-(1-tert.Butyloxycarbonyl-4-piperidinyl)benzoesäure

$R_f$-Wert: 0,34 (Kieselgel; Methylenchlorid/Cyclohexan/Methanol/konz. wäßriges Ammoniak = 68:15:15:2)

(3) 4-(1-tert.Butyloxycarbonyl-4-piperidinyl)phenol

$R_f$-Wert: 0,63 (Kieselgel; Methylenchlorid/Ethanol = 15:1)

Beispiel II

4-(5-Cyano-2-pyridyl)phenol

3,4 g 4-(5-Cyano-2-pyridyl)anisol und 35 g Pyridin-hydrochlorid werden 2 1/2 Stunden bei 180°C gerührt. Nach dem Abkühlen wird mit Wasser digeriert und der Niederschlag abgesaugt. Der Niederschlag wird in Essigester gelöst, getrocknet und eingeengt.

Ausbeute: 2,3 g (73 % der Theorie),
Schmelzpunkt: 172-175°C
$R_f$-Wert: 0,48 (Kieselgel; Methylenchlorid/Essigester = 9:1)

Analog werden folgende Verbindungen erhalten:

(1) 4-(2-Cyano-5-pyridyl)phenol

Schmelzpunkt: 191-193°C
$R_f$-Wert: 0,65 (Kieselgel, Methylenchlorid/Essigester = 9:1)

(2) 4-(5-Cyano-2-pyrazinyl)phenol

Schmelzpunkt: 205-209°C
$R_f$-Wert: 0,45 (Kieselgel; Methylenchlorid/Essigester = 9:1)

(3) 4-(5-Cyano-2-thiazolyl)phenol

Schmelzpunkt: 218-220°C
$R_f$-Wert: 0,44 (Kieselgel; Methylenchlorid/Essigester = 9:1)

(4) 6-(4-Hydroxyphenyl)isochinolin

$R_f$-Wert: 0,50 (Kieselgel; Essigester/Cyclohexan = 7:1)

Beispiel III

4-(5-Cyano-2-pyridyl)anisol

9 g 4-(5-Brom-2-pyridyl)anisol, 7,7 g Kupfer(I)cyanid und 45 ml trockenes Dimethylformamid werden 6 Stunden unter Rückfluß erhitzt. Nach dem Abkühlen wird eingeengt und der Rückstand zwischen 180 ml 6N Salzsäure und Essigester verteilt.

Die organische Phase wird abgetrennt, die wäßrige Phase zweimal mit Essigester extrahiert und die vereinigten organischen Phasen mit Wasser und verdünntem wäßrigen Ammoniak gewaschen. Die organische Phase wird getrocknet und eingeengt.

Ausbeute: 4,4 g (62 % der Theorie),
Schmelzpunkt: 103-105°C
$R_f$-Wert: 0,44 (Kieselgel; Methylenchlorid)

| | | | |
|---|---|---|---|
| Ber.: | C 74,27 | H 4,79 | N 13,33 |
| Gef.: | 73,98 | 4,58 | 13,04 |

Analog wird folgende Verbindung erhalten:

(1) 4-(5-Cyano-2-pyrazinyl)anisol

Schmelzpunkt: 132-134°C
$R_f$-Wert: 0,68 (Kieselgel; Methylenchlorid)

Beispiel IV

4-(2-Cyano-5-pyridyl)anisol

1,75 g 3-(4-Methoxyphenyl)pyridin-N-oxid, 3,5 ml Trimethylsilylcyanid, 2,4 ml Triethylamin und 10 ml Acetonitril werden 8 Stunden unter Rückfluß erhitzt. Nach Zugabe von 2 ml Trimethylsilylcyanid wird weitere 8 Stunden unter Rückfluß erhitzt. Nach dem Abkühlen wird mit 3M Natriumcarbonat-Lösung versetzt und mit Methylenchlorid extrahiert. Die organische Phase wird getrocknet und eingeengt. Der Rückstand wird durch Kieselgelchromatographie mit Cyclohexan/Essigester (3:1) gereinigt.

Ausbeute: 1,05 g (57 % der Theorie),
Schmelzpunkt: 120-122°C
$R_f$-Wert: 0,33 (Kieselgel; Cyclohexan/Essigester = 4:1)

| | | | |
|---|---|---|---|
| Ber.: C | 74,27 | H 4,79 | N 13,33 |
| Gef.: | 74,51 | 4,86 | 13,28 |

Beispiel V

3-(4-Methoxyphenyl)pyridin-N-oxid

2,4 g 3-(4-Methoxyphenyl)pyridin, 10 ml Eisessig und 3 ml 35%iges wäßriges Wasserstoffperoxid werden 4 Stunden bei 60°C und 5 Stunden bei 100°C gerührt. Nach dem Abkühlen werden 10 ml Wasser zugesetzt und der Ansatz auf die Hälfte eingeengt. Nach Versetzen mit Eiswasser wird mit Natronlauge neutralisiert und der Niederschlag abgesaugt. Das Filtrat wird mit Methylenchlorid extrahiert und der Niederschlag in Methylenchlorid aufgenommen. Die vereinigten Methylenchlorid-Lösungen werden getrocknet und einrotiert. Nach Reinigung über eine Kieselgelsäule mit Essigester/Methanol (4:1) verbleiben 1,9 g (73 % der Theorie).

Schmelzpunkt: 109-110°C
$R_f$-Wert: 0,70 (Kieselgel; Essigester/Methanol = 4:1)

| | | | |
|---|---|---|---|
| Ber.: | C 71,62 | H 5,51 | N 6,96 |
| Gef.: | 71,30 | 5,50 | 6,85 |

Beispiel VI

2-Brom-5-(4-methoxyphenyl)pyrazin

26,1 g 5-(4-Methoxyphenyl)-2(1H)pyrazinon und 140 g Phosphorxybromid werden 45 Minuten auf 100°C erhitzt. Nach dem Abkühen wird die erstarrte Masse portionsweise unter Rühren auf Eis und Wasser gegeben. Nach 30 Minuten Rühren wird 18 Stunden stehengelassen und der Niederschlag abgesaugt. Das Filtrat wird mit Methylenchlorid extrahiert und der Niederschlag im Methylenchlorid-Extrakt gelöst. Die Methylenchloridlösung wird mit Natriumbicarbonatlösung gewaschen, getrocknet, über Aktivkohle filtriert und einrotiert.

Ausbeute: 23,5 g (69 % der Theorie),
Schmelzpunkt: 140-141°C

Beispiel VII

5-Isoindolincarbonsäure-hydrochlorid

6,83 g 2-Acetyl-5-isoindolincarbonsäure x 0,2 $H_20$ werden in 160 ml halbkonzentrierter Salzsäure 16 Stunden am Rückfluß erhitzt. Danach wird abgekühlt und zur Trockene eingeengt. Das Rohprodukt wird ohne weitere Reinigung in Beispiel I eingesetzt.

$R_f$-Wert: 0,11 (Kieselgel; Methylenchlorid/Methanol = 10:1)

Analog werden folgende Verbindungen erhalten:

(1) 4-(4-Piperidinyl)benzoesäure-hydrochlorid

Schmelzpunkt: > 230°C

(2) 4-(4-Piperidinyl)phenol

$R_f$-Wert: 0,30 (Kieselgel; Methylenchlorid/Methanol/konz. wäßriges Ammoniak = 4:1:0,25)

Beispiel VIII

1-Amino-6-(4-hydroxyphenyl)isochinolin

2,5 g 1-Amino-6-(4-benzyloxyphenyl)isochinolin in 50 ml Dimethylformamid werden mit 2,5 g Palladium/Aktivkohle (10 % Palladium) bei Raumtemperatur und dann bei 50°C unter einem Wasserstoffdruck von 3,4 bar 3 Stunden hydriert. Dann wird der Katalysator abgesaugt, der Ansatz zur Trockene einrotiert und der Rückstand durch Chromatographie über eine Kieselgelsäule mit Methylenchlorid/Methanol (4:1) gereinigt.

Ausbeute: 0,5 g (28 % der Theorie),
$R_f$-Wert: 0,20 (Kieselgel; Methylenchlorid/Methanol = 4:1)

Analog werden folgende Verbindungen erhalten:

(1) 4-[(2-Benzylamino-4-pyridyl)aminocarbonyl]phenol

$R_f$-Wert: 0,26 (Kieselgel; Methylenchlorid/Methanol = 9:1) und
4-[(2-Benzylamino-6-chlor-4-pyridyl)aminocarbonyl]phenol
$R_f$-Wert: 0,58 (Kieselgel; Methylenchlorid/Methanol = 9:1) durch Hydrierung von 4-[(2-Benzylamino-6-chlor-4-pyridyl)aminocarbonyl]-1-benzyloxy-benzol bei Raumtemperatur.

Beispiel IX

1-Amino-6-(4-benzyloxyphenyl)isochinolin

Zu 26,2 ml N,N,N',N'-Tetramethylethylendiamin und 30 ml trockenem Xylol werden 7,1 g Natriumamid und 10,8 g 6-(4-Benzyloxyphenyl)isochinolin gegeben und 18 Stunden bei 120°C gerührt. Anschließend wird auf 0°C abgekühlt und vorsichtig tropfenweise mit 20 ml Eiswasser versetzt. Der Ansatz wird zur Trockene eingeengt und der Rückstand mit 150 ml Methanol aufgekocht. Nach dem Abkühlen wird der Niederschlag abgesaugt und getrocknet.

Ausbeute: 4,6 g (41 % der Theorie),
$R_f$-Wert: 0,17 (Kieselgel; Methylenchlorid/Methanol = 10:1)

Beispiel X

6-(4-Benzyloxyphenyl)isochinolin

13,3 g 6-Trifluormethylsulfonyloxy-isochinolin, 15,1 g 4-Benzyloxyphenylboronsäureanhydrid, 2,8 g Tetrakistriphenylphosphinpalladium, 72 ml 2M wäßrige Natriumcarbonatlösung und 150 ml 1,2-Dimethoxyethan werden 24 Stunden unter Stickstoff am Rückfluß gekocht. Nach dem Abkühlen werden 200 ml Wasser zugegeben, der Niederschlag wird abgesaugt, mit Wasser gewaschen und getrocknet. Nach Reinigung über eine Kieselgelsäule mit Methylenchlorid/Methanol (20:1) werden 11,8 g (79 % der Theorie) erhalten.

Schmelzpunkt: 167-168°C
$R_f$-Wert: 0,48 (Kieselgel; Methylenchlorid/Methanol = 20:1)

| | | | |
|---|---|---|---|
| Ber.: | C 84,86 | H 5,50 | N 4,50 |
| Gef.: | 84,78 | 5,52 | 4,42 |

Analog wird folgende Verbindung erhalten:

(1) 6-(4-Methoxyphenyl)isochinolin

$R_f$-Wert; 0,57 (Kieselgel; Essigester/Cyclohexan = 7:1)

Beispiel XI

4-Benzyloxyphenylboronsäureanhydrid

Zu einer Lösung von 80 g Borsäuretriisopropylester in 300 ml Tetrahydrofuran/Toluol (1:1) wird bei -70°C eine Grignard-Lösung, hergestellt aus 55,5 g 4-Benzyloxybrombenzol und 5,3 g Magnesium in 300 ml Tetrahydrofuran/Toluol (4:1), innerhalb von 45 Minuten zugetropft. Der Ansatz wird über Nacht langsam auf Raumtemperatur erwärmt und dann auf eine Mischung aus 200 g Eis und 100 ml konz. Salzsäure gegeben. Nach einer Stunde Rühren wird die organische Phase abgetrennt und die wäßrige Phase fünfmal mit insgesamt 1 l Methylenchlorid/Methanol (9:1) extrahiert. Die vereinigten organischen Phasen werden getrocknet und einrotiert und der Rückstand durch Chromatographie über eine Kieselgelsäule mit Methylenchlorid/Essigester (4:1) und Methylenchlorid/Methanol (4:1) gereinigt.

Ausbeute: 19,1 g (43,1 % der Theorie),
Schmelzpunkt: 199-201°C
$R_f$-Wert: 0,28 (Kieselgel, Methylenchlorid/Essigester = 4:1)

| | | |
|---|---|---|
| Ber.: | C 74,34 | H 5,28 |
| Gef.: | 74,32 | 5,31 |

Beispiel XII

6-Trifluormethylsulfonyloxy-isochinolin

10 g 6-Hydroxyisochinolin werden in 150 ml trockenem Pyridin bei 50°C gelöst und auf -20°C abgekühlt. Dann werden unter starkem Rühren 11,6 ml Trifluormethansulfonsäureanhydrid innerhalb von 15 Minuten zugetropft. Es wird noch 30 Minuten bei -20°C und dann 16 Stunden bei 0°C gerührt. Das Reaktionsgemisch wird einrotiert und der Rückstand mehrmals mit Toluol abrotiert. Der Rückstand wird in 250 ml tert.Butyl-methylether aufgenommen und fünfmal mit 20 ml Wasser ausgeschüttelt. Die organische Phase wird abgetrennt, getrocknet und einrotiert und der Rückstand durch Chromatographie über eine Kieselgelsäule mit Essigester gereinigt.

Ausbeute: 15,9 g (84 % der Theorie),
$R_f$-Wert: 0,76 (Kieselgel; Essigester/Cyclohexan = 9:1)

| | | | | |
|---|---|---|---|---|
| Ber.: | C 43,33 | H 2,18 | N 5,05 | S 11,56 |
| Gef.: | 43,29 | 2,35 | 5,15 | 11,62 |

Beispiel XIII

2-Acetyl-5-isoindolincarbonsäure x 0,2 $H_2O$

1,76 g 2,5-Diacetylisoindolin werden in einer Lösung von 5,2 g Natriumhydroxid in 50 ml Wasser suspendiert. Unter starkem Rühren wird bei 10°C innerhalb von 20 Minuten 1,4 ml Brom zugetropft. Das Reaktionsgemisch wird 20 Minuten bei 10°C und 15 Minuten bei 20°C gerührt. Dann wird 100 ml Wasser zugegeben, das abgeschiedene Bromoform abgetrennt und die wäßrige Phase mit Methylenchlorid extrahiert. Die wäßrige Phase wird mit etwas Natriumdisulfit versetzt und dann unter Eiskühlung mit 10 ml konzentrierter Salzsäure angesäuert. Der Niederschlag wird abgesaugt und mit Eiswasser gewaschen. Das Rohprodukt wird 4 Stunden mit 2N Salzsäure gerührt, abgesaugt, mit Wasser gewaschen und bei 80°C im Vakuum getrocknet.

Ausbeute: 1,1 g (65 % der Theorie),
Schmelzpunkt: > 220°C
$R_f$-Wert: 0,38 (Kieselgel; Essigester/Methanol = 10:1)

| | | | |
|---|---|---|---|
| Ber.: | C 63,27 | H 5,50 | N 6,71 |
| Gef.: | 63,20 | 5,22 | 6,67 |

Analog werden folgende Verbindungen erhalten:

(1) 2-tert.Butyloxycarbonyl-1,2,3,4-tetrahydroisochinolin-7-carbonsäure

$R_f$-Wert: 0,65 (Kieselgel; Cyclohexan/Essigester = 1:1)

(2) 4-(1-Acetyl-4-piperidinyl)benzoesäure

$R_f$-Wert: 0,25 (Kieselgel; Methylenchlorid/Cyclohexan/Methanol/konz. wäßriges Ammoniak = 68:15:15:2)

Beispiel XIV

2,5-Diacetylisoindolin

Zu 10,3 g Aluminiumtrichlorid in 30 ml Methylenchlorid werden bei Raumtemperatur 5,0 g 2-Acetylisoindolin in 10 ml Methylenchlorid zugetropft. Nach 16 Stunden Rühren bei Raumtemperatur wird das Reaktionsgemisch unter

starkem Rühren auf ein Gemisch aus 100 g Eis und 20 ml konzentrierter Salzsäure gegossen. Die organische Phase wird abgetrennt und die wäßrige Phase viermal mit je 50 ml Methylenchlorid extrahiert. Die vereinigten organischen Phasen werden mit gesättigter wäßriger Kochsalzlösung gewaschen, getrocknet und einrotiert. Das Rohprodukt wird durch Chromatographie über eine Kieselgelsäule mit Essigester/Methanol (15:1) gereinigt.

Ausbeute: 1,9 g (30 % der Theorie),
Schmelzpunkt: 136-140°C
$R_f$-Wert: 0,40 (Kieselgel; Essigester/Methanol = 10:1)

| | | | |
|---|---|---|---|
| Ber.: | C 70,92 | H 6,45 | N 6,89 |
| Gef.: | 71,12 | 6,56 | 6,90 |

Analog wird folgende Verbindung erhalten:

(1) 4-(1-Acetyl-4-piperidinyl)acetophenon

$R_f$-Wert: 0,44 (Kieselgel; Methylenchlorid/Ethanol = 15:1)

<u>Beispiel XV</u>

<u>(3R,5S)-3-Allyl-5-[(4'-cyano-4-biphenylyl)oxymethyl]-1-(2-pyridyl)-2-pyrrolidinon</u>

6,3 g (3R,5S)-3-Allyl-5-[(4'-cyano-4-biphenylyl)oxymethyl]-2-pyrrolidinon, 4,0 g 2-Brompyridin, 5,5 g Kaliumcarbonat, 1,3 g Tris-[2-(2-methoxyethoxy)ethyl]amin und je 0,3 g Kupfer(I)chlorid und Kupfer(I)jodid werden in 100 ml Xylol unter Stickstoff 5 Stunden unter Rückfluß erhitzt. Nach dem Abkühlen wird mit Essigester verdünnt, filtriert und das Filtrat mit Wasser, verdünnter Natriumhydrogensulfit-Lösung, Wasser und gesättigter Kochsalzlösung gewaschen, getrocknet und einrotiert. Das Rohprodukt wird durch Chromatographie über eine Kieselgelsäule mit Cyclohexan/Essigester (85:15) gereinigt.

Ausbeute: 6,3 g (81 % der Theorie),
Schmelzpunkt: 107-109°C
$R_f$-Wert: 0,41 (Kieselgel; Cyclohexan/Essigester = 8:2)

| | | | |
|---|---|---|---|
| Ber.: | C 76,26 | H 5,66 | N 10,26 |
| Gef.: | 75,80 | 5,68 | 10,09 |

Analog werden folgende Verbindungen erhalten:

(1) (3R,5S)-3-Allyl-5-[(4'-cyano-4-biphenylyl)oxymethyl]-1-(3-pyridyl)-2-pyrrolidinon

Schmelzpunkt: 118-121°C
$R_f$-Wert: 0,41 (Kieselgel; Cyclohexan/Essigester = 2:8)

| | | | |
|---|---|---|---|
| Ber.: | C 76,26 | H 5,66 | N 10,26 |
| Gef.: | 75,90 | 5,73 | 10,00 |

Beispiel XVI

7-Cyano-1H-2,3,4, 5-tetrahydro-3-benzazepin

Zu 10,9 g 3-tert.Butyloxycarbonyl-7-cyano-1H-2,3,4,5-tetrahydro-3-benzazepin in 80 ml Methylenchlorid werden 80 ml Trifluoressigsäure in 80 ml Methylenchlorid zugetropft. Nach 15 Minuten wird eingedampft, der Rückstand zwischen 1N Kaliumcarbonatlösung und Methylenchlorid verteilt, die organische Phase mit Wasser gewaschen, getrocknet und eingedampft.

Ausbeute: 5,9 g (85 % der Theorie),
$R_f$-Wert: 0,67 (Kieselgel; Methanol/konz. wäßriges Ammoniak = 9:1)

Analog wird folgende Verbindung erhalten:

(1) 7-Cyano-1,2,3,4-tetrahydroisochinolin

$R_f$-Wert: 0,40 (Kieselgel; Methanol)

| | | | |
|---|---|---|---|
| Ber.: | C 75,92 | H 6,37 | N 17,71 |
| Gef.: | 75,43 | 6,53 | 17,45 |

Beispiel XVII

3-tert.Butyloxycarbonyl-7-cyano-1H-2,3,4,5-tetrahydro-3-benzazepin

17 g 3-tert.Butyloxycarbonyl-1H-2,3,4,5-tetrahydro-3-benzazepin-7-carbonsäureamid, 19,2 g Triphenylphosphin, 5,9 ml Tetrachlorkohlenstoff, 8,2 ml Triethylamin und 90 ml Chloroform werden 9 Stunden bei 60°C gerührt. Das Reaktionsgemisch wird mit Wasser gewaschen, getrocknet und einrotiert. Der Rückstand wird durch Chromatographie über eine Kieselgelsäule mit Cyclohexan/Essigester (1:1) gereinigt.

Ausbeute: 10,9 g (68 % der Theorie),
$R_f$-Wert: 0,77 (Kieselgel; Cyclohexan/Essigester = 1:1)

Analog wird folgende Verbindung erhalten:

(1) 2-tert.Butyloxycarbonyl-7-cyano-1,2,3,4-tetrahydroisochinolin

$R_f$-Wert: 0,82 (Kieselgel; Cyclohexan/Essigester = 1:1)

Beispiel XVIII

3-tert.Butyloxycarbonyl-1H-2,3,4,5-tetrahydro-3-benzazepin-7-carbonsäureamid

Zu einer Lösung von 19,3 g 3-tert.Butyloxycarbonyl-1H-2,3,4,5-tetrahydro-3-benzazepin-7-carbonsäure und 10,8 g 1-Hydroxy-1H-benzotriazol in 350 ml Tetrahydrofuran werden bei 0°C 15 g N,N'-Dicyclohexylcarbodiimid zugegeben und 15 Minuten bei 0°C und 45 Minuten nach Entfernen des Eisbades gerührt. Bei 10°C werden dann 6,7 ml konz. wäßriges Ammoniak zugegeben und 2 1/2 Stunden bei Raumtemperatur gerührt. Es wird vom Niederschlag abgesaugt und das Filtrat eingedampft. Der Rückstand wird zwischen Essigester und Wasser verteilt, die organische Phase abgetrennt, getrocknet und einrotiert. Der Rückstand wird mit Diisopropylether verrührt und der Feststoff abgesaugt. Der Feststoff wird durch Chromatographie über eine Kieselgelsäule mit Essigester gereinigt.

Ausbeute: 15,5 g (81 % der Theorie),
Schmelzpunkt: 173-174°C
$R_f$-Wert: 0,53 (Kieselgel; Essigester)

| | | | |
|---|---|---|---|
| Ber.: | C 66,18 | H 7,64 | N 9,65 |
| Gef.: | 66,42 | 7,81 | 9,77 |

Analog wird folgende Verbindung erhalten:

(1) 2-tert.Butyloxycarbonyl-1,2,3,4-tetrahydroisochinolin-7-carbonsäureamid

$R_f$-Wert: 0,52 (Kieselgel; Essigester)

Beispiel XIX

(3R,5S)-3-Allyl-5-[[(3-tert.butyloxycarbonyl-1H-2,3,4,5-tetrahydro-3-benzazepin-7-yl)carbonylamino]methyl]-1-(3-phenylpropyl)-2-pyrrolidinon

Zu 4,4 g 3-tert.Butyloxycarbonyl-1H-2,3,4,5-tetrahydro-3-benzazepin-7-ylcarbonsäure in 30 ml trockenem Tetrahydrofuran werden 3,4 g Carbonyldiimidazol zugegeben und unter leichtem Erwärmen eine Stunde gerührt. Dann werden 4,1 g (3R,5S)-3-Allyl-5-aminomethyl-1-(3-phenylpropyl)-2-pyrrolidinon in 10 ml Tetrahydrofuran zugetropft und bei Raumtemperatur 16 Stunden gerührt. Es werden 100 ml 1N Salzsäure zugegeben und der Ansatz mit Essigester extrahiert. Die vereinigten organischen Phasen werden mit Wasser und Natriumhydrogencarbonatlösung gewaschen, getrocknet, über Aktivkohle und etwas Kieselgel filtriert und einrotiert.

Ausbeute: 6,6 g (80 % der Theorie),
$R_f$-Wert: 0,37 (Kieselgel; Essigester/Cyclohexan = 2:1)

Analog wird folgende Verbindung erhalten:

(1) (3R,55)-3-Allyl-5-[[[(1-tert.butyloxycarbonyl-4-piperidinyl)phenyl]carbonylamino]methyl]-1-(3-phenylpropyl)-2-pyrrolidinon

$R_f$-Wert: 0,30 (Kieselgel; Essigester/Cyclohexan = 2:1)

Beispiel XX

(3R,5S)-3-Allyl-5-[(3-tert.butyloxycarbonyl-1H-2,3,4,5-tetrahydro-3-benzazepin-7-yl)oxymethyl]-1-(3-phenylpropyl)-2-pyrrolidinon

5,1 g 3-tert.Butyloxycarbonyl-7-hydroxy-1H-2,3,4,5-tetrahydro-3-benzazepin in 80 ml Dimethylformamid werden mit 8,2 g Cäsiumcarbonat eine halbe Stunde bei 55°C gerührt. Dann werden 6,8 g (3R,5S)-3-Allyl-5-[(methansulfonyloxy)methyl]-1-(3-phenylpropyl)-2-pyrrolidinon in 40 ml Dimethylformamid zugetropft und 16 Stunden bei 55°C gerührt. Das Reaktionsgemisch wird auf Wasser gegeben und mit Essigester extrahiert. Die vereinigten organischen Phasen werden mit Wasser und gesättigter Kochsalzlösung gewaschen, getrocknet, über Aktivkohle filtriert und einrotiert. Der Rückstand wird durch Chromatographie über eine Kieselgelsäule mit Cyclohexan/Essigester (2:1) gereinigt.

Ausbeute: 7,5 g (77 % der Theorie),
$R_f$-Wert: 0,36 (Kieselgel; Cyclohexan/Essigester = 2:1)

Analog wird folgende Verbindung erhalten:

(1) (3R,5S)-3-Allyl-5-[[4-(1-tert.butyloxycarbonyl-4-piperidinyl)phenyl]oxymethyl]-1-(3-phenylpropyl)-2-pyrrolidinon

$R_f$-Wert: 0,39 (Kieselgel; Cyclohexan/Essigester = 2:1)

Beispiel XXI

3-Acetyl-7-hydroxy-1H-2,3,4,5-tetrahydro-3-benzazepin

11,9 g 7-Acetoxy-3-acetyl-1H-2,3,4,5-tetrahydro-3-benzazepin und 9 g Kaliumcarbonat werden in 100 ml Methanol eine Stunde unter Rückfluß gekocht. Es wird abgesaugt und das Filtrat eingeengt. Der Rückstand wird zwischen Essigester und verdünnter Salzsäure verteilt. Die organische Phase wird abgetrennt, getrocknet über Aktivkohle und wenig Kieselgel filtriert und das Filtrat einrotiert. Der Rückstand wird mit wenig Methylenchlorid verrieben, abgesaugt und getrocknet.

Ausbeute: 5,0 g (51 % der Theorie)
$R_f$-Wert: 0,41 (Kieselgel; Methylenchlorid/Ethanol = 15:1)

Analog wird folgende Verbindung erhalten:

(1) 4-(1-Acetyl-4-piperidinyl)phenol

$R_f$-Wert: 0,40 (Kieselgel; Methylenchlorid/Ethanol = 15:1)

Beispiel XXII

7-Acetoxy-3-acetyl-1H-2,3,4,5-tetrahydro-3-benzazepin

11,5 g 3,7-Diacetyl-1H-2,3,4,5-tetrahydro-3-benzazepin, 17 g m-Chlorperbenzoesäure und 100 ml Chloroform werden 4 Tage bei Raumtemperatur im Dunkeln gerührt. Es wird vom Niederschlag abgesaugt, der Niederschlag wird mit Methylenchlorid gewaschen und das gesamte Filtrat nach Verdünnung mit Methylenchlorid, zweimal mit Natriumhydrogensulfitlösung, zweimal mit Natriumhydrogencarbonatlösung und einmal mit gesättigter Kochsalzlösung geschüttelt, getrocknet und einrotiert.

Ausbeute: 11,9 g (97 % der Theorie),
$R_f$-Wert: 0,50 (Kieselgel; Methylenchlorid/Ethanol = 15:1)

Analog wird folgende Verbindung erhalten:

(1) 1-Acetyl-4-(4-acetoxyphenyl)-piperidin

$R_f$-Wert: 0,44 (Kieselgel; Methylenchlorid/Ethanol = 15:1)

Beispiel XXIII

(3R,5S)-3-Allyl-5-[[6-(4-cyanophenyl)-3-pyridazinyl]oxymethyl]-1-(3-phenylpropyl)-2-pyrrolidinon

3,8 g (3R,5S)-3-Allyl-5-hydroxymethyl-1-(3-phenylpropyl)-2-pyrrolidinon in 30 ml Dimethylformamid werden mit 0,65 g Natriumhydrid (55%ige Dispersion in Paraffinöl) versetzt und 10 Minuten bei Raumtemperatur gerührt. Dann werden portionsweise 3,0 g 3-Chlor-6-(4-cyanophenyl)pyridazin zugegeben und eine Stunde bei Raumtemperatur gerührt. Das Reaktionsgemisch wird in Wasser eingerührt, es wird etwas Kochsalz zugegeben und dann mit Essigester extrahiert. Die organische Phase wird mit Wasser gewaschen, getrocknet, filtriert und einrotiert. Nach Chromatographie über eine Kieselgelsäule mit Cyclohexan/Essigester (2:1; Zusatz von 5 % Methanol) verbleiben 3,3 g (52 % der Theorie).

$R_f$-Wert: 0,31 (Kieselgel; Methylenchlorid/Methanol = 50:1)

Beispiel XXIV

(3R,5S)-3-Allyl-5-[[6-(4-cyanophenyl)-3-pyridazinyl]aminome thyl]-1-(3-phenylpropyl)-2-pyrrolidinon

6,2 g (3R,5S)-3-Allyl-5-aminomethyl]-2-pyrrolidinon, 4,9 g 3-Chlor-6-(4-cyanophenyl)pyridazin, 3,2 g Natriumcarbonat und 50 ml Dimethylsulfoxid werden 5 Stunden bei 160°C gerührt, abgekühlt, in eine wäßrige Kochsalzlösung

gegeben und mit Essigester extrahiert. Die organische Phase wird mit Wasser gewaschen, getrocknet, filtriert und einrotiert. Nach Reinigung über eine Kieselgelsäule mit Methylenchlorid/Methanol (15:1) verbleiben 4,2 g (41 % der Theorie).

$R_f$-Wert: 0,33 (Kieselgel; Methylenchlorid/Methanol = 15:1)

Beispiel XXV

3-[(4-Cyanophenyl)aminocarbonyl]-2(1H)-pyridon

84 g 2-Chlor-3-[(4-cyanophenyl)aminocarbonyl]-pyridin, 1 l Eisessig und 111 g Natriumacetat-trihydrat werden 48 Stunden bei 130°C gerührt. Dann wird eingeengt und der Rückstand in 1 l Wasser gegeben. Der Niederschlag wird abgesaugt, mehrmals mit Wasser, Aceton und Diethylether gewaschen und getrocknet.

Ausbeute: 74 g (95 % der Theorie),
Schmelzpunkt: 320-322°C

| | | | |
|---|---|---|---|
| Ber.: | C 65,27 | H 3,79 | N 17,56 |
| Gef.: | 65,25 | 3,89 | 17,55 |

Das Ausgangsmaterial (Schmelzpunkt: 198-201°C) wird durch Umsetzung von 2-Chlor-nicotinsäurechlorid mit 4-Aminobenzonitril in Gegenwart von Triethylamin erhalten.
Analog werden folgende Verbindungen erhalten:

(1) 5-[(4-Cyanophenyl)aminocarbonyl]-2(1H)-pyridon

Schmelzpunkt: 330-332°C

Das Ausgangsmaterial (Schmelzpunkt: 216-218°C) wird durch Umsetzung von 6-Chlor-nicotinsäurechlorid mit 4-Aminobenzonitril in Gegenwart von Triethylamin erhalten.

(2) 5-[(4-Cyanophenyl)aminocarbonyl]-2,4(1H,3H)-pyrimidindion

Schmelzpunkt: > 330°C

Das Ausgangsmaterial (Schmelzpunkt: 162-164°C) wird durch Umsetzung von 2,4-Dichlor-pyrimidin-5-carbonsäurechlorid und 4-Aminobenzonitril in Gegenwart von Triethylamin erhalten.

Beispiel XXVI

4-[(2-Benzylamino-6-chlor-4-pyridyl)aminocarbonyl]-1-benzyloxybenzol

Das aus 2,4 g 4-Benzyloxybenzoesäure mit Thionylchlorid erhaltene Säurechlorid wird mit 50 ml Methylenchlorid, 2,5 g 4-Amino-2-benzylamino-6-chlor-pyridin, 1,5 ml Triethylamin und 0,5 g 4-Dimethylaminopyridin insgesamt 7 Stunden am Rückfluß erhitzt und dann 2 1/2 Tage bei Raumtemperatur gerührt. Es wird abgekühlt und das Kristallisat abgesaugt.

Ausbeute: 3,4 g (73 % der Theorie)
$R_f$-Wert: 0,56 (Kieselgel; Diethylether/Petrolether = 7:3)

Beispiel XXVII

4-Amino-2-benzylamino-6-chlor-pyridin

23,5 g 2-Benzylamino-6-chlor-4-nitro-pyridin-N-oxid in 1,5 l Tetrahydrofuran werden mit 2 g Raney-Nickel 12 Stunden bei 40°C und einem Wasserstoffdruck von 5 bar hydriert. Die Filtration des Ansatzes ergibt Filtrat 1. Der Rückstand wird mit 2 g Raney-Nickel in Tetrahydrofuran/Dimethylformamid weitere 5 Stunden bei 40°C hydriert. Der Ansatz wird filtriert, das Filtrat eingeengt und der Rückstand mit Methylenchlorid/Methanol (50:1) verrieben. Der Feststoff wird abgesaugt und verworfen und die Mutterlauge zusammen mit Filtrat 1 eingedampft. Die Reinigung des Rückstandes durch mehrmalige Chromatographie über eine Kieselgelsäule mit Methylenchlorid/Methanol (50:1) und Kristallisation aus Methylenchlorid ergeben 5,4 g (28 % der Theorie).

$R_f$-Wert: 0,68 (Kieselgel; Methylenchlorid/Methanol = 19:1)

Beispiel XXVIII

2-Benzylamino-6-chlor-4-nitropyridin-N-oxid

18,5 g 2,6-Dichlor-4-nitropyridin-N-oxid, suspendiert in 100 ml Ethanol, werden bei Raumtemperatur mit 18,6 ml Benzylamin versetzt und dann 3 Stunden unter Rückfluß erhitzt. Nach dem Abkühlen wird der Niederschlag abgesaugt und mit Ethanol und Diethylether gewaschen.

Ausbeute: 23,7 g (95 % der Theorie)
$R_f$-Wert: 0,41 (Kieselgel; Methylenchlorid/Methanol = 19:1)

Beispiel XXIX

4-(5-Cyano-2-thiazolyl)anisol

Zu 74,4 g Kalium-tert.butylat in 600 ml tert.Butyl-methylether und 200 ml trockenem Ethanol werden bei -30°C ein Gemisch von 42 ml Chloracetoniril und 54 ml Ameisensäure-ethylester in 200 ml tert.Butyl-methylether zugetropft. Dann wird 2 1/2 Tage bei Raumtemperatur gerührt. Zu der erhaltenen Suspension werden 9,1 g 4-Methoxythiobenzamid und 400 ml trockenes Ethanol gegeben und 3 Stunden unter Rückfluß erhitzt. Das Reaktionsgemisch wird eingeengt, mit Wasser versetzt und mit Essigester extrahiert. Die organische Phase wird abgetrennt, getrocknet und einrotiert. Der Rückstand wird durch Chromatographie über eine Kieselgelsäule mit Methylenchlorid gereinigt. Die Produktfraktionen werden einrotiert, der Rückstand mit tert.Butyl-methylether verrieben und das Kristallisat abgesaugt und getrocknet.

Ausbeute: 1,06 g (9 % der Theorie, bezogen auf 4-Methoxythio benzamid),
Schmelzpunkt: 141-142°C
$R_f$-Wert: 0,56 (Kieselgel; Toluol/Essigester = 19:1)

| | | | | |
|---|---|---|---|---|
| Ber.: | C 61,09 | H 3,72 N | 12,96 | S 14,83 |
| Gef.: | 61,18 | 3,79 | 13,23 | 14,61 |

Beispiel XXX

3-(4-Cyanobenzoyl)-2-hydroxy-propionsäure

25 g 4-Cyanoacetophenon und 24 g Glyoxylsäure-monohydrat werden im Wasserstrahl-Vakuum 5 Stunden bei 95°C gerührt. Man erhält ein zähes Öl, welches in einem Gemisch aus gesättigter Natriumhydrogencarbonat-Lösung und Essigester gelöst wird. Die organische Phase wird abgetrennt, die wäßrige Phase mit 2N Salzsäure angesäuert und mehrmals mit Essigester extrahiert. Die vereinigten Essigester-Phasen werden mit Wasser gewaschen, getrocknet und eingedampft. Der Rückstand wird mit Diethylether verrieben, abgesaugt und als Rohprodukt weiter umgesetzt.

Ausbeute: 18,2 g (48 % der Theorie),
$R_f$-Wert: 0,25 (Kieselgel; Methylenchlorid/Methanol/Eisessig = 8:2:0,2)

Beispiel XXXI

6-(4-Cyanophenyl)-2H-pyridazin-3-on

19,2 g 3-(4-Cyanobenzoyl)-2-hydroxy-propionsäure und 20 ml 80%ige Hydrazinlösung in 200 ml Eisessig werden 2 Stunden unter Rückfluß erhitzt. Man läßt abkühlen, saugt den Niederschlag ab und wäscht mit Essigsäure und Diethylether.

Ausbeute: 10,3 g (60 % der Theorie)
$R_f$-Wert: 0,32 (Kieselgel; Methylenchlorid/Methanol = 19:1)

Beispiel XXXII

3-Chlor-6-(4-cyanophenyl)pyridazin

Eine Suspension von 27,6 g 6-(4-Cyanophenyl)-2H-pyridazin-3-on in 200 ml Phosphoroxychlorid wird 2 Stunden unter Rückfluß erhitzt. Die Reaktionslösung wird in 1 l Wasser eingerührt, der Niederschlag abgesaugt, mit Wasser nachgewaschen und getrocknet.

Ausbeute: 25,7 g (85 % der Theorie),
$R_f$-Wert: 0,75 (Kieselgel; Methylenchlorid/Methanol = 19:1)

Beispiel XXXIII

[2-(4-Bromphenyl)-2-oxo-ethyl]malonsäure-diethylester

Zu einer Lösung von 165 ml Malonsäure-diethylester in 600 ml Dimethylformamid gibt man 124 g Kalium-tert.butylat. Unter Kühlung im Wasserbad werden 307 g 4-Bromphenacylbromid portionsweise zugegeben, wobei sich die Reaktionslösung auf ca. 70°C erwärmt. Man rührt 2 Stunden bei Raumtemperatur, gießt die Reaktionslösung in verdünnte Kochsalzlösung und extrahiert mit Essigester. Die organische Phase wird mit Wasser gewaschen, über Natriumsulfat getrocknet und über Aktivkohle filtriert. Nach Entfernen des Lösungsmittels am Rotationsverdampfer erhält man 398 g eines roten Öls, welches ohne Reinigung weiter umgesetzt wird.

$R_f$-Wert: 0,40 (Kieselgel; Cyclohexan/Essigester = 5:1)

Beispiel XXXIV

6-(4-Bromphenyl)-4,5-dihydro-4-ethoxycarbonyl-2H-pyridazin-3-on

Zu einer Lösung von 398 g [2-(4-Bromphenyl)-2-oxo-ethyl]malonsäure-diethylester in 1,5 l Eisessig gibt man 240 ml 80%ige Hydrazinhydrat-Lösung und erhitzt 2 Stunden zum Rückfluß. Beim Abkühlen der Reaktionslösung entsteht ein Niederschlag, der abgenutscht, mit Wasser gewaschen und getrocknet wird. Um weiteres Produkt zu isolieren wird die Mutterlauge mit Wasser versetzt, der Niederschlag abgenutscht und aus Eisessig umkristallisiert.

Ausbeute: 213 g (61 % der Theorie),
$R_f$-Wert: 0,31 (Kieselgel; Cyclohexan/Essigester = 2:1)

Beispiel XXXV

6-(4-Bromphenyl)-4-ethoxycarbonyl-2H-pyridazin-3-on

Zu einer Suspension von 213 g 6-(4-Bromphenyl)-4,5-dihydro-4-ethoxycarbonyl-2H-pyridazin-3-on in 2,0 l Eisessig tropft man eine Lösung von 40 ml Brom in 100 ml Eisessig und rührt 45 Minuten bei Raumtemperatur. Man verdünnt mit 2,0 l Wasser und zerstört überschüssiges Brom mit verdünnter Natriumsulfit-Lösung. Der Niederschlag wird abgenutscht, mit Wasser gewaschen und getrocknet.

Ausbeute: 206 g (97 % der Theorie),
$R_f$-Wert: 0,39 (Kieselgel; Methylenchlorid/Methanol = 15:1)

Beispiel XXXVI

6-(4-Cyanophenyl)-4-ethoxycarbonyl-2H-pyridazin-3-on

Zu einer Lösung von 24,6 g 6-(4-Bromphenyl)-4-ethoxycarbonyl-2H-pyridazin-3-on in 100 ml Dimethylformamid gibt man 6,9 g Kupfer(I)cyanid und erhitzt 6 Stunden unter Rückfluß. Die Reaktionslösung wird abgekühlt und in Wasser eingerührt. Der Niederschlag wird abgenutscht und getrocknet. Man erhält 23 g Rohprodukt, welches ohne Reinigung weiter umgesetzt wird.

$R_f$-Wert: 0,39 (Kieselgel; Methylenchlorid/Methanol = 15:1)

Beispiel XXXVII

6-(4-Cyanophenyl)-4-ethoxycarbonyl-2-methyl-2H-pyridazin-3-on

Zu einer Suspension aus 18 g rohem 6-(4-Cyanophenyl)-4-ethoxycarbonyl-2H-pyridazin-3-on, 7,5 ml Methyliodid, 2,0 g Methyl-trioctylammoniumchlorid in 200 ml 2M Kaliumhydrogencarbonat-Lösung, 500 ml Tetrahydrofuran und 1,6 l Toluol wird 16 Stunden bei Raumtemperatur gerührt. Es werden 3 ml Methyliodid hinzugegeben und weitere 4 Stunden gerührt. Der Niederschlag wird abgenutscht und das Filtrat mit Wasser gewaschen. Die organische Phase wird über Natriumsulfat getrocknet, das Lösungsmittel im Vakuum abgedampft und das Rohprodukt über Kieselgel chromatographiert.

Ausbeute: 11,2 g (59 % der Theorie),
$R_f$-Wert: 0,49 (Kieselgel; Methylenchlorid/Methanol = 9:1)

Beispiel XXXVIII

4-Carboxy-6-(4-cyanophenyl)-2-methyl-2H-pyridazin-3-on

Zu einer Lösung von 12,0 g 6-(4-Cyanophenyl)-4-ethoxycarbonyl-2-methyl-2H-pyridazin-3-on in 200 ml Tetrahydrofuran gibt man eine Lösung von 7,14 g Lithiumhydroxyd-Monohydrat in 170 ml Wasser und rührt 1,5 Stunden. Es wird mit 1N Salzsäure angesäuert, das Tetrahydrofuran im Vakuum abgedampft, der Niederschlag abgenutscht und getrocknet.

Ausbeute: 11,2 g,
Schmelzpunkt: 190-194°C
$R_f$-Wert: 0,33 (Kieselgel; Methylenchlorid/Methanol/konz. wäßriges Ammoniak = 4:1:0,25)

Analog werden folgende Verbindungen erhalten:

(1) 4-Carboxy-6-(4-cyanophenyl)-2H-pyridazin-3-on

Schmelzpunkt: über 290°C

Beispiel 1

(3S,5S)-5-[[4-(5-Amidino-2-pyridyl)phenyl]oxymethyl]-3-carboxymethyl-2-pyrrolidinon

Zu einer Suspension von 0,35 g (3S,5S)-5-[[4-(5-Amidino-2-pyridyl)phenyl]oxymethyl]-3[(tert.butyloxycarbonyl)methyl]-2-pyrrolidinon-hydrochlorid in 3 ml Methylenchlorid werden 3 ml Trifluoressigsäure gegeben. Nach 75 Minuten Rühren bei Raumtemperatur wird eingeengt. Der Rückstand wird mit Wasser versetzt und mit 1N Natronlauge weitgehend gelöst. Es wird filtriert und das Filtrat mit 2N Salzsäure auf pH 7 gebracht. Der gallertartige Niederschlag wird abgesaugt, mit wenig Wasser und Aceton gewaschen und bei 80°C im Vakuum getrocknet.

Ausbeute: 254 mg (84 % der Theorie),

Schmelzpunkt: 259-261°C
$R_f$-Wert: 0,59 (reversed phase Kieselgel; Methanol/5%ige wäßrige Kochsalzlösung = 6:4)

Analog werden folgende Verbindungen erhalten:

(1) (3S,5S)-5-[[4-(2-Amidino-5-pyrimidyl)phenyl]oxymethyl]-3-carboxymethyl-2-pyrrolidinon x 0,75 HCl x 2 $H_2O$

Schmelzpunkt: 207-209°C (Zers., ab 186°C sintern)
$R_f$-Wert: 0,58 (reversed phase Kieselgel; Methanol/5%ige wäßrige Kochsalzlösung = 6:4)

| | | | | |
|---|---|---|---|---|
| Ber.: | C 49,96 | H 5,49 | N 16,18 | Cl 6,14 |
| Gef.: | 49,77 | 5,27 | 15,95 | 6,36 |

(2) (3S,5S)-5-[[4-(5-Amidino-2-pyrimidyl)phenyl]oxymethyl]-3-carboxymethyl-2-pyrrolidinon

$R_f$-Wert: 0,72 (reversed phase Kieselgel; Methanol/5%ige wäßrige Kochsalzlösung = 6:4)

| | | | |
|---|---|---|---|
| Ber.: | C 58,53 | H 5,18 | N 18,96 |
| Gef.: | 58,22 | 5,25 | 18,81 |

(3) (3S,5S)-5-[[4-(5-Amidino-2-thiazolyl)phenyl]oxymethyl]-3-carboxymethyl-2-pyrrolidinon x 0,6 $H_2O$

$R_f$-Wert: 0,63 (reversed phase Kieselgel; Methanol/5%ige wäßrige Kochsalzlösung = 6:4)

| | | | | |
|---|---|---|---|---|
| Ber.: | C 53,00 | H 5,03 | N 14,55 | S 8,32 |
| Gef.: | 52,91 | 5,11 | 14,44 | 8,53 |

Massenspektrum: $(M + H)^+ = 375$

(4) (3S,5S)-3-Carboxymethyl-5-[[4-(6-isochinolinyl)phenyl]oxymethyl]-2-pyrrolidinon

$R_f$-Wert: 0,42 (reversed phase Kieselgel; Methanol/10%ige wäßrige Kochsalzlösung = 3:1)
Massenspektrum: $M^+ = 376$

<u>Beispiel 2</u>

<u>(3S,5S)-5-[[4-(5-Amidino-2-pyridyl)phenyl]oxymethyl]-3-[(tert.butyloxycarbonyl)methyl]-2-pyrrolidinon-hydrochlorid</u>

2,1 g (3S,5S)-3-[(tert.Butyloxycarbonyl)methyl]-5-[[4-(5-cyano-2-pyridyl)phenyl]oxymethyl]-2-pyrrolidinon in 50 ml trockenem Methanol werden mit 8,5 ml 0,13 M Natriummethylat-Lösung 40 Stunden bei Raumtemperatur gerührt. Es werden 63 ml Eisessig und dann 0,5 g Ammoniumchlorid zugegeben und 2 1/2 Tage bei Raumtemperatur gerührt. Nach dem Einengen wird durch Säulenchromatographie auf Kieselgel mit Methylenchlorid/Methanol (6:1) gereinigt.

Ausbeute: 1 g (42 % der Theorie),
Schmelzpunkt: 207°C (Zers.)
$R_f$-Wert: 0,56 (Kieselgel; Methylenchlorid/Methanol = 4:1)

Analog werden folgende Verbindungen erhalten:

(1) (3S,5S)-5-[[4-(2-Amidino-5-pyridyl)phenyl]oxymethyl]-3-[(tert.butyloxycarbonyl)methyl]-2-pyrrolidinon-hydrochlorid

$R_f$-Wert: 0,50 (Kieselgel; Methylenchlorid/Methanol = 6:1)

(2) (3S,5S)-5-[[4-(5-Amidino-2-pyrazinyl)phenyl]oxymethyl]-3-[(tert.butyloxycarbonyl)methyl]-2-pyrrolidinon-hydrochlorid

Schmelzpunkt: 213°C (Zers.)
$R_f$-Wert: 0,55 (Kieselgel; Methylenchlorid/Methanol = 6:1)

(3) (3S,5S)-5-[[4-(2-Amidino-5-pyrimidyl)phenyl]oxymethyl]-3-[(tert.butyloxycarbonyl)methyl]-2-pyrrolidinon-hydrochlorid

Schmelzpunkt: 197-198°C
$R_f$-Wert: 0,33 (reversed phase Kieselgel; Methanol/5%ige wäßrige Kochsalzlösung = 6:4)

(4) (3S,5S)-5-[[4-(5-Amidino-2-pyrimidyl)phenyl]oxymethyl]-3-[(tert.butyloxycarbonyl)methyl]-2-pyrrolidinon-hydrochlorid

Schmelzpunkt: 277-279°C (Zers.)
$R_f$-Wert: 0,38 (reversed phase Kieselgel; Methanol/5%ige wäßrige Kochsalzlösung = 6:4)

(5) (3S,5S)-5-[[4-(5-Amidino-2-thiazolyl)phenyl]oxymethyl]-3-[(tert.butyloxycarbonyl)methyl]-2-pyrrolidinon-hydrochlorid

$R_f$-Wert: 0,46 (reversed phase Kieselgel; Methanol/5%ige wäßrige Kochsalzlösung = 6:4)

(7) (3R,5R)-5-[[4-(5-Amidino-2-pyrimidyl)phenyl]oxymethyl]-3-[(methoxycarbonyl)methyl]-2-pyrrolidinon-hydrochlorid
(8) (3R,5S)-5-[[4-(5-Amidino-2-pyrazinyl)phenyl]oxymethyl]-3-[(methoxycarbonyl)methyl]-2-pyrrolidinon-hydrochlorid
(9) (3S,5S)-5-[[4-(5-Amidino-2-pyrimidyl)phenyl]oxymethyl]-3-[(methoxycarbonyl)methyl]-1-nicotinoyl-pyrrolidin-hydrochlorid

Beispiel 3

(3S,5S)-5-[[4-(5-Amidino-2-pyridyl)phenyl]oxymethyl]-3-[(methoxycarbonyl)methyl]-2-pyrrolidinon x 1,6 HCl x 1,5 $H_20$

0,55 g (3S,5S)-5-[[4-(5-Amidino-2-pyridyl)phenyl]oxymethyl]-3-[(tert.butyloxycarbonyl)methyl]-2-pyrrolidinon in 15 ml trockenem Methanol werden mit 2 ml gesättigter methanolischer Salzsäure versetzt und 18 Stunden bei Raumtemperatur gerührt. Es werden nochmal 15 ml Methanol und 2 ml gesättigte methanolische Salzsäure zugegeben und weitere 24 Stunden gerührt. Der Ansatz wird eingeengt und der Rückstand mit Methanol versetzt und zur Trockene eingedampft. Der Rückstand wird mit tert.Butyl-methylether und wenig Methanol verrieben und abgesaugt. Reinigung über eine Kieselgelsäule mit Methylenchlorid/Methanol (4:1) ergibt 0,28 g (46 % der Theorie).

Schmelzpunkt: 253°C (Zers.)
$R_f$-Wert: 0,42 (reversed phase Kieselgel; Methanol/5%ige wäßrige Kochsalzlösung = 6:4)

| | | | | |
|---|---|---|---|---|
| Ber.: | C 51,35 | H 5,73 | N 11,98 | Cl 12,13 |
| Gef.: | 51,49 | 5,51 | 11,71 | 12,12 |

Massenspektrum: $(M + H)^+ = 383$

Analog werden folgende Verbindungen erhalten:

(1) (3S,5S)-5-[[4-(2-Amidino-5-pyridyl)phenyl]oxymethyl]-3-[(methoxycarbonyl)methyl]-2-pyrrolidinon x 1,3 HCl x 0,3 $H_2$0

$R_f$-Wert: 0,54 (reversed phase Kieselgel; Methanol/5%ige wäßrige Kochsalzlösung = 6:4)

| | | | | |
|---|---|---|---|---|
| Ber.: | C 55,19 | H 5,54 | N 12,87 | Cl 10,59 |
| Gef.: | 55,13 | 5,76 | 12,56 | 10,83 |

(2) (3S,5S)-5-[[4-(5-Amidino-2-pyrazinyl)phenyl]oxymethyl]-3-[(methoxycarbonyl)methyl]-2-pyrrolidinon-hydrochlorid

$R_f$-Wert: 0,52 (reversed phase Kieselgel; Methanol/5%ige wäßrige Kochsalzlösung = 6:4)

(3) (3S,5S)-5-[[4-(2-Amidino-5-pyrimidyl)phenyl]oxymethyl]-3-[(methoxycarbonyl)methyl]-2-pyrrolidinon x 1,5 HCl x 1 $H_2$0

Schmelzpunkt: 193-195°C
$R_f$-Wert: 0,51 (reversed phase Kieselgel; Methanol/5%ige wäßrige Kochsalzlösung = 6:4)

| | | | | |
|---|---|---|---|---|
| Ber.: | C 50,03 | H 5,42 | N 15,36 | Cl 11,66 |
| Gef.: | 49,85 | 5,61 | 15,59 | 11,76 |

(4) (3S,5S)-5-[[4-(5-Amidino-2-pyrimidyl)phenyl]oxymethyl]-3-[(methoxycarbonyl)methyl]-2-pyrrolidinon x 1,4 HCl x 2 $H_2$0

$R_f$-Wert: 0,45 (reversed phase Kieselgel; Methanol/5%ige wäßrige Kochsalzlösung = 6:4)

| | | | | |
|---|---|---|---|---|
| Ber.: | C 48,50 | H 5,66 | N 14,89 | Cl 10,55 |
| Gef.: | 48,72 | 5,64 | 14,57 | 10,75 |

(5) (3S,5S)-5-[[4-(5-Amidino-2-thiazolyl)phenyl]oxymethyl]-3-[(methoxycarbonyl)methyl]-2-pyrrolidinon-hydrochlorid

$R_f$-Wert: 0,45 (reversed phase Kieselgel; Methanol/5%ige wäßrige Kochsalzlösung = 6:4)
Massenspektrum: $(M + H)^+$ = 389

Beispiel 4

(3S,5S)-5-[[4-(2-Amidino-5-pyridyl)phenyl]oxymethyl]-3-carboxymethyl-2-pyrrolidinon

180 mg (3S,5S)-5-[[4-(2-Amidino-5-pyridyl)phenyl]oxymethyl]-3-[(methoxycarbonyl)methyl]-2-pyrrolidinon x 1,3 HCl x 0,3 $H_2$0, 5 ml Methanol und 1,3 ml 1N NaOH werden 5 Stunden bei Raumtemperatur gerührt. Dann werden 0,26 g Ammoniumchlorid zugegeben und eine halbe Stunde gerührt. Der Ansatz wird eingeengt und der Niederschlag abgesaugt und getrocknet. Ausbeute: 115 mg (72 % der Theorie),

Schmelzpunkt: 276-278°C
$R_f$-Wert: 0,62 (reversed phase Kieselgel; Methanol/5%ige wäßrige Kochsalzlösung = 6:4)

| | | | |
|---|---|---|---|
| Ber.: | C 60,47 | H 5,61 | N 14,85 |
| Gef.: | 60,54 | 5,66 | 14,77 |

Analog werden folgende Verbindungen erhalten:

(1) (3S,5S)-5-[[4-(5-Amidino-2-pyrazinyl)phenyl]oxymethyl]-3-carboxymethyl-2-pyrrolidinon

Schmelzpunkt: 272-275°C
$R_f$-Wert: 0,66 (reversed phase Kieselgel; Methanol/5%ige wäßrige Kochsalzlösung = 6:4)

| | | | |
|---|---|---|---|
| Ber.: | C 56,33 | H 5,41 | N 18,25 |
| Gef.: | 56,47 | 5,50 | 17,78 |

(2) (3S,5S)-5-[(4'-Amidino-4-biphenylyl)oxymethyl]-3-carboxymethyl-1-(2-pyrimidyl)-pyrrolidin x 0,25 HCl x 0,4 $H_20$

Schmelzpunkt: 254-257°C
$R_f$-Wert: 0,27 (reversed phase Kieselgel; Methanol/5%ige wäßrige Kochsalzlösung = 6:4)

| | | | | |
|---|---|---|---|---|
| Ber.: | C 64,37 | H 5,86 | N 15,64 | Cl 1,98 |
| Gef.: | 64,46 | 5,65 | 15,45 | 1,85 |

(3) (3S,5S)-5-[[4-(1-Amino-6-isochinolinyl)phenyl]oxymethyl]-3-carboxymethyl-2-pyrrolidinon-hydrochlorid
Nach der Zugabe von Ammoniumchlorid wird mit 1N Salzsäure versetzt und der Niederschlag abgesaugt.

Schmelzpunkt: > 200°C
$R_f$-Wert: 0,47 (reversed phase Kieselgel; Methanol/5%ige wäßrige Kochsalzlösung = 6:4)
Massenspektrum: $M^+$ = 391

(4) (3S,5S)-5-[(4'-Amidino-4-biphenylyl)oxymethyl]-3-carboxymethyl-1-nicotinoyl-pyrrolidin

Schmelzpunkt: 227-230°C (Zers.)
$R_f$-Wert: 0,08 (Kieselgel; Methylenchlorid/Methanol/konz. wäßriges Ammoniak = 10:2:0,4)

(5) (3S,5S)-5-[(4'-Amidino-4-biphenylyl)oxymethyl]-3-carboxymethyl-1-(2-pyridyl)-2-pyrrolidinon

Schmelzpunkt: 230-233°C (Zers.)
$R_f$-Wert: 0,28 (reversed phase Kieselgel; Methanol/10%ige wäßrige Kochsalzlösung = 6:4)

(6) (3S,5S)-5-[(4'-Amidino-4-biphenylyl)oxymethyl]-3-carboxymethyl-1-(3-pyridyl)-2-pyrrolidinon x 1,2 $H_20$

Schmelzpunkt: 209-211°C (Zers.)
$R_f$-Wert: 0,54 (reversed phase Kieselgel; Methanol/10%ige wäßrige Kochsalzlösung = 6:4)

| | | | |
|---|---|---|---|
| Ber.: | C 64,45 | H 5,71 | N 12,03 |
| Gef.: | 64,33 | 5,70 | 11,94 |

(7) (3S,5S)-5-[2-[(7-Amidino-1,2,3,4-tetrahydro-2-isochinolinyl)carbonylamino]ethyl]-3-carboxymethyl-1-(3-phenylpropyl)-2-pyrrolidinon x 1,5 $H_20$

$R_f$-Wert: 0,24 (Kieselgel; Methylenchlorid/Methanol/konz. wäßriges Ammoniak = 80:20:5)

| | | | |
|---|---|---|---|
| Ber.: | C 63,14 | H 7,19 | N 13,15 |
| Gef.: | 63,09 | 7,27 | 13,05 |

(8) (3S,5S)-5-[[(7-Amidino-1,2,3,4-tetrahydro-2-isochinolinyl)carbonylamino]methyl]-3-carboxymethyl-1-(3-phenylpropyl)-2-pyrrolidinon x 0,5 $H_20$

$R_f$-Wert: 0,22 (Kieselgel; Methylenchlorid/Methanol/konz. wäßriges Ammoniak = 80:20:5)

| | | | |
|---|---|---|---|
| Ber.: | C 64,78 | H 6,85 | N 13,99 |
| Gef.: | 64,67 | 6,84 | 14,00 |

(9) (3S,5S)-5-[[6-(4-Amidinophenyl)-3-pyridazinyl]oxymethyl-3-carboxymethyl-1-(3-phenylpropyl)-2-pyrrolidinon x 1 $H_20$

$R_f$-Wert: 0,16 (Kieselgel; Methylenchlorid/Methanol/konz. wäßriges Ammoniak = 16:4:1)

| | | | |
|---|---|---|---|
| Ber.: | C 64,14 | H 6,18 | N 13,85 |
| Gef.: | 64,07 | 6,14 | 13,96 |

(10) (3S,5S)-5-[[6-(4-Amidinophenyl)-3-pyridazinyl]aminomethyl]-3-carboxymethyl-1-(3-phenylpropyl)-2-pyrrolidinon

$R_f$-Wert: 0,28 (Kieselgel; Methylenchlorid/Methanol/konz. wäßriges Ammoniak = 8:4:1)

(11) (3S,5S)-5-[[6-(4-Amidinophenyl)-3-pyridazinyl]aminomethyl]-3-carboxymethyl-2-pyrrolidinon

$R_f$-Wert: 0,13 (Kieselgel; Methylenchlorid/Methanol/konz. wäßriges Ammoniak = 8:4:1)

(12) (3S,5S)-5-[[[6-(4-Amidinophenyl)-3(2H)-pyridazinon-4-yl]carbonyl]aminomethyl]-3-carboxymethyl-2-pyrrolidinon

$R_f$-Wert: 0,094 (Kieselgel; Methylenchlorid/Methanol/konz. wäßriges Ammoniak = 8:4:1)

(13) (3S,5S)-5-[[[6-(4-Amidinophenyl)-2-methyl-3(2H)-pyridazinon-4-yl]carbonyl]aminomethyl]-3-carboxymethyl-2-pyrrolidinon

$R_f$-Wert: 0,24 (Kieselgel; Methylenchlorid/Methanol/konz. wäßriges Ammoniak = 8:4:1)

(14) (3S,5S)-5-[[[6-(4-Amidinophenyl)-3(2H)-pyridazinon4-yl]carbonyl]aminomethyl]-3-carboxymethyl-1-(3-phenylpropyl)-2-pyrrolidinon

$R_f$-Wert: 0,30 (Kieselgel; Methylenchlorid/Methanol/konz. wäßriges Ammoniak = 8:4:1)

(15) (3S,5S)-5-[[3-[(4-Amidinophenyl)aminocarbonyl]-2(1H)-pyridon-1-yl]methyl]-3-carboxymethyl-2-pyrrolidinon

$R_f$-Wert: 0,36 (Kieselgel; Methylenchlorid/Methanol/Eisessig = 30:10:1, zweifache Entwicklung)

(16) (3S,5S)-5-[[3-[(4-Amidinophenyl)aminocarbonyl]-2(1H)-pyridon-1-yl]methyl]-3-carboxymethyl-1-(3-phenylpropyl)-2-pyrrolidinon x 1,5 $H_2O$

$R_f$-Wert: 0,26 (Kieselgel; Tetrahydrofuran/1N-Salzsäure = 10:1)

| | | | |
|---|---|---|---|
| Ber.: | C 62,58 | H 6,16 | N 12,58 |
| Gef.: | 62,42 | 6,21 | 12,64 |

(17) (3S,5S)-5-[[5-[(4-Amidinophenyl)aminocarbonyl]-2(1H)-pyridon-1-yl]methyl]-3-carboxymethyl-1-(3-phenylpropyl)-2-pyrrolidinon x 1 $H_2O$

$R_f$-Wert: 0,33 (Kieselgel; Methylenchlorid/Methanol/Eisessig = 30:10:1)

| | | | |
|---|---|---|---|
| Ber.: | C 63,61 | H 6,07 | N 12,79 |
| Gef.: | 63,83 | 6,14 | 12,90 |

(18) (3S,5S)-5-[[5-[(4-Amidinophenyl)aminocarbonyl]-2,4-(1H,3H)-pyrimidindion-3-yl]methyl]-3-carboxymethyl-1-(3-phenylpropyl)-2-pyrrolidinon x 1 $H_2O$

$R_f$-Wert: 0,14 (Kieselgel; Methylenchlorid/Methanol/konz. wäßriges Ammoniak = 10:10:1)

| | | | |
|---|---|---|---|
| Ber.: | C 59,57 | H 5,71 | N 14,89 |
| Gef.: | 59,42 | 5,86 | 14,62 |

(19) (3S,55)-5-[[6-(4-Amidinopheny1)-3-pyridazinyl]oxymethyl]-3-carboxymethyl-2-pyrrolidinon
(20) (3S,5S)-5-[[4-(6-Amidino-3-pyridazinyl)phenyl]oxymethyl]-3-carboxymethyl-2-pyrrolidinon
(21) (3R,5R)-5-[[4-(5-Amidino-2-pyrimidyl)phenyl]oxymethyl]-3-carboxymethyl-2-pyrrolidinon
(22) (3R,5S)-5-[[4-(5-Amidino-2-pyrazinyl)phenyl]oxymethyl]-3-carboxymethyl-2-pyrrolidinon
(23) (3S,5S)-5-[(4'-Amidino-4-biphenylyl)oxymethyl]-3-carboxymethyl-1-(4-pyridylcarbonyl)-pyrrolidin
(24) (3S,5S)-5-[[4-(5-Amidino-2-pyrimidyl)phenyl]oxymethyl]-3-carboxymethyl-1-nicotinoyl-pyrrolidin
(25) (3S,5S)-5-[[4-(4-Amidinophenyl)-2-thiazolyl]aminomethyl]-3-carboxymethyl-2-pyrrolidinon
(26) (3S,5S)-5-[[1-(4-Amidinophenyl)piperidin-4-yl]aminomethyl]-3-carboxymethyl-2-pyrrolidinon
(27) (3S,5S)-5-[[1-(4-Amidinophenyl)piperidin-4-yl]oxymethyl]-3-carboxymethyl-1-(3-phenylpropyl)-2-pyrrolidinon
(28) (3S,5S)-5-[2-[1-(4-Amidinophenyl)piperazin-4-yl]ethyl]-3-carboxymethyl-2-pyrrolidinon
(29) (3R,5S)-5-[[4-(5-Amidino-2-thiazolyl)phenyl]oxymethyl]-3-(2-carboxyethyl)-2-pyrrolidinon
(30) (3S,5S)-5-[[1-(4-Amidinophenyl)piperidin-4-yl]oxymethyl]-3-carboxymethyl-2-pyrrolidinon

## Beispiel 5

### (3S,5S)-5-[[4-[5-(N-Methoxycarbonylamidino)-2-pyridyl]phenyl]-oxymethyl]-3-[(methoxycarbonyl)methyl]-2-pyrrolidinon

Zu 100 mg (3S,5S)-5-[[4-(5-Amidino-2-pyridyl)phenyl]oxymethyl]-3-[(methoxycarbonyl)methyl]-2-pyrrolidinon x 1,6 HCl x 1,5 $H_2O$ und 19,4 ml Chlorameisensäuremethylester in 10 ml Methylenchlorid werden unter kräftigem Rühren bei Raumtemperatur 3 ml 0,2 M Natronlauge zugetropft. Dann werden weitere 2 1/2 Stunden bei Raumtemperatur gerührt. Der Ansatz wird mit Methylenchlorid verdünnt, es wird die organische Phase abgetrennt, getrocknet und einrotiert.

Ausbeute: 50,4 mg (55 % der Theorie),
Schmelzpunkt: 180-183°C
Massenspektrum: $(M + H)^+ = 441$

Analog werden folgende Verbindungen erhalten:

(1) (3S,5S)-5-[[4-[2-(N-Methoxycarbonylamidino)-5-pyridyl]phenyl]oxymethyl]-3-[(methoxycarbonyl)methyl]-2-pyrrolidinon

Schmelzpunkt: 167-169°C
Massenspektrum: $(M + H)^+ = 441$

(2) (3S,5S)-5-[[4-[5-(N-Methoxycarbonylamidino)-2-pyrazinyl]phenyl]oxymethyl]-3-[(methoxycarbonyl)methyl]-2-pyrrolidinon

Schmelzpunkt: 210-212°C
Massenspektrum: $(M + H)^+ = 442$

(3) (3S,55)-5-[[4-[5-(N-Ethoxycarbonylamidino)-2-pyridyl]phenyl]oxymethyl]-3-[(ethoxycarbonyl)methyl]-2-pyrrolidinon

(4) (3S,5S)-5-[[4-[5-(N-Isobutoxycarbonylamidino)-2-pyrimidyl]phenyl]oxymethyl]-3-[(methoxycarbonyl)methyl]-2-pyrrolidinon

Beispiel 6

(3S,5S)-5-[(4'-Amidino-4-biphenylyl)oxymethyl]-3-[(methoxycarbonyl)methyl]-1-(2-pyrimidyl)-pyrrolidin x 2,25 HCl x 1 $H_2O$

12 ml trockenes Methanol werden unter Eiskühlung mit trockenem Salzsäuregas gesättigt. Die methanolische Salzsäure wird zu einer Lösung von 0,86 g (3S,5S)-5-[(4'-Cyano-4-biphenylyl)oxymethyl]-3-[(methoxycarbonyl)methyl]-1-(2-pyrimidyl)pyrrolidin in 2 ml trockenem-Methanol gegeben und die Mischung nochmals mit Salzsäure gesättigt. Der Ansatz wird mit Petrolether überschichtet und über Nacht stehen gelassen. Dann wird eingeengt und der Rückstand mit 12 ml trockenem Methanol und 0,9 g Ammoniumcarbonat 20 Stunden bei Raumtemperatur gerührt. Danach wird eingeengt und der Rückstand mit 20 ml Methylenchlorid/Methanol (85:15) gerührt. Es wird abgesaugt, das Filtrat wird eingeengt und mit 5 ml Methanol und 4 ml konzentriertem wäßrigen Ammoniak 18 Stunden bei Raumtemperatur gerührt. Danach wird mit Salzsäure angesäuert und eingeengt. Der Rückstand wird mit Methylenchlorid/Methanol (85:15) gerührt, dann wird filtriert und einrotiert. Nach Chromatographie über eine Kieselgelsäule werden 0,46 g (42 %) der Theorie erhalten.

$R_f$-Wert: 0,48 (Kieselgel; Methylenchlorid/Methanol = 4:1)

| | | | | |
|---|---|---|---|---|
| Ber.: | C 55,04 | H 5,77 | N 12,84 | Cl 14,62 |
| Gef.: | 54,78 | 5,70 | 12,74 | 14,32 |

Massenspektrum: $M^+ = 445$

Analog werden folgende Verbindungen erhalten:

(1) (3S,5S)-5-[(4'-Amidino-4-biphenylyl)oxymethyl]-3-[(methoxycarbonyl)methyl]-1-nicotinoyl-pyrrolidin-hydrochlorid

$R_f$-Wert: 0,48 (Kieselgel; Methylenchlorid/Methanol = 5:1)

(2) (3S,5S) 5-[(4`-Amidino-4-biphenylyl)oxymethyl]-3-[(methoxycarbonyl)methyl]-1-(2-pyridyl)-2-pyrrolidinon x 1,1

HCl x 0,5 $H_2O$

$R_f$-Wert: 0,23 (reversed phase Kieselgel; Methanol/100%ige wäßrige Kochsalzlösung = 6:4)

| | | | | |
|---|---|---|---|---|
| Ber.: | C 61,52 | H 5,58 | N 11,04 | CL 7,68 |
| Gef.: | 61,73 | 5,68 | 10,92 | 7,54 |

(3) (3S,5S)-5-[(4`-Amidino-4-biphenylyl)oxymethyl]-3-[(methoxycarbonyl)methyl]-1-(3-pyridyl)-2-pyrrolidinon x 1,2 HCl x 1 $H_2O$

$R_f$-Wert: 0,39 (reversed phase Kieselgel; Methanol/10%ige wäßrige Kochsalzlösung = 6:4)

| | | | | |
|---|---|---|---|---|
| Ber.: | C 60,02 H | 5,66 | N 10,77 | Cl 8,18 |
| Gef.: | 60,16 | 5,94 | 10,55 | 8,04 |

(4) (3S,5S)-5-[2-[(7-Amidino-1,2,3,4-tetrahydro-2-isochinolinyl)carbonylamino]ethyl]-3-[(methoxycarbonyl)methyl]-1-(3-phenylpropyl)-2-pyrrolidinon-hydrochlorid x 0,6 $H_20$

$R_f$-Wert: 0,29 (Kieselgel; Methylenchlorid/Methanol/konz. wäßriges Ammoniak = 80:20:5)

| | | | |
|---|---|---|---|
| Ber.: | C 61,44 | H 6,97 | N 12,35 |
| Gef.: | 61,32 | 6,94 | 12,58 |

(5) (3S,5S)-5-[[(7-Amidino-1,2,3,4-tetrahydro-2-isochinolinyl)carbonylamino]methyl]-3-[(methoxycarbonyl)methyl]-1-(3-phenylpropyl)-2-pyrrolidinon-hydrochlorid x 0,75 $H_20$

$R_f$-Wert: 0,50 (Kieselgel; Methylenchlorid/Methanol/konz. wäßriges Ammoniak = 80:20:5)

| | | | |
|---|---|---|---|
| Ber.: | C 60,53 | H 6,80 | N 12,60 |
| Gef.: | 60,57 | 6,86 | 12,54 |

(6) (3S,5S)-5-[[6-(4-Amidinophenyl)-3-pyridazinyl]oxymethyl]-3-[(methoxycarbonyl)methyl]-1-(3-phenylpropyl)-2-pyrrolidinon-hydrochlorid

$R_f$-Wert: 0,32 (Kieselgel; Methylenchlorid/Cyclohexan/Methanol/konz. wäßriges Ammoniak = 68:15:15:2)

(7) (3S,5S)-5-[[6-(4-Amidinophenyl)-3-pyridazinyl]aminomethyl]-3-[(methoxycarbonyl)methyl]-1-(3-phenylpropyl)-2-pyrrolidinon-hydrochlorid

$R_f$-Wert: 0,31 (Kieselgel; Methylenchlorid/Methanol/konz. wäßriges Ammoniak = 16:4:1)

(8) (3S,5S)-5-[[6-(4-Amidinophenyl)-3-pyridazinyl]aminomethyl]-3-[(methoxycarbonyl)methyl]-2-pyrrolidinon-hydrochlorid

$R_f$-Wert: 0,27 (Kieselgel; Methylenchlorid/Methanol/konz. wäßriges Ammoniak = 16:4:1)

(9) (3S,5S)-5-[[[6-(4-Amidinophenyl)-3(2H)-pyridazinon4-yl]carbonyl]aminomethyl]-3-[(methoxycarbonyl)methyl]-2-pyrrolidinon-hydrochlorid

$R_f$-Wert: 0,34 (Kieselgel; Methylenchlorid/Methanol/konz. wäßriges Ammoniak = 8:4:1)

(10) (3S,5S)-5-[[[6-(4-Amidinophenyl)-2-methyl-3(2H)-pyridazinon-4-yl]carbonyl]aminomethyl]-3-[(methoxycarbonyl)methyl]-2-pyrrolidinon-hydrochlorid

$R_f$-Wert: 0,31 (Kieselgel; Methylenchlorid/Methanol/konz. wäßriges Ammoniak = 16:4:1)

(11 (3S,5S)-5-[[[6-(4-Amidinophenyl)-3(2H)-pyridazinon-4-yl]carbonyl]aminomethyl]-3-[(methoxycarbonyl)methyl]-1-(3-phenylpropyl)-2-pyrrolidinon-hydrochlorid

$R_f$-Wert: 0,19 (Kieselgel; Methylenchlorid/Methanol/Cyclohexan/konz. wäßriges Ammoniak = 68:15:15:2)

(12) (3S,5S)-5-[[3-[(4-Amidinophenyl)aminocarbonyl]-2(1H)-pyridon-1-yl]methyl]-3-[(methoxycarbonyl)methyl]-2-pyrrolidinon-hydrochlorid

$R_f$-Wert: 0,55 (Kieselgel; Methylenchlorid/Methanol/Eisessig = 30:10:1)

(13) (3S,5S)-5-[[3-[(4-Amidinophenyl)aminocarbonyl]-2(1H)-pyridon-1-yl]methyl]-3-[(methoxycarbonyl)methyl]-1-(3-phenylpropyl)-2-pyrrolidinon-hydrochlorid

$R_f$-Wert: 0,54 (Kieselgel; Methylenchlorid/Methanol/Eisessig = 80:20:1)

(14) (3S,5S)-5-[[5-[(4-Amidinophenyl)aminocarbonyl]-2(1H)-pyridon-1-yl]methyl]-3-[(methoxycarbonyl)methyl]-1-(3-phenylpropyl)-2-pyrrolidinon-hydrochlorid

$R_f$-Wert: 0,44 (Kieselgel; Methylenchlorid/Methanol/Eisessig = 80:20:1)

(15) (3S,5S)-5-[[5-[(4-Amidinophenyl)aminocarbonyl]-2,4-(1H,3H)-pyrimidindion-3-yl]methyl]-3-[(methoxycarbonyl)methyl]-1-(3-phenylpropyl)-2-pyrrolidinon-hydrochlorid

$R_f$-Wert: 0,54 (Kieselgel; Methylenchlorid/Methanol = 3:1)

(16) (3S,5S)-5-[[6-(4-Amidinophenyl)-3-pyridazinyl]oxymethyl]-3-[(methoxycarbonyl)methyl]-2-pyrrolidinon-hydrochlorid
(17) (3S,5S)-5-[(4'-Amidino-4-biphenylyl)oxymethyl]-3-[(methoxycarbonyl)methyl]-1-(4-pyridylcarbonyl)-pyrrolidin-hydrochlorid
(18) (3S,5S)-5-[[4-(4-Amidinophenyl)-2-thiazolyl]aminomethyl]-3-[(methoxycarbonyl)methyl]-2-pyrrolidinon-hydrochlorid
(19) (3S,5S)-5-[[1-(4-Amidinophenyl)piperidin-4-yl]aminomethyl]-3-[(methoxycarbonyl)methyl]-2-pyrrolidinon-hydrochlorid
(20) (3S,5S)-5-[[1-(4-Amidinophenyl)piperidin-4-yl]oxymethyl]-3-[(methoxycarbonyl)methyl]-1-(3-phenylpropyl)-2-pyrrolidinon-hydrochlorid
(21) (3S,5S)-5-[2-[1-(4-Amidinophenyl)piperazin-4-yl]ethyl]-3-[(methoxycarbonyl)methyl]-2-pyrrolidinon-hydrochlorid
(22) (3S,5S)-5-[[1-(4-Amidinophenyl)piperidin-4-yl]oxymethyl]-3-[(methoxycarbonyl)methyl]-2-pyrrolidinon-hydrochlorid

Beispiel 7

(3S,5S)-5-[[4-(1-Amino-6-isochinolinyl)phenyl]oxymethyl]-3-[(methoxycarbonyl)methyl]-2-pyrrolidinon x 0,25 $H_20$

0,5 g 1-Amino-6-(4-hydroxyphenyl)isochinolin, 0,62 g (3S,5S)-5-[(Methansulfonyloxy)methyl]-3-[(methoxycarbonyl)methyl]-2-pyrrolidinon und 0,76 g Cäsiumcarbonat werden in 20 ml trockenem Dimethylformamid 48 Stunden bei 60°C gerührt. Es werden noch 0,3 g (3S,5S)-5-[(Methansulfonyloxy)methyl]-3-[(methoxycarbonyl)methyl]-2-pyrrolidinon und 0,5 g Cäsiumcarbonat zugeben und weitere 2 1/2 Tage bei 60°C gerührt. Nach dem Abkühlen wird auf 100 ml

Wasser gegossen und siebenmal mit je 50 ml Methylenchlorid extrahiert. Die vereinigten organischen Phasen werden getrocknet und eingeengt. Nach Chromatographie über eine Kieselgelsäule mit Essigester/Methanol (4:1) werden 0,25 g (29 % der Theorie) erhalten.

Schmelzpunkt: 220-222°C
$R_f$-Wert: 0,22 (Kieselgel; Essigester/Methanol = 4:1)

| | | | |
|---|---|---|---|
| Ber.: C | 67,39 | H 5,78 | N 10,25 |
| Gef.: | 67,54 | 5,98 | 9,95 |

Analog wird folgende Verbindung erhalten:

(1) (3S,5S)-5-[[4-(6-Isochinolinyl)phenyl]oxymethyl]-3-[(tert.butyloxycarbonyl)methyl]-2-pyrrolidinon

$R_f$-Wert: 0,23 (Kieselgel; Essigester)

Beispiel 8

(3S,5S)-3-[(tert.Butyloxycarbonyl)methyl]-5-[[4-(5-cyano-2-pyridyl)phenyl]oxymethyl]-2-pyrrolidinon

1,7 g 4-(5-Cyano-2-pyridyl)phenol, 3,3 g (3S,5S)-3-[(tert.Butyloxycarbonyl)methyl]-5-[(methansulfonyloxy)methyl]-2-pyrrolidinon und 3,6 g Cäsiumcarbonat werden in 25 ml trockenem Dimethylformamid 2 1/2 Tage bei Raumtemperatur gerührt. Der Ansatz wird mit 150 ml Wasser versetzt, es wird eine halbe Stunde gerührt und dann abgesaugt. Der abgesaugte Feststoff wird in Essigester gelöst, getrocknet und eingeengt. Das Rohprodukt wird durch Chromatographie über eine Kieselgelsäule mit Essigester gereinigt.

Ausbeute: 2,1 g (60 % der Theorie),
Schmelzpunkt: 125-127°C
$R_f$-Wert: 0,66 (Kieselgel; Essigester)

Analog werden folgende Verbindungen erhalten:

(1) (3S,5S)-3-[(tert.Butyloxycarbonyl)methyl]-5-[[4-(2-cyano-5-pyridyl)phenyl]oxymethyl]-2-pyrrolidinon

Schmelzpunkt: 117-118°C

$R_f$-Wert: 0,29 (Kieselgel; Essigester/Cyclohexan = 4:1)

(2) (3S,5S)-3-[(tert.Butyloxycarbonyl)methyl]-5-[[4-(5-cyano-2-pyrazinyl)phenyl]oxymethyl]-2-pyrrolidinon

Schmelzpunkt: 155-156°C
$R_f$-Wert: 0,37 (Kieselgel; Essigester/Cyclohexan = 4:1)

| | | | |
|---|---|---|---|
| Ber.: | C 64,69 | H 5,92 | N 13,72 |
| Gef.: | 64,50 | 6,02 | 13,50 |

(3) (3S,5S)-3-[(tert.Butyloxycarbonyl)methyl]-5-[[4-(2-cyano-2-pyrimidyl)phenyl]oxymethyl]-2-pyrrolidinon
Durchführung mit Kalium-tert.butylat statt Cäsiumcarbonat.

Schmelzpunkt: 152-154°C
$R_f$-Wert: 0,49 (Kieselgel; Essigester/Cyclohexan = 4:1)

| | | | |
|---|---|---|---|
| Ber.: | C 64,69 | H 5,92 | N 13,72 |
| Gef.: | 64,42 | 6,04 | 13,69 |

(4) (3S,55)-5-[[4-[(2-Benzylamino-4-pyridyl)aminocarbonyl]phenyl]oxymethyl]-3-[(methoxycarbonyl)methyl]-1-(3-phenylpropyl)-2-pyrrolidinon

$R_f$-Wert: 0,55 (Kieselgel; Methylenchlorid/Methanol = 9:1)

(5) (3S,5S)-5-[[4-[(2-Benzylamino-6-chlor-4-pyridyl)aminocarbonyl]phenyl]oxymethyl]-3-[(methoxycarbonyl)methyl]-1-(3-phenylpropyl)-2-pyrrolidinon

$R_f$-Wert: 0,53 (Kieselgel; Methylenchlorid/Methanol = 9:1)

(6) (3S,5S)-3-[(tert.Butyloxycarbonyl)methyl]-5-[[4-(5-cyano-2-pyrimidyl)phenyl]oxymethyl]-2-pyrrolidinon

Schmelzpunkt: 160-162°C
$R_f$-Wert: 0,51 (Kieselgel; Essigster/Cyclohexan = 6:1)

(7) (3S,5S)-3-[(tert.Butyloxycarbonyl)methyl]-5-[[4-(5-cyano-2-thiazolyl)phenyl]oxymethyl]-2-pyrrolidinon

Schmelzpunkt: 127-129°C
$R_f$-Wert: 0,48 (Kieselgel; Essigster/Cyclohexan = 6:1)

Beispiel 9

(3S,5S)-5-[(4'-Cyano-4-biphenylyl)oxymethyl]-3-[(methoxycarbonyl)methyl]-1-(2-pyrimidyl)-pyrrolidin

2,0 g (3S,5S)-5-[(4'-Cyano-4-biphenylyl)oxymethyl]-3-[(methoxycarbonyl)methyl]-pyrrolidin-hydrochlorid, 0,62 g 2-Chlorpyrimidin und 1,8 ml N-Ethyl-diisopropylamin werden 3 Stunden bei 140°C gerührt. Nach dem Abkühlen wird zwischen Wasser und Methylenchlorid verteilt, die organische Phase abgetrennt, getrocknet und eingeengt. Nach Reinigung über eine Kieselgelsäule mit Methylenchlorid/Essigester (9:1) werden 1,0 g (45 % der Theorie) erhalten.

$R_f$-Wert: 0,58 (Kieselgel; Methylenchlorid/Essigester = 9:1)

| | | | |
|---|---|---|---|
| Ber.: | C 70,07 | H 5,65 | N 13,08 |
| Gef.: | 69,80 | 5,69 | 12,86 |

Beispiel 10

(3S,5S)-5-[(4'-Cyano-4-biphenylyl)oxymethyl]-3-[(methoxycarbonyl)methyl]-1-nicotinoyl-pyrrolidin

Zu 3,0 g (3S,5S)-5-[(4'-Cyano-4-biphenylyl)oxymethyl]-3-[(methoxycarbonyl)methyl]-pyrrolidin-hydrochlorid in 50 ml Methylenchlorid werden 5 ml Triethylamin und dann 1,4 g Nicotinoylchlorid gegeben. Nach 3 Stunden wird einrotiert, der Rückstand mit 200 ml Wasser versetzt und mit Essigester extrahiert. Die organische Phase wird abgetrennt, getrocknet und einrotiert. Der Rückstand wird durch Chromatographie über eine Kieselgelsäule mit Methylenchlorid/Methanol (10:1) gereinigt.

Ausbeute: 2,4 g (68 % der Theorie),
Schmelzpunkt: 110-112°C
$R_f$-Wert: 0,62 (Kieselgel; Methylenchlorid/Methanol = 10:1)

| | | | |
|---|---|---|---|
| Ber.: | C 71,19 | H 5,53 | N 9,23 |
| Gef.: | 71,10 | 5,25 | 9,20 |

Beispiel 11

(3S,5S)-5-[2-[(2-Amidino-5-isoindolinyl)carbonylamino]ethyl]-3-carboxymethyl-1-(3-phenylpropyl)-2-pyrrolidinon

190 mg (3S,5S)-3-Carboxymethyl-5-[2-[(5-isoindolinyl)carbonylamino]ethyl]-1-(3-phenylpropyl)-2-pyrrolidinon, 34 mg S-Ethylisothioharnstoff-hydrobromid, 54 mg Natriumcarbonat und 3 ml trockenes Dimethylformamid werden 4 Stunden bei 100°C und 16 Stunden bei Raumtemperatur gerührt. Danach wird zur Trockene einrotiert und der Rückstand durch Chromatographie über eine Kieselgelsäule mit Methanol/konz. wäßriges Ammoniak (9:1) gereinigt.

Ausbeute: 49 mg (25 % der Theorie),
$R_f$-Wert: 0,22 (Kieselgel; Methylenchlorid/Methanol/konz. wäßriges Ammoniak = 35:15:4)

Analog werden folgende Verbindungen erhalten:

(1) (3S,5S)-5-[[(2-Amidino-5-isoindolinyl)carbonylamino]methyl]-3-carboxymethyl-1-(3-phenylpropyl)-2-pyrrolidinon x 2 $H_20$

$R_f$-Wert: 0,33 (Kieselgel; Methylenchlorid/Methanol/konz. wäßriges Ammoniak = 35:15:4)

| | | | |
|---|---|---|---|
| Ber.: | C 60,08 | H 6,86 | N 13,63 |
| Gef.: | 60,35 | 6,88 | 13,48 |

(2) (3S,5S)-5-[2-[(2-Amidino-1,2,3,4-tetrahydro-7-isochinolinyl)carbonylamino]ethyl]-3-carboxymethyl-1-(3-phenylpropyl)-2-pyrrolidinon

$R_f$-Wert: 0,34 (Kieselgel; Methanol/konz. wäßriges Ammoniak = 9:1)

(3) (3S,5S)-5-[[(2-Amidino-1,2,3,4-tetrahydro-7-isochinolinyl)carbonylamino]methyl]-3-carboxymethyl-1-(3-phenylpropyl)-2-pyrrolidinon

$R_f$-Wert: 0,36 (Kieselgel; Methanol/konz. wäßriges Ammoniak = 9:1)

| | | | |
|---|---|---|---|
| Ber.: | C 65,97 | H 6,77 | N 14,24 |
| Gef.: | 65,73 | 6,83 | 14,01 |

(4) (3S,5S)-5-[[4-(1-Amidino-4-piperidiny1)pheny1]oxymethyl]-3-carboxymethyl-1-(3-phenylpropyl)-2-pyrrolidinon x 1 $H_20$

$R_f$-Wert: 0,31 (Kieselgel; Methylenchlorid/Methanol/konz. wäßriges Ammoniak = 8:4:1)

| | | | |
|---|---|---|---|
| Ber.: | C 65,86 | H 7,50 | N 10,97 |
| Gef.: | 65,70 | 7,56 | 11,18 |

Beispiel 12

(3S,5S)-3-Carboxymethyl-5-[2-[(5-isoindolinyl)carbonylamino]-ethyl]-1-(3-phenylpropyl)-2-pyrrolidinon

Zu 510 mg (3S,5S)-5-[2-[(2-tert.Butyloxycarbonyl-5-isoindolinyl)carbonylamino]ethyl]-3-carboxymethyl-1-(3-phenylpropyl)-2-pyrrolidinon x 0,75 $H_20$ in 3 ml Methylenchlorid wird bei Raumtemperatur eine Mischung aus 3 ml Trifluoressigsäure und 3 ml Methylenchlorid zugetropft und danach eine Stunde bei Raumtemperatur gerührt. Es wird zur Trockene eingeengt, der Rückstand in 5 ml Methylenchlorid aufgenommen und mit methanolischem Ammoniak gerade alkalisch gestellt. Es wird einrotiert und der Rückstand durch Chromatographie über eine Kieselgelsäule mit Methylenchlorid/Methanol/konz. wäßriges Ammoniak (10:6:1) gereinigt.

Ausbeute: 250 mg (60 % der Theorie),
$R_f$-Wert: 0,46 (Kieselgel; Methylenchlorid/Methanol/konz. wäßriges Ammoniak = 35:15:4)
Massenspektrum: $(M + H)^+ = 450$

Analog werden folgende Verbindungen erhalten:

(1) (3S,5S)-3-Carboxymethyl-5-[[(5-isoindolinyl)carbonylamino]methyl]-1-(3-phenylpropyl)-2-pyrrolidinon

$R_f$-Wert: 0,48 (Kieselgel; Methylenchlorid/Methanol/konz. wäßriges Ammoniak = 35:15:4)

(2) (3S,5S)-3-Carboxymethyl-1-(3-phenylpropyl)-5-[2-[(1,2,3,4-tetrahydro-7-isochinolinyl)carbonylamino]ethyl]-2-pyrrolidinon

$R_f$-Wert: 0,40 (Kieselgel; Methanol/Essigester/konz. wäßriges Ammoniak = 20:10:1)

| | | | |
|---|---|---|---|
| Ber.: | C 69,95 | H 7,18 | N 9,06 |
| Gef.: | 70,10 | 7,26 | 8,99 |

(3) (3S,5S)-3-Carboxymethyl-1-(3-phenylpropyl)-5-[[(1,2,3,4-tetrahydro-7-isochinolinyl)carbonylamino]methyl]-2-pyrrolidinon

$R_f$-Wert: 0,38 (Kieselgel; Methanol/Essigester/konz. wäßriges Ammoniak = 20:10:1)

| | | | |
|---|---|---|---|
| Ber.: | C 69,47 | H 6,95 | N 9,35 |
| Gef.: | 69,64 | 7,02 | 9,09 |

(4) (3S,5S)-3-Carboxymethyl-1-(3-phenylpropyl)-5-[2-[[4-(4-piperidinyl)phenyl]carbonylamino]ethyl]-2-pyrrolidinon x 0,75 $H_20$

$R_f$-Wert: 0,32 (Kieselgel; Methylenchlorid/Methanol = 9:1),

| | | | |
|---|---|---|---|
| Ber.: | C 68,96 | H 7,68 | N 8,32 |
| Gef.: | 69,08 | 7,69 | 8,41 |

(5) (3S,5S)-3-Carboxymethyl-1-(3-phenylpropyl)-5-[[[4-(4-piperidinyl)phenyl]carbonylamino]methyl]-2-pyrrolidinon x 1 $H_20$

$R_f$-Wert: 0,13 (Kieselgel; Methanol/Essigester/konz. wäßriges Ammoniak = 20:10:1),

| | | | |
|---|---|---|---|
| Ber.: | C 67,85 | H 7,53 | N 8,48 |
| Gef.: | 68,03 | 7,52 | 8,51 |

(6) (3S,5S)-3-Carboxymethyl-1-(3-phenylpropyl)-5-[[4-(4-piperidinyl)phenyl]oxymethyl]-2-pyrrolidinon x 1 $H_20$

$R_f$-Wert: 0,14 (Kieselgel; Methylenchlorid/Essigester/konz. wäßriges Ammoniak = 20:10:1),

| | | | |
|---|---|---|---|
| Ber.: | C 69,21 | H 7,74 | N 5,98 |
| Gef.: | 69,38 | 7,71 | 6,15 |

Beispiel 13

(3S,5S)-5-[2-[(2-tert.Butyloxycarbonyl-5-isoindolinyl)carbonylamino]ethyl]-3-carboxymethyl-1-(3-phenylpropyl)-2-pyrrolidinon x 0,75 $H_20$

0,7 g (3S,5S)-5-[2-[(2-tert.Butyloxycarbonyl-5-isoindolinyl)carbonylamino]ethyl]-3-[(methoxycarbonyl)methyl)-1-(3-phenylpropyl)-2-pyrrolidinon x 0,5 $H_20$ in 3 ml Methanol werden mit 3,73 ml 1N Natronlauge versetzt und 3 Stunden bei Raumtemperatur gerührt. Der Methanolanteil wird abrotiert und der Rückstand durch Zugabe von gesättigter wäßriger Kaliumhydrogensulfat-Lösung angesäuert. Der Niederschlag wird abgesaugt und getrocknet.

Ausbeute: 510 mg (73 % der Theorie),
$R_f$-Wert: 0,48 (Kieselgel; Essigester/Eisessig = 50:1),

| | | | |
|---|---|---|---|
| Ber.: | C 66,12 | H 7,25 | N 7,46 |
| Gef.: | 66,08 | 7,30 | 7,42 |

Analog werden folgende Verbindungen erhalten:

(1) (3S,5S)-5-[[(2-tert.Butyloxycarbonyl-5-isoindolinyl)carbonylamino]methyl]-3-carboxymethyl-1-(3-phenylpropyl)-2-pyrrolidinon x 1,2 $H_20$

$R_f$-Wert: 0,54 (Kieselgel; Essigester/Eisessig = 50:1),

| | | | |
|---|---|---|---|
| Ber.: | C 64,66 | H 7,13 | N 7,54 |
| Gef.: | 64,42 | 6,87 | 7,69 |

(2) (3S,5S)-5-[2-[(2-tert.Butyloxycarbonyl-1,2,3,4-tetrahydro-7-isochinolinyl)carbonylamino]ethyl]-3-carboxymethyl-1-(3-phenylpropyl)-2-pyrrolidinon x 0,75 $H_20$

| | | | |
|---|---|---|---|
| Ber.: | C 66,59 | H 7,42 | N 7,28 |
| Gef.: | 66,49 | 7,39 | 7,33 |

(3) (3S,5S)-5-[[(2-tert.Butyloxycarbonyl-1,2,3,4-tetrahydro-7-isochinolinyl)carbonylamino]methyl]-3-carboxymethyl-1-(3-phenylpropyl)-2-pyrrolidinon x 0,75 $H_20$

| | | | |
|---|---|---|---|
| Ber.: | C 66,11 | H 7,25 | N 7,46 |
| Gef.: | 66,05 | 7,26 | 7,52 |

Beispiel 14

(3S,5S)-5-[2-[(2-tert.Butyloxycarbonyl-5-isoindolinyl)carbonylamino]ethyl]-3-[(methoxycarbonyl)methyl]-1-(3-phenylpropyl)-2-pyrrolidinon x 0,5 $H_20$

0,75 g 2-tert.Butyloxycarbonyl-5-isoindolincarbonsäure in 15 ml trockenem Dimethylformamid werden bei -10°C mit 0,41 g 1-Hydroxy-1H-benztriazol, 1,06 g (3S,5S)-5-(2-Aminoethyl)-3-[(methoxycarbonyl)methyl]-1-(3-phenylpropyl)-2-pyrrolidinon-hydrochlorid in 10 ml Dimethylformamid, 0,83 ml Triethylamin und 0,74 g N,N'-Dicyclohexylcarbodiimid versetzt. Die Reaktionsmischung wird 16 Stunden gerührt und dabei langsam auf Raumtemperatur erwärmt. Es wird zur Trockene eingeengt, der Rückstand mit 50 ml Wasser versetzt und viermal mit 20 ml Essigester extrahiert. Die vereinigten organischen Phasen werden viermal mit gesättigter wäßriger Natriumhydrogencarbonat-Lösung und zweimal mit gesättigter wäßriger Kochsalzlösung gewaschen, getrocknet und einrotiert. Der Rückstand wird durch Chromatographie über eine Kieselgelsäule mit Essigester gereinigt.

Ausbeute: 0,7 g (42 % der Theorie),
$R_f$-Wert: 0,33 (Kieselgel; Essigester)

| | | | |
|---|---|---|---|
| Ber.: | C 67,11 | H 7,39 | N 7,34 |
| Gef.: | 67,26 | 7,65 | 7,44 |

Analog werden folgende Verbindungen erhalten:

(1) (3S,5S)-5-[[(2-tert.Butyloxycarbonyl-5-isoindolinyl)carbonylamino]methyl]-3-[(methoxycarbonyl)methyl]-1-(3-phenylpropyl)-2-pyrrolidinon x 0,25 $H_20$

$R_f$-Wert: 0,40 (Kieselgel; Essigester)

| | | | |
|---|---|---|---|
| Ber.: | C 67,19 | H 7,18 | N 7,58 |
| Gef.: | 67,12 | 7,49 | 7,66 |

(2) (3S,5S)-5-[2-[(2-tert.Butyloxycarbonyl-1,2,3,4-tetrahydro-7-isochinolinyl)carbonylamino]ethyl]-3-[(methoxycarbonyl)-methyl]-1-(3-phenylpropyl)-2-pyrrolidinon

$R_f$-Wert: 0,35 (Kieselgel; Essigester)

(3) (3S,5S)-5-[[(2-tert.Butyloxycarbonyl-1,2,3,4-tetrahydro-7-isochinolinyl)carbonylamino]methyl]-3-[(methoxycarbonyl)-methyl]-1-(3-phenylpropyl)-2-pyrrolidinon

$R_f$-Wert: 0,47 (Kieselgel; Essigester)

Beispiel 15

(3S,5S)-5-[(4'-Cyano-4-biphenylyl)oxymethyl]-3-[(methoxycarbonyl)methyl]-1-(2-pyridyl)-2-pyrrolidinon

Zu 6 g (3R,5S)3-Allyl-5-[(4'-cyano-4-biphenylyl)oxymethyl]-1-(2-pyridyl)-2-pyrrolidinon in 60 ml Methylenchlorid werden 50 ml Acetonitril, 300 mg Ruthenium-III-chlorid-hydrat, 19,7 g Natriummetaperjodat und 110 ml Wasser zugegeben und sehr kräftig gerührt. Nach einer Stunde Reaktionszeit werden 110 ml Wasser und 60 ml Methylenchlorid zugesetzt und das Reaktionsgemisch über Kieselgur abgesaugt. Die organische Phase wird mit Natriumsulfit-Lösung und Wasser gewaschen, getrocknet und einrotiert. Der Rückstand wird mit 80 ml Methanol und einigen Millilitern methanolischer Salzsäure versetzt und 30 Minuten bei Raumtemperatur stehen gelassen. Der Ansatz wird einrotiert und zwischen Essigester und 1M Natriumcarbonat-Lösung verteilt. Die organische Phase wird mit Wasser gewaschen, getrocknet und einrotiert. Der Rückstand wird durch Chromatographie über eine Kieselgelsäule mit Cyclohexan/Essigester (75:25) gereinigt.

Ausbeute: 2,8 g (44 % der Theorie),
Schmelzpunkt: 125-127°C
$R_f$-Wert: 0,38 (Kieselgel; Cyclohexan/Essigester = 7:3)

| | | | |
|---|---|---|---|
| Ber.: C | 70,73 | H 5,25 | N 9,52 |
| Gef.: | 70,46 | 5,35 | 9,61 |

Analog wird folgende Verbindung erhalten:

(1) (3S,5S)-5-[(4'-Cyano-4-biphenylyl)oxymethyl]-3-[(methoxycarbonyl)methyl]-1-(3-pyridyl)-2-pyrrolidinon

$R_f$-Wert: 0,39 (Kieselgel; Cyclohexan/Essigester = 1:9)

Beispiel 16

(3S,5S)-5-[[(7-Cyano-1H-2,3,4,5-tetrahydro-3-benzazepin-3-yl)carbonylamino]methyl]-3-[(methoxycarbonyl)methyl]-1-(3-phenylpropyl)-2-pyrrolidinon x 0,25 $H_20$

Zu 0,5 g Carbonyldiimidazol und 0,35 g Imidazol in 15 ml trockenem Tetrahydrofuran wird bei 0°C innerhalb von 5 Minuten die filtrierte Mischung aus 1,0 g (3S,5S)-5-Amino-methyl-3-[(methoxycarbonyl)methyl]-1-(3-phenylpropyl)-2-pyrrolidinonhydrochlorid, 0,4 ml Triethylamin und 20 ml trockenem Tetrahydrofuran zugetropft. Nach 12 Minuten rühren bei 0°C werden 0,57 g 7-Cyano-1H-2,3,4,5-tetrahydro-3-benzazepin in 15 ml Tetrahydrofuran zugegeben. Es wird noch eine halbe Stunde bei 0°C und 4 Stunden bei Raumtemperatur gerührt, dann wird eingeengt und der Rückstand zwischen Essigester und verdünnter wäßriger Salzsäure verteilt. Die organische Phase wird abgetrennt, getrocknet und eingeengt. Nach Chromatographie über eine Kieselgelsäule mit Essigester/Methanol (20:1) werden 0,84 g (56 % der Theorie) erhalten.

$R_f$-Wert: 0,56 (Kieselgel; Essigester/Methanol = 20:1)

| | | | |
|---|---|---|---|
| Ber.: | C 68,69 | H 6,86 | N 11,05 |
| Gef.: | 68,60 | 6,97 | 10,89 |

Analog werden folgende Verbindungen erhalten:

(1) (3S,5S)-5-[2-[(7-Cyano-1,2,3,4-tetrahydro-2-isochinolinyl)carbonylamino]ethyl]-3-[(methoxycarbonyl)methyl]-1-(3-phenylpropyl)-2-pyrrolidinon x 0,7 $H_20$

$R_f$-Wert: 0,49 (Kieselgel; Essigester/Methanol = 19:1)

| | | | |
|---|---|---|---|
| Ber.: | C 67,61 | H 6,93 | N 10,87 |
| Gef.: | 67,78 | 7,13 | 10,60 |

(2) (3S,5S)-5-[[(7-Cyano-1,2,3,4-tetrahydro-2-isochinolinyl)carbonylamino]methyl]-3-[(methoxycarbonyl)methyl]-1-(3-phenylpropyl)-2-pyrrolidinon x 0,25 $H_2O$

$R_f$-Wert: 0,25 (Kieselgel; Essigester)

| | | | |
|---|---|---|---|
| Ber.: | C 68,20 | H 6,64 | N 11,36 |
| Gef.: | 68,27 | 6,80 | 10,97 |

Beispiel 17

(3S,5S)-5-[[(3-tert.Butyloxycarbonyl-1H-2,3,4,5-tetrahydro-3-benzazepin-7-yl)carbonylamino]methyl]-3-carboxymethyl-1-(3-phenylpropyl)-2-pyrrolidinon

Zu 6,6 g (3R,5S)-3-Allyl-5-[[(3-tert.butyloxycarbonyl-1H-2,3,4,5-tetrahydro-3-benzazepin-7-yl)carbonylamino]methyl]-1-(3-phenylpropyl)-2-pyrrolidinon in 30 ml Acetonitril und 30 ml Tetrachlorkohlenstoff werden unter starkem Rühren 17,1 g Natriummetaperjodat in 125 ml Wasser und 80 mg Ruthenium-(III)chlorid-hydrat zugegeben und 5 Stunden bei Raumtemperatur kräftig gerührt. Das Reaktionsgemisch wird über Kieselgur filtriert, mit Methylenchlorid extrahiert, die vereinigten organischen Phasen werden zweimal mit je 100 ml 4%iger Natriumhydrogensulfitlösung, mit Wasser und mit gesättigter Kochsalzlösung gewaschen, getrocknet, über Aktivkohle filtriert und einrotiert. Der Rückstand wird durch Chromatographie über eine Kieselgelsäule mit Methylenchlorid/Cyclohexan/Methanol/konz. wäßriges Ammoniak (68:15:15:2) gereinigt.

Ausbeute: 4 g (52 % der Theorie),
$R_f$-Wert: 0,22 (Kieselgel; Methylenchlorid/Cyclohexan/Methanol/konz. wäßriges Ammoniak = 68:15:15:2)

Analog werden folgende Verbindungen erhalten:

(1) (3S,5S)-5-[[[4-(1-tert.Butyloxycarbonyl-4-piperidinyl)phenyl]carbonylamino]methyl]-3-carboxymethyl-1-(3-phenylpropyl)-2-pyrrolidinon

$R_f$-Wert: 0,25 (Kieselgel; Methylenchlorid/Cyclohexan/Methanol/konz. wäßriges Ammoniak = 68:15:15:2)

(2) (3S,5S)-5-[[4-(1-tert.Butyloxycarbonyl-4-piperidinyl)phenyl]oxymethyl]-3-carboxymethyl-1-(3-phenylpropyl)-2-pyrrolidinon

$R_f$-Wert: 0,38 (Kieselgel; Methylenchlorid/Cyclohexan/Methanol/konz. wäßriges Ammoniak = 68:15:15:2)

(3) (3S,5S)-3-Carboxymethyl-5-[[6-(4-cyanophenyl)-3-pyridazinyl]oxymethyl]-1-(3-phenylpropyl)-2-pyrrolidinon

$R_f$-Wert: 0,31 (Kieselgel; Methylenchlorid/Cyclohexan/Methanol/konz. wäßriges Ammoniak = 68:15:15:2)

Beispiel 18

(3S,5S)-5-[2-[(3-tert.Butyloxycarbonyl-1H-2,3,4,5-tetrahydro-3-benzazepin-7-yl)carbonylamino]ethyl]-3-carboxymethyl-1-(3-phenylpropyl)-2-pyrrolidinon

3,8 g (3S,5S)-5-Aminoethyl-3-carboxymethyl-1-(3-phenylpropyl)-2-pyrrolidinon, 50 ml trockenes Methylenchlorid, 50 ml trockenes Acetonitril und 1,8 ml Chlortrimethylsilan werden 30 Minuten bei Raumtemperatur und 2,5 Stunden

bei 45°C gerührt, dann abgekühlt und eingeengt. Der Rückstand wird in 75 ml trockenem Tetrahydrofuran aufgenommen und auf 0°C abgekühlt (Reaktionslösung a)).
4 g 3-tert.Butyloxycarbonyl-1H-2,3,4,5-tetrahydro-3-benzazepin-7-carbonsäure, 50 ml trockenes Tetrahydrofuran und 1,5 ml N-Methyl-morpholin werden bei -30°C tropfenweise mit 1,8 ml Chlorameisensäure-isobutylester versetzt und 30 Minuten bei dieser Temperatur gerührt. Dann wird Reaktionslösung a) und 1,5 ml N-Methyl-morpholin bei -30°C zugegeben. Es wird 3 Stunden bei -30°C gerührt und über Nacht unter Rühren auf Raumtemperatur erwärmt. Der Ansatz wird mit wäßriger Zitronensäurelösung angesäuert, mit Diethylether extrahiert und die vereinigten organischen Phasen mit 10%iger Zitronensäurelösung und Wasser gewaschen, getrocknet und einrotiert. Nach Reinigung über eine Kieselgelsäule mit Diethylether/Tetrahydrofuran/Wasser (40:20:1) werden 4,1 g (57 % der Theorie) erhalten.

$R_f$-Wert: 0,25 (Kieselgel; Diethylether/Tetrahydrofuran/Wasser = 30:20:1)

Analog wird folgende Verbindung erhalten:

(1) (3S,5S)-5-[2-[[4-[4-(N-tert.Butyloxycarbonyl)piperidinyl]phenyl]carbonylamino]ethyl]-3-carboxymethyl-1-(3-phenylpropyl)-2-pyrrolidinon

$R_f$-Wert: 0,32 (Kieselgel; Diethylether/Tetrahydrofuran/Wasser = 30:20:1)

<u>Beispiel 19</u>

<u>(3S,5S)-5-[[[4-(1-Amidino-4-piperidinyl)phenyl]carbonylamino]-methyl]-3-carboxymethyl-1-(3-phenylpropyl)-2-pyrrolidinon x 1 $H_2O$</u>

0,5 g (3S,5S)-3-Carboxymethyl-1-(3-phenylpropyl)-5-[[[4-(4-piperidinyl)phenyl]carbonylamino]methyl]-2-pyrrolidinon x 1 $H_2O$, 6 ml Ethanol, 0,06 ml Eisessig und 85 mg Cyanamid werden 2 Tage am Rückfluß gekocht, dann abgekühlt und einrotiert. Der Rückstand wird mit Wasser verrieben, das Wasser abdekantiert und der Rückstand mit Aceton verrieben. Der Feststoff wird abgesaugt, getrocknet und durch Chromatographie über eine Kieselgelsäule mit Methanol/konz. wäßriges Ammoniak (95:5) gereinigt.

Ausbeute: 0,2 g (37 % der Theorie),

| | | | |
|---|---|---|---|
| Ber.: | C 64,78 | H 7,31 | N 13,03 |
| Gef.: | 64,71 | 7,18 | 13,10 |

<u>Beispiel 20</u>

<u>(3S,5S)-5-[[6-(4-Cyanophenyl)-3-pyridazinyl]oxymethyl]-3-[(methoxycarbonyl)methyl]-1-(3-phenylpropyl)-2-pyrrolidinon</u>

2,7 g (3S,5S)-3-Carboxymethyl-5-[[6-(4-cyanophenyl)-3-pyridazinyl]oxymethyl]-1-(3-phenylpropyl)-2-pyrrolidinon werden mit 50 ml Methanol und 5 ml methanolischer Salzsäure 45 Minuten bei ca. 50°C gerührt und dann einrotiert. Nach Reinigung über eine Kieselgelsäule mit Methylenchlorid/Methanol (40:1) verbleiben 1,9 g (68 % der Theorie).

$R_f$-Wert: 0,58 (Kieselgel; Methylenchlorid/Methanol = 15:1)

Analog werden folgende Verbindungen erhalten:

(1) (3S,5S)-5-[[6-(4-Cyanophenyl)-3-pyridazinyl]aminomethyl]-3-[(methoxycarbonyl)methyl]-1-(3-phenylpropyl)-2-pyrrolidinon

$R_f$-Wert: 0,86 (Kieselgel; Methylenchlorid/Methanol/Cyclohexan/konz. wäßriges Ammoniak = 68:15:15:2)

(2) (3S,5S)-5-[[4-(5-Amidino-2-pyrimidyl)phenyl]oxymethyl]-3-[(isopropoxycarbonyl)methyl]-2-pyrrolidinon-hydrochlorid Durchführung mit isopropanolischer Salzsäure.

(3) (3S,5S)-3-[(Methoxycarbonyl)methyl]-5-[[4-(1,2,3,4-tetrahydro-6-isochinolinyl)phenyl]oxymethyl]-2-pyrrolidinon-hydrochlorid

(4) (3S,5S)-5-[[4-(5-Amidino-2-pyridyl)phenyl]oxymethyl]-3-[(ethyloxycarbonyl)methyl]-2-pyrrolidinon-hydrochlorid

(5) (3S,5S)-5-[[4-(5-Amidino-2-pyrimidyl)phenyl]oxymethyl]-3-[(cyclohexyloxycarbonyl)methyl]-2-pyrrolidinon-hydrochlorid

Beispiel 21

(3S,5S)-5-[[6-(4-Cyanophenyl)-3-pyridazinyl]aminomethyl]-3-[(methoxycarbonyl)methyl]-2-pyrrolidinon

1,2 g (3S,5S)-5-Aminomethyl-3-[(methoxycarbonyl)methyl]-2-pyrrolidinon, 1,3 g 3-Chlor-6-(4-cyanophenyl)-pyridazin, 0,64 g Natriumcarbonat und 10 ml Dimethylsulfoxid werden 5 Stunden bei 120°C gerührt, anschließend abgekühlt und in eine gesättigte wäßrige Kochsalzlösung eingerührt und mit Essigester, der etwas Tetrahydrofuran enthält, extrahiert. Die organische Phase wird mit Wasser gewaschen, getrocknet, filtriert und einrotiert. Das Rohprodukt wird in ca. 150 ml einer Mischung aus Methylenchlorid und Methanol gelöst, es werden ca. 8 g Kieselgel zugegeben und dann wieder einrotiert. Dieser Rückstand wird auf eine Kieselgelsäule gegeben und das Produkt mit Methylenchlorid/Methanol (15:1) eluiert.

Ausbeute: 0,95 g (43 % der Theorie),
$R_f$-Wert: 0,38 (Kieselgel; Methylenchlorid/Methanol = 15:1)

Beispiel 22

(3S,5S)-5-[[[6-(4-Cyanophenyl)-3(2H)-pyridazinon-4-yl]carbonyl]aminomethyl]-3-[(methoxycarbonyl)methyl]-2-pyrrolidinon

1,2 g 6-(4-Cyanophenyl)-3(2H)-pyridazinon-4-carbonsäure und 0,8 ml N-Methyl-morpholin werden in 100 ml Dimethylformamid erhitzt und die erhaltene Suspension auf -20°C abgekühlt. Bei dieser Temperatur werden 0,7 ml Chlorameisensäure-isobutylester dazugegeben und etwa 1,5 Stunden bei -20°C gerührt. Dazu werden 0,95 g (3S,5S)-5-Aminomethyl-3-[(methoxycarbonyl)methyl]-2-pyrrolidinon in 20 ml Dimethylformamid zugetropft und dann 3 Stunden bei -20°C gerührt. Anschließend wird langsam auf Raumtemperatur erwärmt, das Reaktionsgemisch in gesättigte Kochsalzlösung eingerührt und mit Essigester/Tetrahydrofuran extrahiert. Die organische Phase wird mit Wasser gewaschen, getrocknet, filtriert und einrotiert. Nach Reinigung über eine Kieselgelsäule mit Methylenchlorid/Methanol (15:1) verbleiben 0,7 g (34 % der Theorie).

$R_f$-Wert: 0,31 (Kieselgel; Methylenchlorid/Methanol = 15:1)

Analog werden folgende Verbindungen erhalten:

(1) (3S,5S)-5-[[[6-(4-Cyanophenyl)-2-methyl-3(2H)-pyridazinon-4-yl]carbonyl]aminomethyl]-3-[(methoxycarbonyl)methyl]-2-pyrrolidinon
Durchführung in Tetrahydrofuran

$R_f$-Wert: 0,49 (Kieselgel; Methylenchlorid/Methanol = 15:1)

(2) (3S,5S)-5-[[[6-(4-Cyanophenyl)-3(2H)-pyridazinon-4-yl]carbonyl]aminomethyl]-3-[(methoxycarbonyl)methyl]-1-(3-phenylpropyl)-2-pyrrolidinon
Durchführung in Dimethylsulfoxid/Tetrahydrofuran

$R_f$-Wert: 0,34 (Kieselgel; Methylenchlorid/Methanol = 15:1)

Beispiel 23

(3S,5S)-5-[[3-[(4-Cyanophenyl)aminocarbonyl]-2(1H)-pyridon-1-yl]methyl]-3-[(methoxycarbonyl)methyl]-2-pyrrolidinon

29,9 g 3-[(4-Cyanophenyl)aminocarbonyl]-2(1H)-pyridon und 17,3 g Kaliumcarbonat in 250 ml Dimethylsulfoxid werden eine Stunde bei 80°C gerührt. Dann wird eine Lösung von 33,3 g (3S,5S)-5-[(Methansulfonyloxy)methyl]-3-[(methoxycarbonyl)methyl]-2-pyrrolidinon in Dimethylsulfoxid zugegeben und 27 Stunden bei 80°C gerührt. Nach dem Abkühlen wird das Reaktionsgemisch auf 4 l Eis gegeben, die Suspension 1,5 Stunden verrührt, dann abgesaugt und der Filterrückstand zweimal mit je 100 ml Wasser gewaschen. Der Filterrückstand wird bei 85°C in 500 ml Dimethylformamid gelöst, die Lösung in der Wärme auf ein Gesamtvolumen von ca. 75 ml eingeengt und abgekühlt. Nach Stehen über Nacht wird das Kristallisat abgesaugt, mit 25 ml kaltem Dimethylformamid und zweimal mit je 75 ml Essigester, 50 ml Aceton und Diethylether gewaschen und getrocknet. Ausbeute: 14,7 g (29 % der Theorie),

$R_f$-Wert: 0,57 (Kieselgel; Methylenchlorid/Methanol/Eisessig = 30:1:0,1; zweifache Entwicklung)

Analog werden folgende Verbindungen erhalten:

(1) (3S,5S)-5-[[3-[(4-Cyanophenyl)aminocarbonyl]-2(1H)-pyridon-1-yl]methyl]-3-[(methoxycarbonyl)methyl]-1-(3-phenylpropyl)-2-pyrrolidinon
Reinigung über eine Kieselgelsäule mit Methylenchlorid/Methanol (40:1)

$R_f$-Wert: 0,54 (Kieselgel; Methylenchlorid/Methanol = 30:1; zweifache Entwicklung)

(2) (3S,5S)-5-[[5-[(4-Cyanophenyl)aminocarbonyl]-2(1H)-pyridon-1-yl]methyl]-3-[(methoxycarbonyl)methyl]-1-(3-phenylpropyl)-2-pyrrolidinon
Reinigung über eine Kieselgelsäule mit Essigester/Cyclohexan = 4:1 und 9:1

$R_f$-Wert: 0,18 (Kieselgel; Essigester/Cyclohexan = 4:1)

(3) (3S,5S)-5-[[5-[(4-Cyanophenyl)aminocarbonyl]-2,4(1H,3H)-pyrimidindion-3-yl]methyl]-3-[(methoxycarbonyl)methyl]-1-(3-phenylpropyl)-2-pyrrolidinon
Durchführung mit Natriumhydrogencarbonat. Reinigung über eine Kieselgelsäule mit Methylenchlorid/Methanol (100:1), Methylenchlorid/Methanol/konz. wäßriges Ammoniak (50:1:0,1) und Essigester/Cyclohexan (5:1)

$R_f$-Wert: 0,32 (Kieselgel; Methylenchlorid/Methanol/konz. wäßriges Ammoniak = 19:1:0,1)

Beispiel 24

(3S,5S)-5-[[4-[(2-Benzylamino-4-pyridyl)aminocarbonyl]phenyl]oxymethyl]-3-carboxymethy1-1-(3-phenylpropyl)-2-pyrrolidinon

0,3 g (3S,5S)-5-[[4-[(2-Benzylamino-4-pyridyl)aminocarbonyl]phenyl]oxymethyl]-3-[(methoxycarbonyl)methyl]-1-(3-phenylpropyl)-2-pyrrolidinon, 8 ml Methanol und 1,5 ml 1N Natronlauge werden 30 Minuten auf dem Dampfbad erhitzt. Dann wird abgekühlt, mit Wasser verdünnt, mit 1N Salzsäure angesäuert und eingedampft. Der Rückstand wird durch Chromatographie über eine Kieselgelsäule mit Methylenchlorid/Methanol/konz. wäßriges Ammoniak (85:15:1) und (80:20:2) gereinigt.

Ausbeute: 0,15 g (52 % der Theorie),
$R_f$-Wert: 0,40 (Kieselgel; Methylenchlorid/Methanol/konz. wäßriges Ammoniak = 80:20:2)

Analog wird folgende Verbindung erhalten:

(1) (3S,5S)-5-[[4-[(2-Benzylamino-6-chlor-4-pyridyl)aminocarbonyl]phenyl]oxymethyl]-3-carboxymethyl-1-(3-phenylpropyl)-2-pyrrolidinon

Beispiel 25

(3S,5S)-3-Carboxymethyl-5-[[4-(1,2,3,4-tetrahydro-6-isochinolinyl)phenyl]oxymethyl]-2-pyrrolidinon-hydrochlorid

2,3 g (3S,5S)-3-Carboxymethyl-5-[[4-(6-isochinolinyl)phenyl]oxymethyl]-2-pyrrolidinon werden in 200 ml Eisessig und 20 ml 2N Salzsäsure in Gegenwart von 0,5 g Platin-(IV)-oxid unter einem Wasserstoffdruck von 3 bar bei Raumtemperatur hydriert. Der Katalysator wird abfiltriert und das Filtrat zur Trockene eingedampft. Es wird mehrmals Toluol zugegeben und jeweils zur Trockene eingedampft. Der Rückstand wird mit 50 ml Aceton verrieben, der Feststoff

abgesaugt und mehrmals mit Aceton nachgewaschen.

Ausbeute: 1,8 g (70 % der Theorie),
$R_f$-Wert: 0,60 (reversed phase Kieselgel; Methanol/10%ige wäßrige Kochsalzlösung = 6:4)
Massenspektrum: $(M + H)^+ = 381$

Beispiel 26

Trockenampulle mit 2,5 mg Wirkstoff pro 1 ml

Zusammensetzung:

| | |
|---|---|
| Wirkstoff | 2,5 mg |
| Mannitol | 50,0 mg |
| Wasser für Injektionszwecke | ad 1,0 ml |

Herstellung:
Wirkstoff und Mannitol werden in Wasser gelöst. Nach Abfüllung wird gefriergetrocknet.
Die Auflösung zur gebrauchsfertigen Lösung erfolgt mit Wasser für Injektionszwecke.

Beispiel 27

Trockenampulle mit 35 mg Wirkstoff pro 2 ml

Zusammensetzung:

| | |
|---|---|
| Wirkstoff | 35,0 mg |
| Mannitol | 100,0 mg |
| Wasser für Injektionszwecke | ad 2,0 ml |

Herstellung:
Wirkstoff und Mannitol werden in Wasser gelöst. Nach Abfüllung wird gefriergetrocknet.
Die Auflösung zur gebrauchsfertigen Lösung erfolgt mit Wasser für Injektionszwecke.

Beispiel 28

Tablette mit 50 mg Wirkstoff

Zusammensetzung:

| | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |

(fortgesetzt)

| | |
|---|---|
| | 215,0 mg |

Herstellung:
(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 9 mm.

Beispiel 29

Tablette mit 350 mg Wirkstoff

Zusammensetzung:

| | |
|---|---|
| (1) Wirkstoff | 350,0 mg |
| (2) Milchzucker | 136,0 mg |
| (3) Maisstärke | 80,0 mg |
| (4) Polyvinylpyrrolidon | 30,0 mg |
| (5) Magnesiumstearat | 4,0 mg |
| | 600,0 mg |

Herstellung:
(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

Beispiel 30

Kapseln mit 50 mg Wirkstoff

Zusammensetzung:

| | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2.0 mg |
| | 160,0 mg |

Herstellung:
(1) wird mit (3) verrieben. Diese Verreibung wird der Mischung aus (2) und (4) unter intensiver Mischung zugegeben.
Diese Pulvermischung wird auf einer Kapselabfüllmaschine in Hartgelatine-Steckkapseln Größe 3 abgefüllt.

Beispiel 31

Kapseln mit 350 mg Wirkstoff

Zusammensetzung:

| | |
|---|---|
| (1) Wirkstoff | 300,0 mg |
| (2) Maisstärke getrocknet | 46,0 mg |
| (3) Milchzucker pulverisiert | 30,0 mg |
| (4) Magnesiumstearat | 4.0 mg |
| | 430,0 mg |

Herstellung:
(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 0 abgefüllt.

**Patentansprüche**

1. Cyclische Iminoderivate der allgemeinen Formel

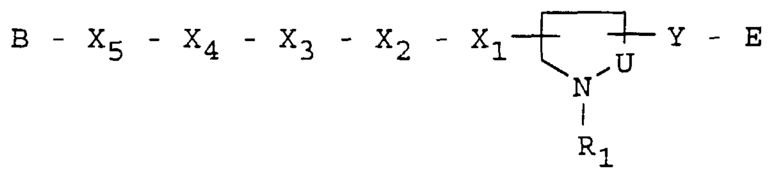

, (I)

in der mit der Maßgabe, daß mindestens einer der Reste $R_1$, $X_3$, $X_5$, $X_5$-$X_4$-$X_3$ zusammen oder $X_5$-$X_4$ zusammen einen heterocyclischen Rest enthält, und der kürzeste Abstand zwischen den Gruppen B und E mindestens 10 Bindungen beträgt,

U eine Methylengruppe und $R_1$ ein Wasserstoffatom, eine Pyridincarbonyl- oder Pyrimidinylgruppe oder

U eine Carbonylgruppe und $R_1$ ein Wasserstoffatom, eine 3-Phenylpropyl- oder Pyridinylgruppe,

$X_1$ eine Methylen- oder Ethylengruppe,

$X_2$ eine Bindung, ein Sauerstoffatom, eine -CONH- oder Iminogruppe,

$X_3$ eine gegebenenfalls durch eine Methylgruppe substituierte 1,4-Phenylen-, Pyridazin-3,6-ylen-, 1H-Pyridin-2-on-1,3-ylen-, 1H-Pyridin-2-on-1,5-ylen-, 2H-Pyridazin-3-on-4,6-ylen- oder 1H,3H-Pyrimidin-2,4-dion-3,5-ylen-gruppe mit der Maßgabe, daß ein Stickstoffatom der Gruppe $X_3$ nicht mit einem Heteroatom oder der -CONH-Gruppe der Gruppe $X_2$ verknüpft ist,

$X_4$ eine Bindung oder eine -NHCO-Gruppe mit der Maßgabe, daß die -NHCO-Gruppe nicht mit einem Stickstoffatom der Gruppe $X_3$ verknüpft ist, und

$X_5$ eine Piperidin-1,4-ylen-gruppe, eine gegebenenfalls durch ein Chloratom substituierte 1,4-Phenylen-, Pyridin-2,4-ylen-, Pyridin-2,5-ylen-, Pyrimidin-2,5-ylen-, Pyrazin-2,5-ylen- oder Thiazol-2,5-ylen-gruppe mit der Maßgabe, daß das Ringstickstoffatom der Piperidin-1,4-ylengruppe nicht mit einem Stickstoffatom der Gruppe $X_3$ oder $X_4$ verknüpft ist, oder

$X_5$-$X_4$-$X_3$ zusammen auch eine Isoindolin-2,5-ylen- oder 1,2,3,4-Tetrahydro-isochinolin-2,7-ylen-gruppe mit der Maßgabe, daß das Ringstickstoffatom dieser Gruppen nicht mit einem Heteroatom der Gruppe $X_2$ verknüpft ist, oder

$X_5$-$X_4$ zusammen auch eine Isochinolin-1,6-ylen-gruppe, wobei der Rest B in Stellung 1 und der Rest $X_3$ in

Stellung 6 steht, oder eine 1,2,3,4-Tetrahydroisochinolin-2,6-ylen-gruppe, wobei der Rest B in Stellung 2 und der Rest $X_3$ in Stellung 6 steht,

B eine Cyanogruppe, eine Amidinogruppe, in der an einem der Stickstoffatome ein Wasserstoffatom durch eine Alkoxycarbonylgruppe mit insgesamt 2 bis 5 Kohlenstoffatomen ersetzt sein kann, eine gegebenenfalls durch eine Benzylgruppe substituierte Aminogruppe mit der Maßgabe, daß die Aminogruppe nicht mit einem Ringstickstoffatom der Gruppe $X_5$-$X_4$-$X_3$ oder $X_5$ verknüpft ist, oder, falls B mit einem Ringstickstoffatom der Gruppe $X_5$-$X_4$-$X_3$ oder $X_5$ verknüpft ist oder, falls B mit der Gruppe $X_5$-$X_4$ verknüpft ist, zusätzlich auch ein Wasserstoffatom,

Y eine Methylengruppe und
E eine Carboxygruppe oder eine Alkoxycarbonylgruppe mit insgesamt 2 bis 5 Kohlenstoffatomen bedeuten,

deren Stereoisomere, deren Tautomere, deren Gemische und deren Salze.

**2.** Cyclische Iminoderivate der allgemeinen Formel I gemäß Anspruch 1, in der mit der Maßgabe, daß mindestens einer der Reste $R_1$, $X_3$, $X_5$, $X_5$-$X_4$-$X_3$ zusammen oder $X_5$-$X_4$ zusammen einen heterocyclischen Rest enthält, und der kürzeste Abstand zwischen den Gruppen B und E mindestens 10 Bindungen beträgt,

U eine Methylengruppe und $R_1$ ein Wasserstoffatom, eine Pyridin-3-carbonyl- oder Pyrimidin-2-ylgruppe oder

U eine Carbonylgruppe und $R_1$ ein Wasserstoffatom, eine 3-Phenylpropyl-, Pyridin-2-yl- oder Pyridin-3-ylgruppe,

$X_2$-$X_1$ eine -O-$CH_2$-, -NH-$CH_2$-, -CONH-$CH_2$-, -CONH-$CH_2CH_2$ - oder, falls $X_3$ eine 1H-Pyridin-2-on-1,3-ylen-, 1H-Pyridin-2-on-1,5-ylen- oder 1H,3H-Pyrimidin-2,4-dion-3,5-ylen-gruppe darstellt, auch eine Methylengruppe,

$X_3$ eine 1,4-Phenylen-, Pyridazin-3,6-ylen-, 1H-Pyridin-2-on-1,3-ylen-, 1H-Pyridin-2-on-1,5-ylen-, 2H-Pyridazin-3-on-4,6-ylen-, 2-Methyl-2H-pyridazin-3-on-4,6-ylen- oder 1H,3H-Pyrimidin-2,4-dion-3,5-ylen-Gruppe, wobei die 1H-Pyridin-2-on-1,3-ylen-, 1H-Pyridin-2-on-1,5-ylen- und die 1H,3H-Pyrimidin-2,4-dion-3,5-ylengruppe über ein Ringstickstoffatom an die Gruppe $X_2$-$X_1$ gebunden ist,

$X_4$ eine Bindung oder eine -NHCO-Gruppe und

$X_5$ eine 1,4-Phenylen-, Pyridin-2,4-ylen-, Pyridin-2,5-ylen-, Pyrimidin-2,5-ylen-, Pyrazin-2,5-ylen-, Piperidin-1,4-ylen-oder Thiazol-2,5-ylen-Gruppe, wobei die Piperidin-1,4-ylen- und die Pyridin-2,4-ylengruppe jeweils über die Position 4 an $X_4$ gebunden ist, oder

$X_5$-$X_4$-$X_3$ zusammen auch eine Isoindolin-2,5-ylen-gruppe, wobei diese über den Benzoteil an die Gruppe $X_2$-$X_1$ gebunden ist, oder

$X_5$-$X_4$ zusammen auch einen Isochinolin-1,6-ylen- oder 1,2,3,4-Tetrahydroisochinolin-2,6-ylen-ring, falls $X_3$ eine 1,4-Phenylengruppe darstellt, wobei der Isochinolin-1,6-ylen- und 1,2,3,4-Tetrahydroisochinolin-2,6-ylen-ring jeweils über die Position 6 an die 1,4-Phenylengruppe gebunden ist,

B eine Amidinogruppe, in der an einem der Stickstoffatome ein Wasserstoffatom durch eine Alkoxycarbonylgruppe mit insgesamt 2 bis 5 Kohlenstoffatomen ersetzt sein kann, oder, falls $X_5$ eine 1,4-Piperidinylengruppe darstellt, zusätzlich auch ein Wasserstoffatom, oder, falls $X_5$-$X_4$ zusammen einen Isochinolin-1,6-ylen-ring darstellt, eine Aminogruppe oder ein Wasserstoffatom oder, falls $X_5$-$X_4$ einen 1,2,3,4-Tetrahydroisochinolin-2,6-ylen-ring darstellt, ein Wasserstoffatom oder, falls $X_5$ einen Pyridin-2,4-ylenring darstellt, eine Benzylaminogruppe,

Y eine Methylengruppe und

E eine Carboxygruppe oder eine Alkoxycarbonylgruppe mit insgesamt 2 bis 5 Kohlenstoffatomen bedeuten,

deren Stereoisomere, deren Tautomere, deren Gemische und deren Salze.

3. Cyclische Iminoderivate der allgemeinen Formel I gemäß Anspruch 1, in der $R_1$, U, B, E, $X_1$ bis $X_5$ und Y wie im Anspruch 2 erwähnt definiert sind und der Rest -Y-E in Position 3 und der Rest B-$X_5$-$X_4$-$X_3$-$X_2$-$X_1$- in Position 5 des 2-Pyrrolidinon- oder Pyrrolidinringes steht, und einer der Reste $X_5$ oder $X_3$ eine 1,4-Phenylengruppe darstellt, deren Stereoisomere, deren Tautomere, deren Gemische und deren Salze.

4. Folgende cyclische Iminoderivate der allgemeinen Formel I gemäß Anspruch 1:

   (1) (3S,5S)-5-[[4-(5-Amidino-2-pyridyl)phenyl]oxymethyl]-3-carboxymethyl-2-pyrrolidinon,

   (2) (3S,5S)-5-[[4-(2-Amidino-5-pyrimidyl)phenyl]oxymethyl]-3-carboxymethyl-2-pyrrolidinon,

   (3) (3S,5S)-5-[[4-(5-Amidino-2-pyrimidyl)phenyl]oxymethyl]-3-carboxymethyl-2-pyrrolidinon,

   (4) (3S,5S)-5-[[4-(5-Amidino-2-thiazolyl)phenyl]oxymethyl]-3-carboxymethyl-2-pyrrolidinon,

   (5) (3S,5S)-5-[[4-(2-Amidino-5-pyridyl)phenyl]oxymethyl]-3-carboxymethyl-2-pyrrolidinon,

   (6) (3S,5S)-5-[[4-(5-Amidino-2-pyrazinyl)phenyl]oxymethyl]-3-carboxymethyl-2-pyrrolidinon,

   (7) (3S,5S)-5-[(4'-Amidino-4-biphenylyl)oxymethyl]-3-carboxymethyl-1-(2-pyrimidyl)-pyrrolidin,

   (8) (3S,5S)-5-[(4'-Amidino-4-biphenylyl)oxymethyl]-3-carboxymethyl-1-nicotinoyl-pyrrolidin,

   (9) (3S,5S)-5-[(4'-Amidino-4-biphenylyl)oxymethyl]-3-carboxymethyl-1-(2-pyridyl)-2-pyrrolidinon,

   (10) (3S,5S)-5-[(4'-Amidino-4-biphenylyl)oxymethyl]-3-carboxymethyl-1-(3-pyridyl)-2-pyrrolidinon,

   (11) (3S,5S)-5-[[6-(4-Amidinophenyl)-3-pyridazinyl]oxymethyl-3-carboxymethyl-1-(3-phenylpropyl)-2-pyrrolidinon,

   (13) (3S,5S)-5-[[6-(4-Amidinophenyl)-3-pyridazinyl]amino-methyl]-3-carboxymethyl-2-pyrrolidinon,

   (14) (3S,5S)-5-[[4-[5-(N-Methoxycarbonylamidino)-2-pyridyl]phenyl]oxymethyl]-3-[(methoxycarbonyl)methyl]-2-pyrrolidinon,

   (15) (3S,5S)-5-[[4-[2-(N-Methoxycarbonylamidino)-5-pyridyl]-phenyl]oxymethyl]-3-[(methoxycarbonyl)methyl]-2-pyrrolidinon,

   (16) (3S,5S)-5-[[4-[5-(N-Methoxycarbonylamidino)-2-pyrazinyl]phenyl]oxymethyl]-3-[(methoxycarbonyl)methyl]-2-pyrrolidinon,

   deren Tautomere und deren Salze.

5. Physiologisch verträgliche Salze der Verbindungen nach mindestens einem der Ansprüche 1 bis 4 mit anorganischen oder organischen Säuren oder Basen.

6. Arzneimittel, enthaltend eine Verbindung nach mindestens einem der Ansprüche 1 bis 4, in denen B mit Ausnahme der Cyanogruppe die in den Ansprüchen 1 bis 4 angegebenen Bedeutungen aufweist, oder ein entsprechendes physiologisch verträgliches Salz gemäß Anspruch 5 neben gegebenenfalls einem oder mehreren inerten Trägerstoffen und/oder Verdünnungsmitteln.

7. Verwendung einer Verbindung nach mindestens einem der Ansprüche 1 bis 4, in denen B mit Ausnahme der Cyanogruppe die in den Ansprüchen 1 bis 4 angegebenen Bedeutungen aufweist, oder ein entsprechendes physiologisch verträgliches Salz gemäß Anspruch 5 zur Herstellung eines Arzneimittels, das zur Bekämpfung bzw. Verhütung von Krankheiten, bei denen kleinere oder größere Zell-Aggregate auftreten oder Zell-Matrixinteraktionen eine Rolle spielen, geeignet ist.

8. Verfahren zur Herstellung eines Arzneimittels gemäß Anspruch 6, dadurch gekennzeichnet, daß auf nichtchemischem Wege eine Verbindung nach mindestens einem der Ansprüche 1 bis 4, in denen B mit Ausnahme der Cyanogruppe die in den Ansprüchen 1 bis 4 angegebenen Bedeutungen aufweist, oder ein entsprechendes physiologisch verträgliches Salz gemäß Anspruch 5 in einen oder mehrere inerte Trägerstoffe und/oder Verdünnungsmittel eingearbeitet wird.

**9.** Verfahren zur Herstellung der cyclischen Iminoderivate gemäß den Ansprüchen 1 bis 5, dadurch gekennzeichnet, daß

a) zur Herstellung von Verbindungen der allgemeinen Formel I, in der E eine Carboxygruppe darstellt, eine Verbindung der allgemeinen Formel

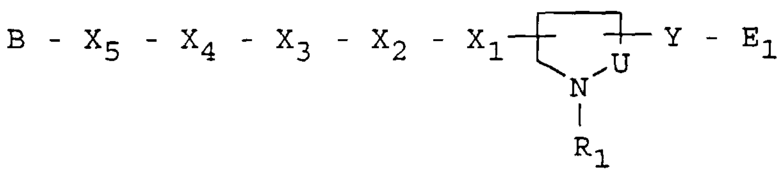

, (II)

in der

$R_1$, B, U, $X_1$ bis $X_5$ und Y wie in den Ansprüchen 1 bis 4 definiert sind und
$E_1$, das an ein Kohlenstoffatom gebunden ist, eine mittels Hydrolyse, Behandlung mit Säuren, Thermolyse oder Hydrogenolyse in eine Carboxygruppe überführbare Gruppe darstellt, in eine Verbindung der allgemeinen Formel I, in der E eine Carboxygruppe darstellt, übergeführt oder

b) zur Herstellung von Verbindungen der allgemeinen Formel I, in der B eine Amidinogruppe darstellt, eine gegebenenfalls im Reaktionsgemisch gebildete Verbindung der allgemeinen Formel

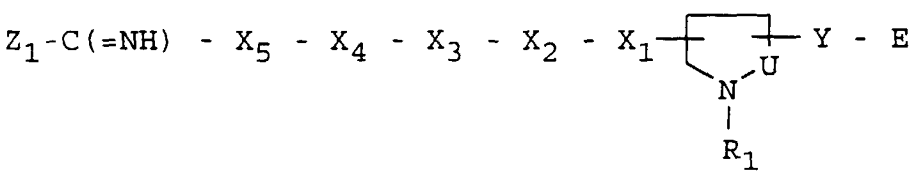

, (III)

in der

$R_1$, E, U, $X_1$ bis $X_5$ und Y wie in den Ansprüchen 1 bis 4 definiert sind und
$Z_1$ eine Alkoxy-, Aralkoxy-, Alkylthio- oder Aralkylthiogruppe darstellt, mit Ammoniak oder mit dessen Säureadditionssalzen umgesetzt wird oder

c) zur Herstellung von Verbindungen der allgemeinen Formel I, in der B eine durch eine Benzylgruppe substituierte Aminogruppe darstellt, eine Verbindung der allgemeinen Formel

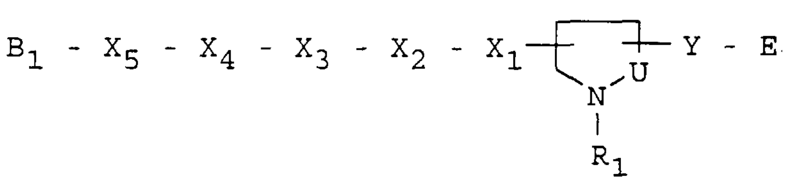

, (IV)

in der

$R_1$, $X_1$ bis $X_5$, E, U und Y wie in den Ansprüchen 1 bis 4 definiert sind und
$B_1$ eine Aminogruppe darstellt, mit einer Verbindung der allgemeinen Formel

$$Z_2 - R_b\ ,\qquad (V)$$

in der
$R_b$ eine Benzylgruppe und
$Z_2$ eine nukleophile Austrittsgruppe bedeuten, umgesetzt wird oder

d) zur Herstellung von Verbindungen der allgemeinen Formel I, in der $X_2$ ein Sauerstoffatom oder eine Iminogruppe darstellt, eine Verbindung der allgemeinen Formel

$$B - X_5 - X_4 - X_3 - X_2' - H\ ,\qquad (VI)$$

in der

$X_3$ bis $X_5$ und B wie in den Ansprüchen 1 bis 4 definiert sind und
$X_2'$ ein Sauerstoffatom oder eine Iminogruppe darstellt, oder deren Alkali- oder Erdalkalisalze mit einer Verbindung der allgemeinen Formel

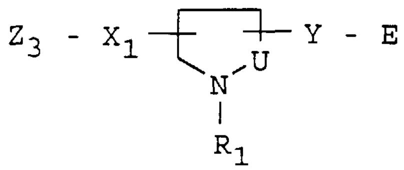
, (VII)

in der
$R_1$, $X_1$, E, U und Y wie in den Ansprüchen 1 bis 4 definiert sind und
$Z_3$ eine nukleophile Austrittsgruppe bedeutet, umgesetzt wird oder

e) zur Herstellung von Verbindungen der allgemeinen Formel I, in der $R_1$ eine Pyridincarbonylgruppe darstellt, eine Verbindung der allgemeinen Formel

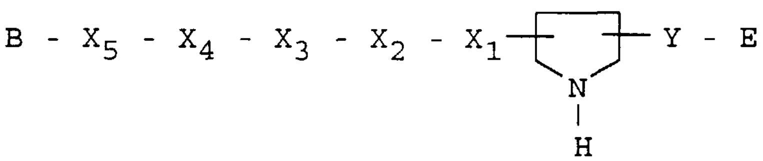
, (VIII)

in der

$X_1$ bis $X_5$, B, E und Y wie in den Ansprüchen 1 bis 4 definiert sind, mit einer Verbindung der allgemeinen Formel

$$Z_4 - R_1'\ ,\qquad (IX)$$

in der
$R_1'$ eine Pyridincarbonylgruppe und
$Z_4$ eine Hydroxy- oder Austrittsgruppe bedeuten, acyliert wird oder

f) zur Herstellung von Verbindungen der allgemeinen Formel I, in der $X_2$ eine -CONH- Gruppe darstellt, eine Verbindung der allgemeinen Formel

$$B - X_5 - X_4 - X_3 - E_2\ ,\qquad (X)$$

in der

$X_3$ bis $X_5$ und B wie in den Ansprüchen 1 bis 4 definiert sind und

$E_2$ eine Carboxygruppe darstellt, mit einer Verbindung der allgemeinen Formel

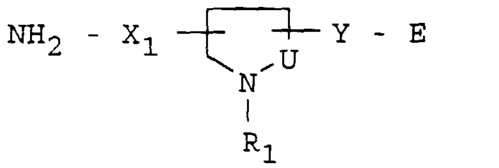

, (XI)

in der

$R_1$, $X_1$, Y, E und U wie in den Ansprüchen 1 bis 4 definiert sind, oder mit deren reaktionsfähigen Derivaten umgesetzt wird oder

g) zur Herstellung von Verbindungen der allgemeinen Formel I, in der E eine Carboxygruppe oder eine Alkoxycarbonylgruppe mit insgesamt 2 bis 5 Kohlenstoffatomen darstellt, eine Verbindung der allgemeinen Formel

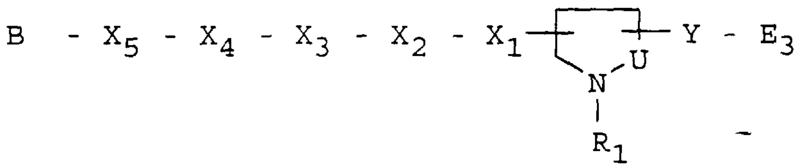

, (XII)

in der

$R_1$, B, U, $X_1$ bis $X_5$ und Y wie in den Ansprüchen 1 bis 4 definiert sind und
$E_3$ eine Vinyl- oder 1,2-Dihydroxyalkylgruppe darstellt, oxidiert und erforderlichenfalls anschließend eine so erhaltene Verbindung mit einem entsprechenden Alkohol verestert wird oder

h) zur Herstellung von Verbindungen der allgemeinen Formel I, in der $R_1$ eine Pyrimidinylgruppe darstellt, eine Verbindung der allgemeinen Formel

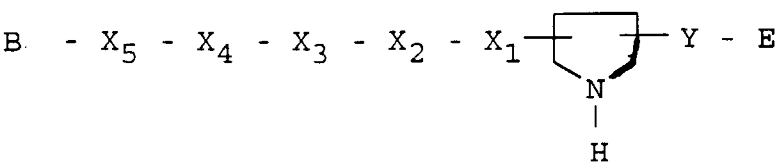

, (XIII)

in der

$X_1$ bis $X_5$, B, E und Y wie in den Ansprüchen 1 bis 4 definiert sind, mit einer Verbindung der allgemeinen Formel

$$Z_6 - R_1'' , \qquad (XIV)$$

in der
$R_1''$ eine Pyrimidinylgruppe und
$Z_6$ eine nukleophile Austrittsgruppe bedeuten, umgesetzt wird oder

i) zur Herstellung von Verbindungen der allgemeinen Formel I, in der E eine Alkoxycarbonylgruppe mit insgesamt 2 bis 5 Kohlenstoffatomen darstellt, eine Verbindung der allgemeinen Formel

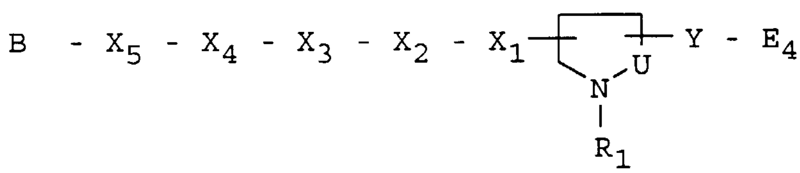 , (XV)

in der

$R_1$, B, U, $X_1$ bis $X_5$ und Y wie in den Ansprüchen 1 bis 4 definiert sind und
$E_4$ eine Carboxygruppe oder deren reaktionsfähigen Derivate darstellt, mit einer Verbindung der allgemeinen Formel

$$H - R_e , \qquad (XVI)$$

in der
$R_e$ eine Alkoxygruppe mit 1 bis 4 Kohlenstoffatomen darstellt, umgesetzt wird oder

j) zur Herstellung von Verbindungen der allgemeinen Formel I in der $X_2$ eine -CONH-Gruppe darstellt und $X_5$-$X_4$-$X_3$ eine cyclische Iminogruppe enthält, deren Ringstickstoffatom mit der Carbonylgruppe von $X_2$ verknüpft ist, eine Verbindung der allgemeinen Formel

$$B - X_5 - X_4 - X_3 -H , \qquad (XVII)$$

in der

B und $X_5$-$X_4$-$X_3$ wie in den Ansprüchen 1 bis 4 definiert sind, mit einer Verbindung der allgemeinen Formel

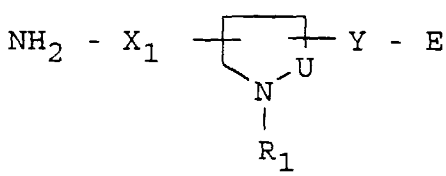 , (XVIII)

in der
$R_1$, E, U, $X_1$ und Y wie in den Ansprüchen 1 bis 4 definiert sind, in Gegenwart einer Verbindung der allgemeinen Formel

$$Z_7 - CO - Z_8 , \qquad (XIX)$$

in der
$Z_7$ und $Z_8$, die gleich oder verschieden sein können, nukleophile Austrittsgruppen bedeuten, umgesetzt wird oder

k) zur Herstellung von Verbindungen der allgemeinen Formel I, in der $X_2$ eine Iminogruppe und $X_3$ eine der in den Ansprüchen 1 bis 4 erwähnten Heteroarylengruppen darstellt, eine Verbindung der allgemeinen Formel

$$B - X_5 - X_4 - X_3' - Z_9 , \qquad (XX)$$

in der

B, $X_4$ und $X_5$ wie in den Ansprüchen 1 bis 4 definiert sind,
$X_3$' eine der für $X_3$ in den Ansprüchen 1 bis 4 erwähnten Heteroarylengruppen und
$Z_9$ eine nukleophile Austrittsgruppe bedeuten, mit einer Verbindung der allgemeinen Formel

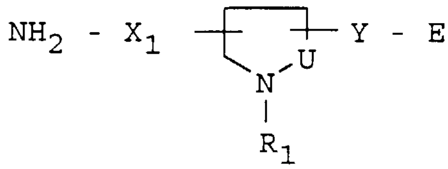 , (XVIII)

in der
$R_1$, E, U, Y und $X_1$ wie in den Ansprüchen 1 bis 4 definiert sind, umgesetzt wird oder

l) zur Herstellung von Verbindungen der allgemeinen Formel I, in der $X_2$ eine Bindung, $X_3$ eine der in den Ansprüchen 1 bis 4 erwähnten Heteroarylengruppen darstellt und $X_3$ mit dem Ringstickstoffatom einer -CO-N-Gruppe an $X_1$ gebunden ist, eine Verbindung der allgemeinen Formel

$$B - X_5 - X_4 - X_3'' - H, \qquad (XXI)$$

in der

B, $X_4$ und $X_5$ wie in den Ansprüchen 1 bis 4 definiert sind und
$X_3''$ eine der für $X_3$ in den Ansprüchen 1 bis 4 erwähnten Heteroarylengruppen, in der eine oder zwei zu einem Stickstoffatom benachbarte Methingruppen jeweils durch eine Hydroxymethingruppe ersetzt sind, darstellt, oder deren Tautomere mit einer Verbindung der allgemeinen Formel

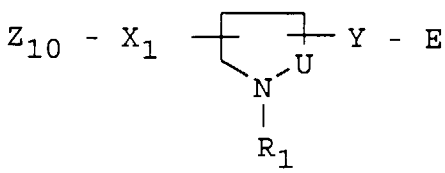 , (XXII)

in der
$R_1$, E, U, $X_1$ und Y wie in den Ansprüchen 1 bis 4 definiert sind und
$Z_{10}$ eine nukleophile Austrittsgruppe bedeutet, umgesetzt wird oder

m) zur Herstellung von Verbindungen der allgemeinen Formel I, in der B, $B-X_5-X_4-X_3$, $B-X_5-X_4$ oder $B-X_5$ eine durch eine Alkoxycarbonylgruppe mit insgesamt 2 bis 5 Kohlenstoffatomen substituierte Amidinogruppe enthält, eine Verbindung der allgemeinen Formel

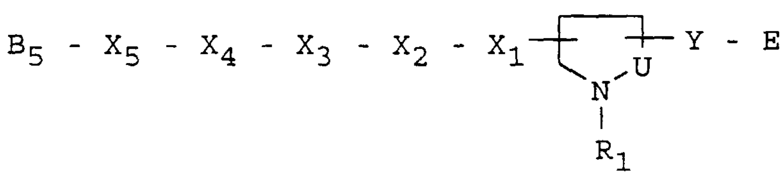 , (XXIII)

in der

$R_1$, E, U, $X_1$ bis $X_5$ und Y wie in den Ansprüchen 1 bis 4 definiert sind und
$B_5$, $B_5-X_5-X_4-X_3$, $B_5-X_5-X_4$ oder $B_5-X_5$ eine Amidinogruppe enthält, mit einer Verbindung der allgemeinen Formel

$$Z_{11} - R_f, \qquad (XXIV)$$

in der

$R_f$ eine Alkoxycarbonylgruppe mit insgesamt 2 bis 5 Kohlenstoffatomen und
$Z_{11}$ eine nukleophile Austrittsgruppe bedeuten, umgesetzt wird oder

n) zur Herstellung von Verbindungen der allgemeinen Formel I, in der E eine Alkoxycarbonylgruppe mit insgesamt 2 bis 5 Kohlenstoffatomen darstellt, eine Verbindung der allgemeinen Formel

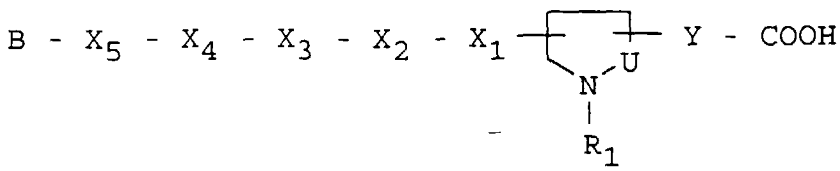

, (XXV)

in der

$R_1$, B, U, $X_1$ bis $X_5$ und Y wie in den Ansprüchen 1 bis 4 definiert sind, mit einer Verbindung der allgemeinen Formel

$$Z_{12} - E_5 , \qquad (XXVI)$$

in der

$E_5$ eine Alkylgruppe mit 1 bis 4 Kohlenstoffatomen und $Z_{12}$ eine nukleophile Austrittsgruppe darstellen, umgesetzt wird und

gewünschtenfalls anschließend eine so erhaltene Verbindung der allgemeinen Formel I, die eine Carbonylbrücke enthält, mittels Reduktion in eine entsprechende Verbindung der allgemeinen Formel I, die eine Methylenbrücke enthält, übergeführt wird und/oder

eine so erhaltene Verbindung der allgemeinen Formel I, die eine Estergruppe enthält, mittels Umesterung in einen entsprechenden Ester übergeführt wird und/oder

erforderlichenfalls ein während der Umsetzungen zum Schutze von reaktiven Gruppen verwendeter Schutzrest abgespalten wird und/oder

gewünschtenfalls eine so erhaltene Verbindung der allgemeinen Formel I in ihre cis-/trans-Isomere, in ihre Enantiomeren und/oder Diastereomeren aufgetrennt wird und/oder

eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.

## Claims

1. Cyclic imino derivatives of general formula

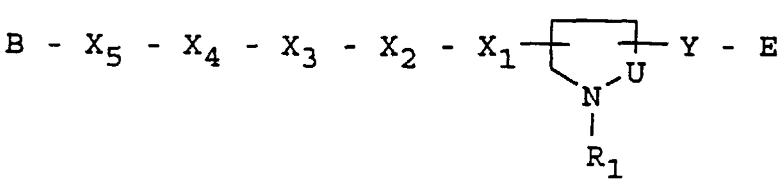

(I)

wherein, with the proviso that at least one of the groups $R_1$, $X_3$, $X_5$, $X_5$-$X_4$-$X_3$ together or $X_5$-$X_4$ together contains a heterocyclic group, and the shortest distance between the groups B and E is at least 10 bonds,

U denotes a methylene group and $R_1$ denotes a hydrogen atom, a pyridinocarbonyl or pyrimidinyl group or

U denotes a carbonyl group and $R_1$ denotes a hydrogen atom, a 3-phenylpropyl or pyridinyl group,

$X_1$ denotes a methylene or ethylene group,

$X_2$ denotes a bond, an oxygen atom, a -CONH- group or an imino group,

$X_3$ denotes an optionally methyl-substituted 1,4-phenylene, pyridazin-3,6-ylene, 1H-pyridin-2-on-1,3-ylene, 1H-pyridin-2-on-1,5-ylene, 2H-pyridazin-3-on-4,6-ylene or 1H,3H-pyrimidin-2,4-dion-3,5-ylene group, with the proviso that a nitrogen atom of the group $X_3$ is not linked to a heteroatom or to the -CONH group of the group $X_2$,

$X_4$ represents a bond or an -NHCO- group, with the proviso that the -NHCO group is not linked to a nitrogen atom of the group $X_3$, and

$X_5$ denotes a piperidin-1,4-ylene group, a 1,4-phenylene, pyridin-2,4-ylene, pyridin-2,5-ylene, pyrimidin-2,5-ylene, pyrazin-2,5-ylene or thiazol-2,5-ylene group optionally substituted by a chlorine atom, with the proviso that the cyclic nitrogen atom of the piperidin-1,4-ylene group is not linked to a nitrogen atom of the group $X_3$ or $X_4$, or

$X_5$-$X_4$-$X_3$ together represent an isoindolin-2,5-ylene or 1,2,3,4-tetrahydro-isoquinolin-2,7-ylene group, with the proviso that the cyclic nitrogen atom of these groups is not linked to a heteroatom of the group X2, or

$X_5$-$X_4$ together may also denote an isoquinolin-1,6-ylene ring, whilst the group B is in position 1 and the group $X_3$ is in position 6, or a 1,2,3,4-tetrahydroisoquinolin-2,6-ylene ring, where the group B is in the 2-position and the group $X_3$ is in the 6 position,

B denotes a cyano group, an amidino group wherein a hydrogen atom on one of the nitrogen atoms may be replaced by an alkoxycarbonyl group having a total of 2 to 5 carbon atoms, an optionally benzyl-substituted amino group, with the proviso that the amino group is not bound to a ring nitrogen atom of the group $X_5$-$X_4$-$X_3$ or $X_5$, or, if B is linked to a ring nitrogen atom of the group $X_5$-$X_4$-$X_3$ or $X_5$, or, if B is linked to the group $X_5$-$X_4$, B may additionally denote a hydrogen atom,

Y denotes a methylene group and

E denotes a carboxy group or an alkoxycarbonyl group having a total of 2 to 5 carbon atoms,

the stereoisomers, tautomers and mixtures thereof and the salts thereof.

**2.** Cyclic imino derivatives of general formula I according to claim 1, wherein, with the proviso that at least one of the groups $R_1$, $X_3$, $X_5$, $X_5$-$X_4$-$X_3$ together or $X_5$-$X_4$ together contains a heterocyclic group, and the shortest distance between the groups B and E is at least 10 bonds,

U denotes a methylene group and $R_1$ denotes a hydrogen atom, a pyridino-3-carbonyl or pyrimidin-2-yl group or

U denotes a carbonyl group and $R_1$ denotes a hydrogen atom, a 3-phenylpropyl, pyridin-2-yl or pyridin-3-yl group,

$X_2$-$X_1$ denotes an $-O-CH_2-$, $-NH-CH_2-$, $-CONH-CH_2-$, $-CONH-CH_2CH_2-$ or, if $X_3$ denotes a 1H-pyridin-2-on-1,3-ylene, 1H-pyridin-2-on-1,5-ylene or 1H,3H-pyrimidin-2,4-dion-3,5-ylene group, $X_2$-$X_1$ may denote a methylene group,

$X_3$ denotes a 1,4-phenylene, pyridazin-3,6-ylene, 1H-pyridin-2-on-1,3-ylene, 1H-pyridin-2-on-1,5-ylene, 2H-pyridazin-3-on-4,6-ylene, 2-methyl-2H-pyridazin-3-on-4,6-ylene or 1H,3H-pyrimidin-2,4-dion-3,5-ylene group, wherein the 1H-pyridin-2-on-1,3-ylene, 1H-pyridin-2-on-1,5-ylene and 1H,3H-pyrimidin-2,4-dion-3,5-ylene group is bound to the group $X_2$-$X_1$ via a ring nitrogen atom,

$X_4$ denotes a bond or an -NHCO- group and

$X_5$ denotes a 1,4-phenylene, pyridin-2,4-ylene, pyridin-2,5-ylene, pyrimidin-2,5-ylene, pyrazin-2,5-ylene, piperidin-1,4-ylene or thiazol-2,5-ylene group, wherein the piperidin-1,4-ylene and pyridin-2,4-ylene group are bound to $X_4$ via position 4, or

$X_5$-$X_4$-$X_3$ together represent an isoindolin-2,5-ylene group, which is bound to the group $X_2$-$X_1$ via the benzo moiety, or

$X_5$-$X_4$ together represent an isoquinolin-1,6-ylene ring or 1,2,3,4-tetrahydroisoquinolin-2,6-ylene ring, if $X_3$ denotes a 1,4-phenylene group, the isoquinolin-1,6-ylene and 1,2,3,4-tetrahydroisoquinolin-2,6-ylene ring being bound to the 1,4-phenylene group via position 6 in each case,

B denotes an amidino group wherein, at one of the nitrogen atoms, a hydrogen atom may be replaced by an alkoxycarbonyl group with a total of 2 to 5 carbon atoms or, if $X_5$ denotes a 1,4-piperidinylene group, B may additionally represent a hydrogen atom or, if $X_5$-$X_4$ together represent an isoquinolin-1,6-ylene ring, B may represent an amino group or a hydrogen atom or, if $X_5$-$X_4$ represents a 1,2,3,4-tetrahydroisoquinolin-2,6-ylene ring, B may represent a hydrogen atom, or, if $X_5$ denotes a pyridin-2,4-ylene ring, B may represent a benzylamino group,

Y denotes a methylene group and

E denotes a carboxy group or an alkoxycarbonyl group having a total of 2 to 5 carbon atoms,

the stereoisomers, tautomers and mixtures thereof and salts thereof.

**3.** Cyclic imino derivatives of general formula I according to claim 1, wherein

$R_1$, U, B, E, $X_1$ to $X_5$ and Y are defined as in claim 2 and the group -Y-E is in position 3 and the group B-$X_5$-$X_4$-$X_3$-$X_2$-$X_1$ is in position 5 of the 2-pyrrolidinone or pyrrolidine ring, and one of the groups $X_5$ or $X_3$ denotes a 1,4-phenylene group,
the stereoisomers and tautomers thereof, the mixtures and salts thereof.

**4.** The following cyclic imino derivatives of general formula I according to claim 1: (1) (3S,5S)-5-[[4-(5-amidino-2-

pyridyl)phenyl]oxymethyl]-3-carboxymethyl-2-pyrrolidinone,

(2) (3S,5S)-5-[[4-(2-amidino-5-pyrimidyl)phenyl]oxymethyl]-3-carboxymethyl-2-pyrrolidinone,

(3) (3S,5S)-5-[[4-(5-amidino-2-pyrimidyl)phenyl]oxymethyl]-3-carboxymethyl-2-pyrrolidinone,

(4) (3S,5S)-5-[[4-(5-amidino-2-thiazolyl)phenyl]oxymethyl]-3-carboxymethyl-2-pyrrolidinone,

(5) (3S,5S)-5-[[4-(2-amidino-5-pyridyl)phenyl]oxymethyl]-3-carboxymethyl-2-pyrrolidinone,

(6) (3S,5S)-5-[[4-(5-amidino-2-pyrazinyl)phenyl]oxymethyl]-3-carboxymethyl-2-pyrrolidinone,

(7) (3S,5S)-5-[(4'-amidino-4-biphenylyl)oxymethyl]-3-carboxymethyl-1-(2-pyrimidyl)-pyrrolidine

(8) (3S,5S)-5-[(4'-amidino-4-biphenylyl)oxymethyl]-3-carboxymethyl-1-nicotinoyl-pyrrolidine,

(9) (3S,5S)-5-[(4'-amidino-4-biphenylyl)oxymethyl]-3-carboxymethyl-1-(2-pyridyl)-2-pyrrolidinone,

(10)(3S,5S)-5-[(4'-amidino-4-biphenylyl)oxymethyl]-3-carboxymethyl-1-(3-pyridyl)-2-pyrrolidinone,

(11) (3S,5S)-5-[[6-(4-amidinophenyl)-3-pyridazinyl]oxymethyl-3-carboxymethyl-1-(3-phenylpropyl)-2-pyrrolidinone,

(13) (3S,5S)-5-[[6-(4-amidinophenyl)-3-pyridazinyl]amino-methyl]-3-carboxymethyl-2-pyrrolidinone,

(14) (3S,5S)-5-[[4-[5-(N-methoxycarbonylamidino)-2-pyridyl]-phenyl]oxymethyl]-3-[(methoxycarbonyl)methyl-2-pyrrolidinone,

(15) (3S,5S)-5-[[4-[2-(N-methoxycarbonylamidino)-5-pyridyl]-phenyl]oxymethyl]-3-[(methoxycarbonyl)methyl-2-pyrrolidinone,

(16) (3S,5S)-5-[[4-[5-(N-methoxycarbonylamidino)-2-pyrazinyl]-phenyl]oxymethyl]-3-[(methoxycarbonyl)methyl-2-pyrrolidinone,

and the tautomers and the salts thereof.

5. Physiologically acceptable salts of the compounds according to at least one of claims 1 to 4 with inorganic or organic acids or bases.

6. Pharmaceutical compositions containing a compound according to at least one of claims 1 to 4 wherein B has the meanings given in claims 1 to 4 with the exception of the cyano group, or a physiologically acceptable salt according to claim 5, optionally together with one or more inert carriers and/or diluents.

7. Use of a compound according to at least one of claims 1 to 4 wherein B has the meanings given in claims 1 to 4 with the exception of the cyano group, or a corresponding physiologically acceptable salt according to claim 5, for preparing a pharmaceutical composition which is suitable for treating or preventing diseases in which smaller or larger cell aggregates occur or cell-matrix interactions are involved.

8. Process for preparing a pharmaceutical composition according to claim 6, characterised in that a compound according to at least one of claims 1 to 4 wherein B has the meanings given in claims 1 to 4 with the exception of the cyano group, or a corresponding physiologically acceptable salt according to claim 5, is incorporated in one or more inert carriers and/or diluents by a non-chemical method.

9. Process for preparing the cyclic imino derivatives according to claims 1 to 5, characterised in that

a) in order to prepare compounds of general formula I wherein E denotes a carboxy group, a compound of general formula

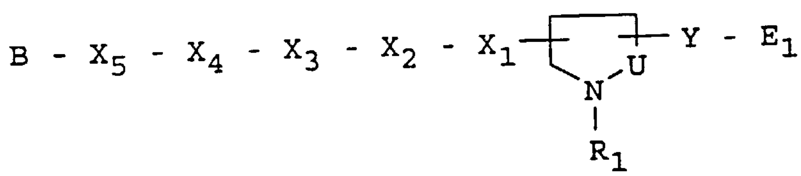 (II)

wherein

$R_1$, B, U, $X_1$ to $X_5$ and Y are defined as in claims 1 to 4 and
$E_1$, which is bound to a carbon atom, denotes a group which may be converted into a carboxy group by hydrolysis, treatment with acids, thermolysis or hydrogenolysis, is converted into a compound of general formula I wherein E is a carboxy group, or

b) in order to prepare compounds of general formula I wherein B denotes an amidino group, a compound of general formula

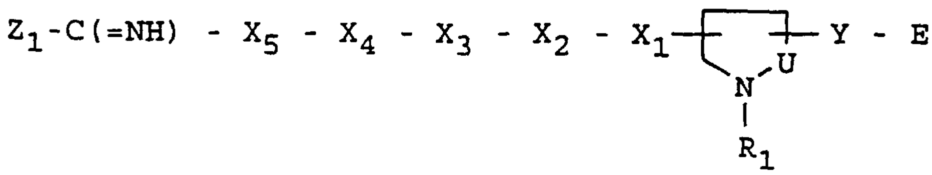 (III)

optionally formed in the reaction mixture, wherein

$R_1$, E, U, $X_1$ to $X_5$ and Y are defined as in claims 1 to 4, and
$Z_1$ denotes an alkoxy, aralkoxy, alkylthio or aralkylthio group, is reacted with ammonia or with the acid addition salts thereof, or

c) in order to prepare compounds of general formula I wherein B denotes an amino group substituted by a benzyl group, a compound of general formula

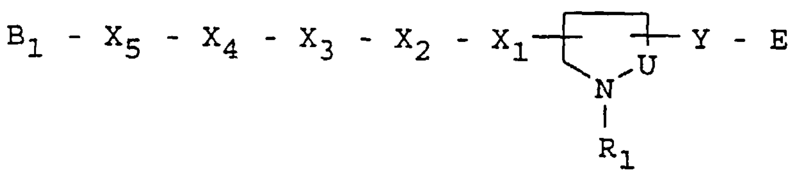 (IV)

wherein

$R_1$,$X_1$ to $X_5$, E, U and Y are defined as in claims 1 to 4 and
$B_1$ denotes an amino group, is reacted with a compound of general formula

$$Z_2 - R_b \qquad (V)$$

wherein
$R_b$ denotes a benzyl group and
$Z_2$ denotes a nucleophilic leaving group, or

d) in order to prepare compounds of general formula I wherein $X_2$ denotes an oxygen atom or an imino group, a compound of general formula

$$B - X_5 - X_4 - X_3 - X_2' - H \qquad (VI)$$

wherein

$X_3$ to $X_5$ and B are defined as in claims 1 to 4 and
$X_2$' denotes an oxygen atom or an imino group, or the alkali metal or alkaline earth metal salts thereof, is reacted with a compound of general formula

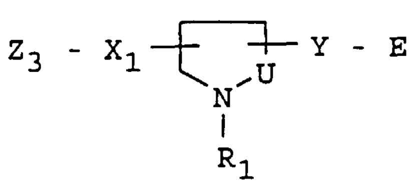 (VII)

wherein
$R_1$, $X_1$, E, U and Y are defined as in claims 1 to 4 and

$Z_3$ denotes a nucleophilic leaving group, or

e) in order to prepare compounds of general formula I wherein $R_1$ represents a pyridinocarbonyl group, a compound of general formula

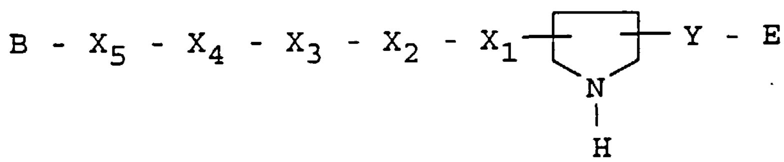

(VIII)

wherein

$X_1$ to $X_5$, B, E and Y are defined as in claims 1 to 4, is acylated with a compound of general formula

$$Z_4 - R_1' \qquad (IX)$$

wherein
$R_1'$ denotes a pyridinocarbonyl group and
$Z_4$ denotes a hydroxy group or a leaving group, or

f) in order to prepare compounds of general formula I wherein $X_2$ denotes a -CONH- group, a compound of general formula

$$B - X_5 - X_4 - X_3 - E_2 \qquad (X)$$

wherein

$X_3$ to $X_5$ and B are defined as in claims 1 to 4 and
$E_2$ denotes a carboxy group, is reacted with a compound of general formula

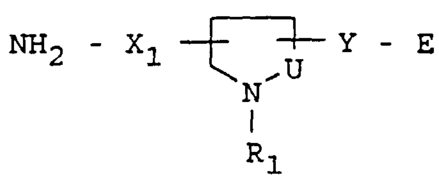

(XI)

wherein
$R_1$, $X_1$, Y, E and U are defined as in claims 1 to 4, or with the reactive derivatives thereof, or

g) in order to prepare compounds of general formula I wherein E denotes a carboxy group or an alkoxycarbonyl group having a total of 2 to 5 carbon atoms, a compound of general formula

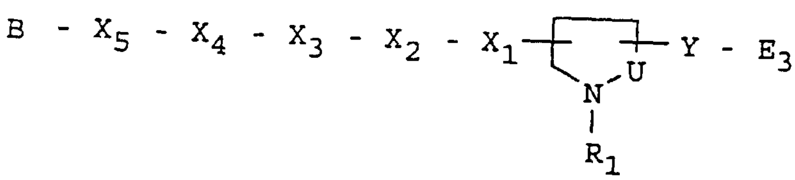

(XII)

wherein

$R_1$, B, U, $X_1$ to $X_5$ and Y are defined as in claims 1 to 4 and

$E_3$ denotes a vinyl or 1,2-dihydroxyalkyl group, is oxidised and subsequently, if necessary, a compound thus obtained is esterified with a corresponding alcohol or

h) in order to prepare compounds of general formula I wherein $R_1$ denotes a pyrimidinyl group, a compound of general formula

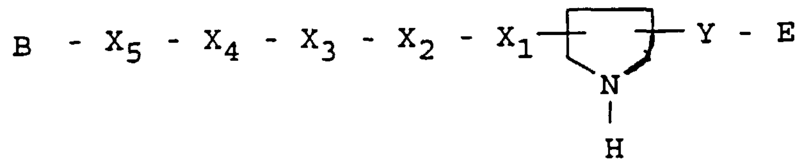

(XIII)

wherein $X_1$ to $X_5$, B, E and Y are defined as in claims 1 to 4, is reacted with a compound of general formula

$$Z_6 - R_1'' \quad \text{(XIV)}$$

wherein

$R_1''$ denotes a pyrimidinyl group and
$Z_6$ denotes a nucleophilic leaving group, or

i) in order to prepare compounds of general formula I wherein E denotes an alkoxycarbonyl group having a total of 2 to 5 carbon atoms, a compound of general formula

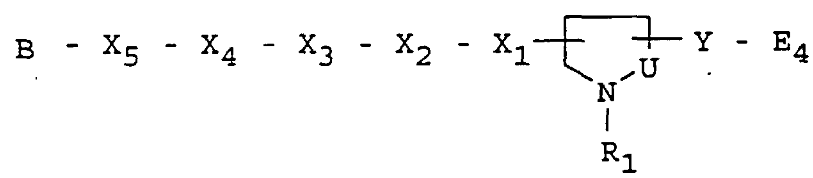

(XV)

wherein

$R_1$, B, U, $X_1$ to $X_5$ and Y are defined as in claims 1 to 4 and
$E_4$ denotes a carboxy group or the reactive derivatives thereof, is reacted with a compound of general formula

$$H - R_e \quad \text{(XVI)}$$

wherein
$R_e$ denotes a $C_{1-4}$-alkoxy group, or

j) in order to prepare compounds of general formula I wherein $X_2$ denotes a -CONH- group and $X_5$-$X_4$-$X_3$ contains a cyclic imino group the ring nitrogen atom of which is linked to the carbonyl group of $X_2$, a compound of general formula

$$B - X_5 - X_4 - X_3 - H \quad \text{(XVII)}$$

wherein

B and $X_5$-$X_4$-$X_3$ are defined as in claims 1 to 4, is reacted with a compound of general formula

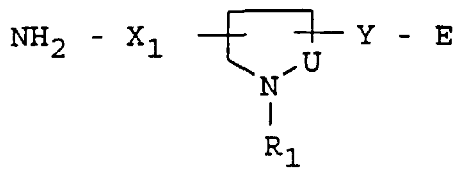

(XVIII)

wherein
$R_1$, E, U, $X_1$ and Y are defined as in claims 1 to 4, in the presence of a compound of general formula

$$Z_7 - CO - Z_8 \quad (XIX)$$

wherein
$Z_7$ and $Z_8$, which may be identical or different, denote nucleophilic leaving groups, or

k) in order to prepare compounds of general formula I wherein $X_2$ represents an imino group and $X_3$ denotes one of the heteroarylene groups mentioned in claims 1 to 4, a compound of general formula

$$B - X_5 - X_4 - X_3' - Z_9 \quad (XX)$$

wherein

B, $X_4$ and $X_5$ are defined as in claims 1 to 4,
$X_3$' represents one of the heteroarylene groups mentioned for $X_3$ in claims 1 to 4 and
$Z_9$ denotes a nucleophilic leaving group, is reacted with a compound of general formula

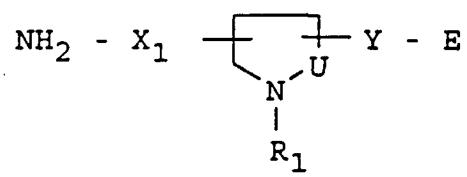

(XVIII)

wherein
$R_1$, E, U, Y and $X_1$ are defined as in claims 1 to 4, or

l) in order to prepare compounds of general formula I wherein $X_2$ denotes a bond, $X_3$ denotes one of the heteroarylene groups mentioned in claims 1 to 4 and $X_3$ is bound to $X_1$ by the ring nitrogen atom of a -CO-N- group, a compound of general formula

$$B - X_5 - X_4 - X_3'' - H \quad (XXI)$$

wherein

B, $X_4$ and $X_5$ are defined as in claims 1 to 4 and
$X_3$" represents one of the heteroarylene groups mentioned for $X_3$ in claims 1 to 4, wherein one or two methine groups adjacent to a nitrogen atom are each replaced by a hydroxymethine group, or the tautomers thereof, is reacted with a compound of general formula

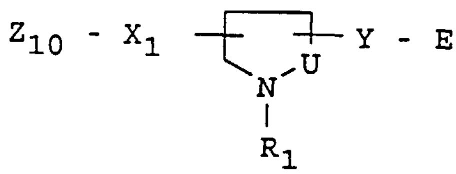

(XXII)

wherein
$R_1$, E, U, $X_1$ and Y are defined as in claims 1 to 4 and
$Z_{10}$ denotes a nucleophilic leaving group, or

m) in order to prepare compounds of general formula I wherein B, $B\text{-}X_5\text{-}X_4\text{-}X_3$, $B\text{-}X_5\text{-}X_4$ or $B\text{-}X_5$ contains an amidino group substituted by an alkoxycarbonyl group having a total of 2 to 5 carbon atoms, a compound of general formula

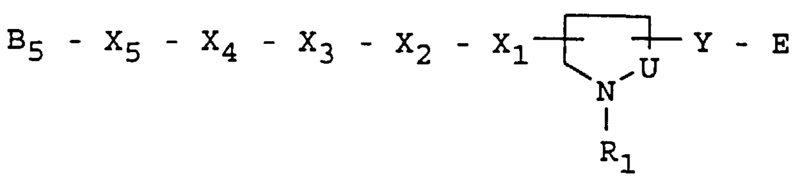

(XXIII)

wherein

$R_1$, E, U, $X_1$ to $X_5$ and Y are defined as in claims 1 to 4 and
$B_5$, $B_5\text{-}X_5\text{-}X_4\text{-}X_3$, $B_5\text{-}X_5\text{-}X_4$ or $B_5\text{-}X_5$ contains an amidino group, is reacted with a compound of general formula

$$Z_{11} - R_f \qquad \text{(XXIV)}$$

wherein
$R_f$ denotes an alkoxycarbonyl group having a total of 2 to 5 carbon atoms and
$Z_{11}$ denotes a nucleophilic leaving group, or

n) in order to prepare compounds of general formula I wherein E denotes an alkoxycarbonyl group having a total of 2 to 5 carbon atoms, a compound of general formula

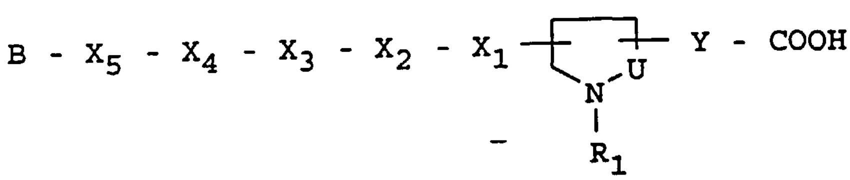

(XXV)

wherein

$R_1$, B, U, $X_1$ to $X_5$ and Y are defined as in claims 1 to 4, is reacted with a compound of general formula

$$Z_{12} - E_5 \qquad \text{(XXVI)}$$

wherein

$E_5$ denotes a $C_{1\text{-}4}$-alkyl group and

$Z_{12}$ denotes a nucleophilic leaving group, and

subsequently, if desired, a compound of general formula I thus obtained which contains a carbonyl bridge is converted by reduction into a corresponding compound of general formula I which contains a methylene bridge, and/or

a compound of general formula I thus obtained which contains an ester group is converted by transesterification into a corresponding ester and

if necessary any protecting group used during the reactions to protect reactive groups is cleaved and/or

if desired a compound of general formula I thus obtained is resolved into the cis/trans-isomers, into the enantiomers and/or diastereomers thereof, and/or

a compound of general formula I thus obtained is converted into the salts thereof, more particularly for pharmaceutical use into the physiologically acceptable salts thereof with an inorganic or organic acid or base.

## Revendications

1. Dérivés imino cycliques de formule générale

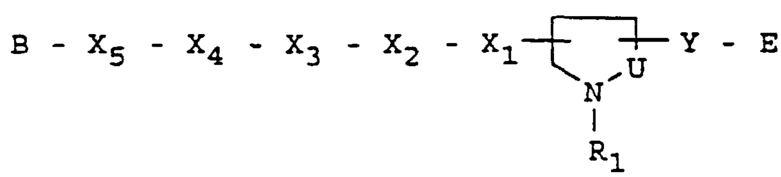

(I)

où, à condition qu'au moins l'un des restes $R_1$, $X_3$, $X_5$, $X_5$-$X_4$-$X_3$ ensemble ou $X_5$-$X_4$ ensemble contienne un reste hétérocyclique, et que la plus courte distance entre les groupes B et E soit d'au moins 10 liaisons,

U représente un groupe méthylène et $R_1$ représente un atome d'hydrogène, un groupe pyridinecarbonyle ou pyrimidinyle ou
U représente un groupe carbonyle et $R_1$ représente un atome d'hydrogène, un groupe 3-phénylpropyle ou pyridinyle,
$X_1$ représente un groupe méthylène ou éthylène,
$X_2$ représente une liaison, un atome d'oxygène, un groupe -CONH- ou imino,
$X_3$ représente un groupe 1,4-phénylène, pyridazin-3,6-ylène, 1H-pyridin-2-on-1,3-ylène, 1H-pyridin-2-on-1,5-ylène, 2H-pyridazin-3-on-4,6-ylène ou 1H,3H-pyrimidine-2,4-dion-3,5-ylène éventuellement substitué par un groupe méthyle à condition qu'un atome d'azote du groupe $X_3$ ne soit pas lié à un hétéroatome ou au groupe -CONH- du groupe $X_2$,
$X_4$ représente une liaison ou un groupe -NHCO- à condition que le groupe -NHCO- ne soit pas lié à un atome d'azote du groupe $X_3$, et
$X_5$ représente un groupe pipéridin-1,4-ylène, un groupe 1,4-phénylène, pyridin-2,4-ylène, pyridin-2,5-ylène, pyrimidin-2,5-ylène, pyrazin-2,5-ylène ou thiazol-2,5-ylène éventuellement substitué par un atome de chlore à condition que l'atome d'azote cyclique du groupe pipéridin-1,4-ylène ne soit pas lié à un atome d'azote du groupe $X_3$ ou $X_4$, ou
$X_5$-$X_4$-$X_3$ ensemble représente aussi un groupe isoindolin-2,5-ylène ou 1,2,3,4-tétrahydro-isoquinolin-2,7-ylène à condition que l'atome d'azote cyclique de ces groupes ne soit pas lié à un hétéroatome du groupe $X_2$, ou
$X_5$-$X_4$ ensemble représente aussi un groupe isoquinolin-1,6-ylène, où le reste B est en position 1 et le reste $X_3$ est en position 6, ou un groupe 1,2,3,4-tétrahydroisoquinolin-2,6-ylène, où le reste B est en position 2 et le reste $X_3$ est en position 6,
B représente un groupe cyano, un groupe amidino, où sur l'un des atomes d'azote un atome d'hydrogène peut être remplacé par un groupe alcoxycarbonyle ayant au total 2 à 5 atomes de carbone, un groupe amino éventuellement substitué par un groupe benzyle à condition que le groupe amino ne soit pas lié à un atome d'azote

cyclique du groupe $X_5$-$X_4$-$X_3$ ou $X_5$, ou, au cas où B est lié à un atome d'azote cyclique du groupe $X_5$-$X_4$-$X_3$ ou $X_5$ ou, au cas où B est lié au groupe $X_5$-$X_4$, en outre aussi un atome d'hydrogène,

Y représente un groupe méthylène et

E représente un groupe carboxyle ou un groupe alcoxycarbonyle ayant au total 2 à 5 atomes de carbone, leurs stéréoisomères, leurs tautomères, leurs mélanges et leurs sels.

**2.** Dérivés imino cycliques de formule générale I selon la revendication 1, où, à condition qu'au moins l'un des restes $R_1$, $X_3$, $X_5$, $X_5$-$X_4$-$X_3$ ensemble ou $X_5$-$X_4$ ensemble contienne un reste hétérocyclique, et que la plus courte distance entre les groupes B et E soit d'au moins 10 liaisons,

U représente un groupe méthylène et $R_1$ représente un atome d'hydrogène, un groupe pyridine-3-carbonyle ou pyrimidin-2-yle ou

U représente un groupe carbonyle et $R_1$ représente un atome d'hydrogène, un groupe 3-phénylpropyle, pyridin-2-yle ou pyridin-3-yle,

$X_2$-$X_1$ représente un groupe -O-$CH_2$-, -NH-$CH_2$-, -CONH-$CH_2$-, -CONH-$CH_2CH_2$- ou, au cas où $X_3$ représente un groupe 1H-pyridin-2-on-1,3-ylène, 1H-pyridin-2-on-1,5-ylène ou 1H,3H-pyrimidine-2,4-dion-3,5-ylène, aussi un groupe méthylène,

$X_3$ représente un groupe 1,4-phénylène, pyridazin-3,6-ylène, 1H-pyridin-2-on-1,3-ylène, 1H-pyridin-2-on-1,5-ylène, 2H-pyridazin-3-on-4,6-ylène, 2-méthyl-2H-pyridazin-3-on-4,6-ylène ou 1H,3H-pyrimidine-2,4-dion-3,5-ylène, où le groupe 1H-pyridin-2-on-1,3-ylène, 1H-pyridin-2-on-1,5-ylène et le groupe 1H,3H-pyrimidine-2,4-dion-3,5-ylène sont liés au groupe $X_2$-$X_1$ par un atome d'azote cyclique,

$X_4$ représente une liaison ou un groupe -NHCO- et

$X_5$ représente un groupe 1,4-phénylène, pyridin-2,4-ylène, pyridin-2,5-ylène, pyrimidin-2,5-ylène, pyrazin-2,5-ylène, pipéridin-1,4-ylène ou thiazol-2,5-ylène, où le groupe pipéridin-1,4-ylène et le groupe pyridin-2,4-ylène sont liés chacun par la position 4 au groupe $X_4$, ou

$X_5$-$X_4$-$X_3$ ensemble représente aussi un groupe isoindolin-2,5-ylène, où celui-ci est lié au groupe $X_2$-$X_1$ par la partie benzo, ou

$X_5$-$X_4$ ensemble représente aussi un cycle isoquinolin-1,6-ylène ou 1,2,3,4-tétrahydroisoquinolin-2,6-ylène, au cas ou $X_3$ représente un groupe 1,4-phénylène, où les cycles isoquinolin-1,6-ylène et 1,2,3,4-tétrahydroisoquinolin-2,6-ylène sont liés chacun au groupe 1,4-phénylène par la position 6,

B représente un groupe amidino, où sur l'un des atomes d'azote un atome d'hydrogène peut être remplacé par un groupe alcoxycarbonyle ayant au total 2 à 5 atomes de carbone, ou, au cas où $X_5$ représente un groupe 1,4-pipéridinylène, en outre aussi un atome d'hydrogène, ou, au cas où $X_5$-$X_4$ ensemble représente un cycle isoquinolin-1,6-ylène, un groupe amino ou un atome d'hydrogène ou, au cas où $X_5$-$X_4$ représente un cycle 1,2,3,4-tétrahydroisoquinolin-2,6-ylène, un atome d'hydrogène ou, au cas où $X_5$ représente un cycle pyridin-2,4-ylène, un groupe benzylamino,

Y représente un groupe méthylène et

E représente un groupe carboxyle ou un groupe alcoxycarbonyle ayant au total 2 à 5 atomes de carbone, leurs stéréoisomères, leurs tautomères, leurs mélanges et leurs sels.

**3.** Dérivés imino cycliques de formule générale I selon la revendication 1 où $R_1$, U, B, E, $X_1$ à $X_5$ et Y sont définis comme indiqué dans la revendication 2 et le reste -Y-E est en position 3 et le reste B-$X_5$-$X_4$-$X_3$-$X_2$-$X_1$- est en position 5 du cycle 2-pyrrolidinone ou pyrrolidine, et l'un des restes $X_5$ et $X_3$ représente un groupe 1,4-phénylène, leurs stéréoisomères, leurs tautomères, leurs mélanges et leurs sels.

**4.** Dérivés imino cycliques de formule générale I selon la revendication 1 suivants:

(1) (3S,5S)-5-[[4(5-amidino-2-pyridyl)phényl]oxyméthyl]-3-carboxyméthyl-2-pyrrolidinone,
(2) (3S,5S)-5-[[4-(2-amidino-5-pyrimidyl)phényl]oxyméthyl]-3-carboxyméthyl-2-pyrrolidinone,
(3) (3S,5S)-5-[[4-(5-amidino-2-pyrimidyl)phényl]oxyméthyl]-3-carboxyméthyl-2-pyrrolidinone,
(4) (3S,5S)-5-[[4-(5-amidino-5-thiazolyl)phényl]oxyméthyl]-3-carboxyméthyl-2-pyrrolidinone,
(5) (3S,5S)-5-[[4-12-amidino-5-pyridyl)phényl]oxyméthyl]-3-carboxyméthyl-2-pyrrolidinone,
(6) (3S,5S)-5-[[4-(5-amidino-2-pyrazinyl)phényl]oxyméthyl]-3-carboxyméthyl-2-pyrrolidinone,
(7) (3S,5S)-5-[(4'-amidino-4-biphénylyl)oxyméthyl]-3-carboxyméthyl-1-(2-pyrimidyl)-pyrrolidine,
(8) (3S,5S)-5-[(4'-amidino-4-biphénylyl)oxyméthyl]-3-carboxyméthyl-1-nicotinoyl-pyrrolidine,
(9) (3S,5S)-5-[(4'-amidino-4-biphénylyl)oxyméthyl]-3-carboxyméthyl-1-(2-pyridyl)-2-pyrrolidinone,
(10) (3S,5S)-5-[(4'-amidino-4-biphénylyl)oxyméthyl]-3-carboxyméthyl-1-(3-pyridyl)-2-pyrrolidinone,
(11) (3S,5S)-5-[[6-(4-amidinophényl)-3-pyridazinyl]oxyméthyl]-3-carboxyméthyl-1-(3-phénylpropyl)-2-pyrrolidinone,

(13) (3S,5S)-5-[[6-(4-amidinophényl)-3-pyridazinyl]aminométhyl]-3-carboxyméthyl-2-pyrrolidinone,

(14) (3S,5S)-5-[[4-[5-(N-méthoxycarbonylamidino]-2-pyridyl]phényl]oxyméthyl]-3[(méthoxycarbonyl)méthyl]-2-pyrrolidinone,

(15) (3S,5S)-5-[[4-[2-(N-méthoxycarbonylamidino]-5-pyridyl]phényl]oxyméthyl]-3-[(méthoxycarbonyl)méthyl]1-2-pyrrolidinone,

(16) (3S,5S)-5-[[4-[5-(N-méthoxycarbonylamidino]-2-pyrazinyl]phényl]oxyméthyl]-3-[(méthoxycarbonyl)méthyl]-2-pyrrolidinone,

leurs tautomères et leurs sels.

**5.** Sels physiologiquement acceptables des composés selon au moins l'une des revendications 1 à 4 avec des acides ou des bases inorganiques ou organiques.

**6.** Médicament contenant un composé selon au moins l'une des revendications 1 à 4 dans lesquels B présente les significations indiquées dans les revendications 1 à 4 à l'exception du groupe cyano, ou un sel physiologiquement acceptable correspondant selon la revendication 5 outre éventuellement un ou plusieurs supports et/ou diluants inertes.

**7.** Utilisation d'un composé selon au moins l'une des revendications 1 à 4 dans lesquels B présente les significations indiquées dans les revendications 1 à 4 à l'exception du groupe cyano, ou d'un sel physiologiquement acceptable correspondant selon la revendication 5 pour la préparation d'un médicament qui convient pour la lutte contre ou la prévention de maladies dans lesquelles il apparaît des agrégats cellulaires relativement petits ou relativement grands ou des interactions cellules-matrice jouent un rôle.

**8.** Procédé de préparation d'un médicament selon la revendication 6 caractérisé en ce que, par voie non chimique, un composé selon au moins l'une des revendications 1 à 4 où B présente les significations indiquées dans les revendications 1 à 4 à l'exception du groupe cyano, ou un sel physiologiquement acceptable correspondant selon la revendication 5 est incorporé dans un ou plusieurs supports et/ou diluants inertes.

**9.** Procédé de préparation des dérivés imino cycliques selon les revendications 1 à 5 caractérisé en ce que

a) pour la préparation de composés de formule générale I où E représente un groupe carboxyle, un composé de formule générale

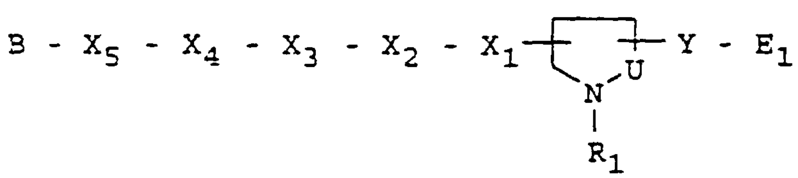

(II)

où

$R_1$, B, U, $X_1$ à $X_5$ et Y sont définis comme dans les revendications 1 à 4 et
$E_1$, qui est lié à un atome de carbone, représente un groupe qui peut être converti en un groupe carboxyle par hydrolyse, traitement avec des acides, thermolyse ou hydrogénolyse, est converti en un composé de formule générale I où E représente un groupe carboxyle, ou

b) pour la préparation de composés de formule générale I où B représente un groupe amidino, un composé éventuellement formé dans le mélange réactionnel de formule générale

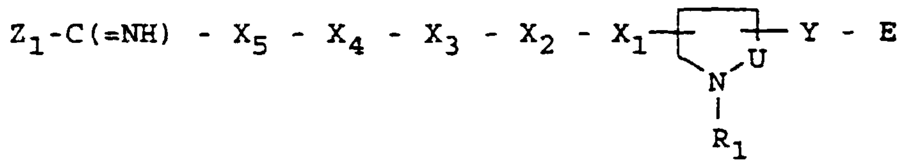

(III)

où

$R_1$, E, U, $X_1$ à $X_5$ et Y sont définis comme dans les revendications 1 à 4 et
$Z_1$ représente un groupe alcoxy, aralcoxy, alkylthio ou aralkylthio, est mis à réagir avec l'ammoniac ou avec ses sels d'addition d'acide ou

c) pour la préparation de composés de formule générale I où B représente un groupe amino substitué par un groupe benzyle, un composé de formule générale

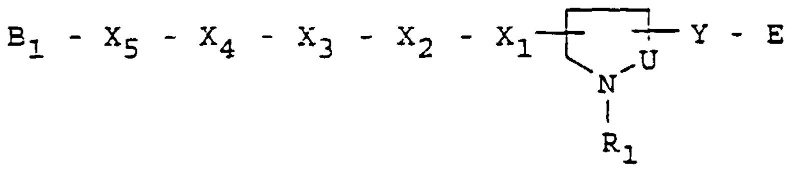

(IV)

où

$R_1$, $X_1$ à $X_5$, E, U et Y sont définis comme dans les revendications 1 à 4 et
$B_1$ représente un groupe amino, est mis à réagir avec un composé de formule générale

$$Z_2 - R_b \qquad (V)$$

où
$R_b$ représente un groupe benzyle et
$Z_2$ représente un groupe partant nucléophile, ou

d) pour la préparation de composés de formule générale I où $X_2$ représente un atome d'oxygène ou un groupe imino, un composé de formule générale

$$B - X_5 - X_4 - X_3 - X_2' - H \qquad (VI)$$

où

$X_3$ à $X_5$ et B sont définis comme dans les revendications 1 à 4 et
$X_2'$ représente un atome d'oxygène ou un groupe imino, ou ses sels alcalins ou alcalino-terreux, est mis à réagir avec un composé de formule générale

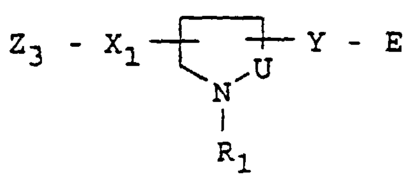

(VII)

où
$R_1$, $X_1$, E, U et Y sont définis comme dans les revendications 1 à 4 et

$Z_3$ représente un groupe partant nucléophile, ou

e) pour la préparation de composés de formule générale I où $R_1$ représente un groupe pyridinecarbonyle, un composé de formule générale

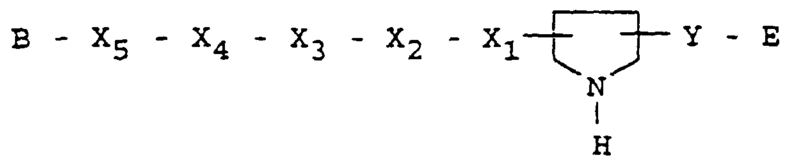

(VIII)

où

$X_1$ à $X_5$, B, E et Y sont définis comme dans les revendications 1 à 4, est acylé avec un composé de formule générale

$$Z_4 - R_1' \qquad (IX)$$

où
$R_1$' représente un groupe pyridinecarbonyle et
$Z_4$ représente un groupe hydroxyle ou partant, ou

f) pour la préparation de composés de formule générale I où $X_2$ représente un groupe -CONH-, un composé de formule générale

$$B - X_5 - X_4 - X_3 - E_2 \qquad (X)$$

où

$X_3$ à $X_5$ et B sont définis comme dans les revendications 1 à 4 et
$E_2$ représente un groupe carboxyle, est mis à réagir avec un composé de formule générale

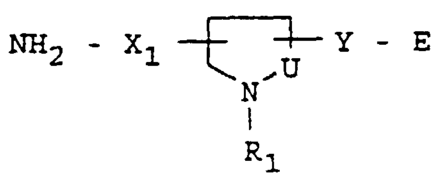

(XI)

où
$R_1$, $X_1$, Y, E et U sont définis comme dans les revendications 1 à 4 ou avec ses dérivés réactifs ou

g) pour la préparation de composés de formule générale I où E représente un groupe carboxyle ou un groupe alcoxycarbonyle ayant au total 2 à 5 atomes de carbone, un composé de formule générale

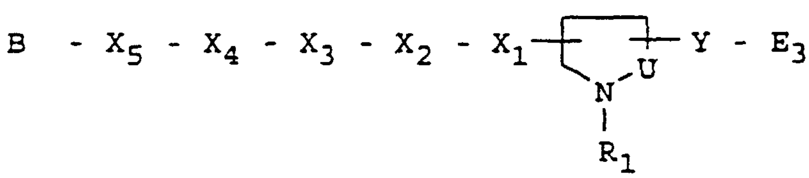

(XII)

où

$R_1$, B, U, $X_1$ à $X_5$ et Y sont définis comme dans les revendications 1 à 4 et

$E_3$ représente un groupe vinyle ou 1,2-dihydroxyalkyle, est oxydé et, si nécessaire, un composé ainsi obtenu est ensuite estérifié avec un alcool correspondant ou

h) pour la préparation de composés de formule générale I où $R_1$ représente un groupe pyrimidinyle, un composé de formule générale

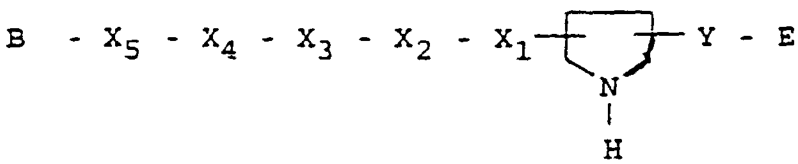 (XIII)

où

$X_1$ à $X_5$, B, E et Y sont définis comme dans les revendications 1 à 4, est mis à réagir avec un composé de formule générale

$$Z_6 - R_1'' \qquad \text{(XIV)}$$

où

$R_1''$ représente un groupe pyrimidinyle et

$Z_6$ représente un groupe partant nucléophile, ou

i) pour la préparation de composés de formule générale I où E représente un groupe alcoxycarbonyle ayant au total 2 à 5 atomes de carbone, un composé de formule générale

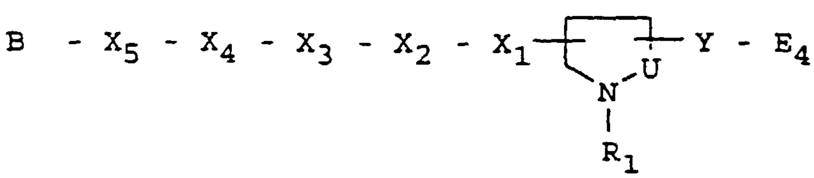 (XV)

où

$R_1$, B, U, $X_1$ à $X_5$ et Y sont définis comme dans les revendications 1 à 4 et

$E_4$ représente un groupe carboxyle ou ses dérivés réactifs, est mis à réagir avec un composé de formule générale

$$H - R_e \qquad \text{(XVI)}$$

où

$R_e$ représente un groupe alcoxy de 1 à 4 atomes de carbone, ou

j) pour la préparation de composés de formule générale I où $X_2$ représente un groupe -CONH- et $X_5$-$K_4$-$X_3$ contient un groupe imino cyclique dont l'atome d'azote cyclique est lié au groupe carbonyle de $X_2$, un composé de formule générale

$$B - X_5 - X_4 - X_3 - H \qquad \text{(XVII)}$$

où

B et $X_5$-$X_4$-$X_3$ sont définis comme dans les revendications 1 à 4, est mis à réagir avec un composé de formule générale

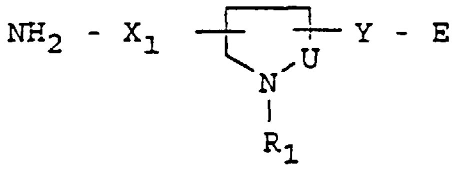

(XVIII)

où

$R_1$, E, U, $X_1$ et Y sont définis comme dans les revendications 1 à 4, en présence d'un composé de formule générale

$$Z_7 - CO - Z_8 \qquad (XIX)$$

où

$Z_7$ et $Z_8$, qui peuvent être identiques ou différents, représentent des groupes partants nucléophiles, ou

k) pour la préparation de composés de formule générale I où $X_2$ représente un groupe imino et $X_3$ représente l'un des groupes hétéroarylène cités dans les revendications 1 à 4, un composé de formule générale

$$B - X_5 - X_4 - X_3' - Z_9 \qquad (XX)$$

où

B, $X_4$ et $X_5$ sont définis comme dans les revendications 1 à 4, $X_3$' représente l'un des groupes hétéroarylène cités pour $X_3$ dans les revendications 1 à 4 et
$Z_9$ représente un groupe partant nucléophile, est mis à réagir avec un composé de formule générale

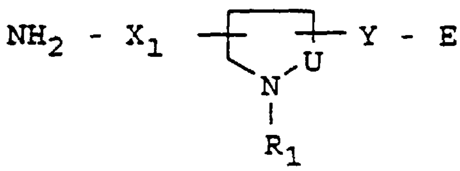

(XVIII)

où

$R_1$, E, U, Y et $X_1$ sont définis comme dans les revendications 1 à 4 ou

l) pour la préparation de composés de formule générale I où $X_2$ représente une liaison, $X_3$ représente l'un des groupes hétéroarylène cités dans les revendications 1 à 4 et $X_3$ est lié à $X_1$ par l'atome d'azote cyclique d'un groupe -CO-N-, un composé de formule générale

$$B - X_5 - X_4 - X_3'' - H \qquad (XXI)$$

où

B, $X_4$ et $X_5$ sont définis comme dans les revendications 1 à 4 et $X_3$'' représente l'un des groupes hétéroarylène cités dans les revendications 1 à 4 pour $X_3$ où un ou deux groupes méthine voisins d'un atome d'azote sont remplacés chacun par un groupe hydroxyméthine, ou ses tautomères, est mis à réagir avec un composé de formule générale

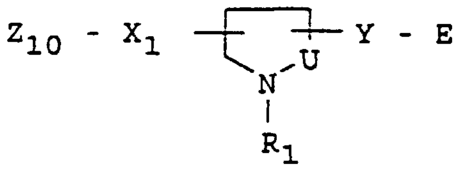 (XXII)

où

$R_1$, E, U, $X_1$ et Y sont définis comme dans les revendications 1 à 4 et
$Z_{10}$ représente un groupe partant nucléophile ou

m) pour la préparation de composés de formule générale I où B, B-$X_5$-$X_4$-$X_3$, B-$X_5$-$X_4$ ou B-$X_5$ contient un groupe amidino substitué par un groupe alkyloxycarbonyle ayant au total 2 à 5 atomes de carbone, un composé de formule générale

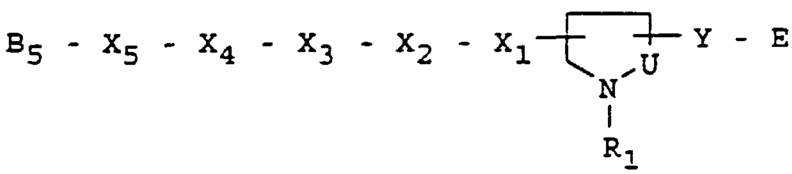 (XXIII)

où

$R_1$, E, U, $X_1$ à $X_5$ et Y sont définis comme dans les revendications 1 à 4 et
$B_5$, $B_5$-$X_5$-$X_4$-$X_3$, $B_5$-$X_5$-$X_4$ ou $B_5$-$X_5$ contient un groupe amidino, est mis à réagir avec un composé de formule générale

$$Z_{11} - R_f \qquad (XXIV)$$

où
$R_f$ représente un groupe alcoxycarbonyle ayant au total 2 à 5 atomes de carbone et
$Z_{11}$ représente un groupe partant nucléophile, ou

n) pour la préparation de composés de formule générale I où E représente un groupe alcoxycarbonyle ayant au total 2 à 5 atomes de carbone, un composé de formule générale

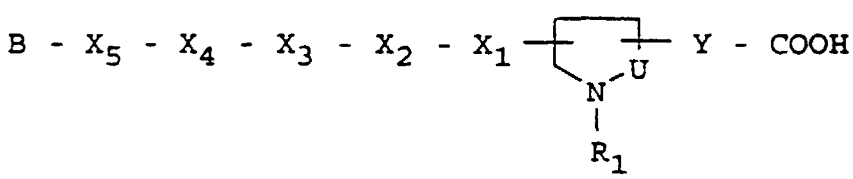 (XXV)

où

$R_1$, B, U, $X_1$ à $X_5$ et Y sont définis comme dans les revendications 1 à 4, est mis à réagir avec un composé de formule générale

$$Z_{12} - E_5 \qquad (XXVI)$$

où
$E_5$ représente un groupe alkyle de 1 à 4 atomes de carbone et

$Z_{12}$ représente un groupe partant nucléophile, et

si on le souhaite un composé de formule générale I ainsi obtenu, qui contient un pont carbonyle, est ensuite converti par réduction en un composé de formule générale I

correspondant, qui contient un pont méthylène, et/ou un composé de formule générale I ainsi obtenu, qui contient un groupe ester, est converti par transestérification en un ester correspondant, et/ou

si nécessaire, un reste protecteur utilisé pendant les réactions pour la protection de groupes réactifs est clivé et/ou

si on le souhaite un composé de formule générale I ainsi obtenu est résolu en ses isomères cis/trans, en ses énantiomères et/ou en ses diastéréoisomères et/ou

un composé de formule générale I ainsi obtenu est converti en ses sels, en particulier pour l'utilisation pharmaceutique en ses sels physiologiquement acceptables avec un acide ou une base inorganique ou organique.